(19) Europäisches Patentamt
European Patent Office
Office européen des brevets

(11) EP 2 420 496 B1

(12) EUROPEAN PATENT SPECIFICATION

(45) Date of publication and mention of the grant of the patent:
**24.12.2014 Bulletin 2014/52**

(21) Application number: **10720187.3**

(22) Date of filing: **16.04.2010**

(51) Int Cl.:
***C07D 309/30*** (2006.01) ***A61K 31/366*** (2006.01)
***A61P 3/00*** (2006.01) ***A61P 9/00*** (2006.01)
***A61P 25/08*** (2006.01) ***A61P 25/28*** (2006.01)
***A61P 31/12*** (2006.01)

(86) International application number:
**PCT/ES2010/070234**

(87) International publication number:
**WO 2010/119161 (21.10.2010 Gazette 2010/42)**

(54) **Antiepileptic, hypocholesterolemic and neuroprotective compound**

Neuroprotektive, hypercholesterolemische und antiepileptische Verbindung

Composé neuroprotecteur, hypocholestérolémiant et antiépileptique

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **16.04.2009 EP 09382051**

(43) Date of publication of application:
**22.02.2012 Bulletin 2012/08**

(73) Proprietor: **Neuron Biopharma, S.A.**
**18100 Armilla - Granada (ES)**

(72) Inventors:
- **BURGOS MUÑOZ, Javier Santos**
**E-18100 Armilla - Granada (ES)**
- **ADRIO FONDEVILA, José Luis**
**E-18100 Armilla - Granada (ES)**
- **RAMOS MARTÍN, Maria del Carmen**
**E-18100 Armilla - Granada (ES)**
- **SIERRA ÁVILA, Saleta**
**E-18100 Armilla - Granada (ES)**
- **ALFARO SÁNCHEZ, Juan María**
**E-18100 Armilla - Granada (ES)**
- **RAMÍREZ MORENO, Carlos**
**E-18100 Armilla - Granada (ES)**
- **CAMPOY GARCÍA, Sonia**
**E-18100 Armilla - Granada (ES)**
- **VELASCO ALVAREZ, Javier**
**E-18100 Armilla - Granada (ES)**
- **RUMBERO SÁNCHEZ, Ángel**
**E-18100 Armilla - Granada (ES)**

(74) Representative: **ABG Patentes, S.L.**
**Avenida de Burgos, 16D**
**Edificio Euromor**
**28036 Madrid (ES)**

(56) References cited:
**EP-A- 0 033 538** **EP-A- 0 605 230**
**WO-A-99/11258** **US-A- 4 293 496**
**US-A- 4 444 784** **US-A- 4 450 171**

- **CHEMICAL ABSTRACTS, vol. 130, no. 223113, 22 March 1999 (1999-03-22), Columbus, Ohio, US; abstract no.: 1999:184128,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Field of the Invention

The present invention discloses the prevention and/or the treatment of neurodegenerative diseases or of diseases associated with an unwanted oxidation or of age-associated pathological processes, as well as to the prevention and/or the treatment of epilepsy, of epileptic seizures or of convulsions, to the decrease of LDL cholesterol levels and to the inhibition of the enzyme 3-hydroxy-3-methylglutaryl coenzyme A reductase for the prevention of dyslipemia and of cardiovascular diseases.

Background of the Invention

The high incidence of neurodegenerative diseases and of age-associated diseases is a problem of the first order worldwide. It is therefore necessary to search for neuroprotective compounds preventing or palliating said diseases. Of all of them, Alzheimer's disease (AD) is the most prevalent, it being estimated that 81 million people will suffer from this disease in 2040 (Blennow et al., Lancet 2006; 368: 387-403). It is estimated that half a million people are currently suffering from AD in Spain alone. The costs associated to this disease are proportionally high, and it is calculated that the total cost derived from caring for Alzheimer's patients is 81,000 and 22,000 million € in the United States and in the United Kingdom, respectively. Currently there are no effective drugs which prevent or impede this disease, therefore it is necessary to search for and validate novel neuroprotective compounds which prevent neuronal damage.

Different strategies are currently being followed for obtaining novel compounds, since it has been seen that the current drugs offer little benefits to the patients. These drugs temporarily delay (one year, at best) some symptoms of the illness but do not prevent their evolution. The current therapeutic options are based on the inhibition of acetylcholinesterase with drugs such as donepezil, galantamine or rivastigmine, or on the capacity of memantine in antagonizing a glutamate receptor, NMDA (N-methyl-D-aspartic acid).

Due to the low success of these drugs new lines of research have opened up and among them, research on inhibitors of the 3-hydroxy-3-methylglutaryl-coenzyme A reductase (HMGR) enzyme (known as statins) as therapeutic agents stands out in recent years. HMGR is the enzyme which catalyzes the limiting step in cholesterol biosynthesis; therefore its inhibition by statins is a usual therapeutic strategy to reduce the high cholesterol levels associated to the low density lipoproteins (LDL). These drugs reduce the risk of myocardial infarction and coronary death, and are considered safe. Moreover, hypercholesterolemia (defined as a high blood cholesterol level) is the main risk factor for ischemic cardiovascular disease, such as atherosclerosis.

Several genetic and environmental factors affecting cholesterol metabolism are associated with AD. For example, the apolipoprotein E ε4 (apoE4) isoform is a risk factor for AD and is linked to an increase in cholesterol levels. Atherosclerosis, which has hypercholesterolemia as the main risk factor, also seems to be associated to AD. Furthermore, epidemiological studies indicate that high serum cholesterol levels increase the risk of AD, and it has been proposed that homeostatic regulation of cholesterol metabolism can be altered in Alzheimer's. On the other hand, a significant reduction of the risk of Alzheimer's in patients treated with statins has been described. All these studies jointly suggest that the reduction of cholesterol levels can inhibit the pathogenesis of Alzheimer's disease (Cole & Vassar; Neurobiol Dis 2006; 22[2]:209-22).

Cholesterol is transported through blood by means of different types of lipoproteins, in which the major cholesterol carriers are low density lipoproteins (LDL) and high density lipoproteins (HDL). LDLs are lipoproteins specialized in transporting cholesterol and triglycerides from the liver to peripheral tissues, in which they are captured by the cells through the LDL receptors (LDL-R) in cell membrane. LDLs also regulate cholesterol synthesis, and high LDL cholesterol levels have been associated to the risk of suffering from cardiovascular diseases (CVD). In turn, HDLs are lipoproteins which transport cholesterol from the different tissues to the liver. Due to the fact that HDLs can remove cholesterol from arteries and transport it back to the liver for its excretion, they are given a protective role against cardiovascular diseases. HMGR inhibitors are the most successful hypolipidemic agents in history, being capable of reducing total cholesterol levels based on decreasing LDL cholesterol levels without altering HDL cholesterol levels.

New properties of the statins have recently been described, especially at the level of brain damage caused by trauma or in dementias, new antioxidant and anti-inflammatory activities being proposed (Pahan, Cell Mol Life Sci. 2006; 63[10]:1165-78), and certain statins (e.g., simvastatin) have been demonstrated to intensify the learning and memory capacity in mice (Ling et al., Ann Neurol. 2006; 60[6]:729-39) or protect them against convulsive seizures associated to epileptic phenomena (Lee et al., Neurosci Lett. 2008; 440: 260-4). Moreover, the statins have also demonstrated their effectiveness in phase II clinical trials which suggest positive results against the treatment of cerebral vasospasm (Fandino et al., Neurocirugia. 2007; 18: 16-27), as well as against neuronal death induced by ischemic damage in the retina (Honjo et al., Arch. Ophthalmol. 2002; 120: 1707-13). Nevertheless, it is currently being discussed whether the neuroprotective effects of the different commercial statins (e.g., atorvastatin, lovastatin, simvastatin, etc.) are due to a direct effect on

the lipid metabolism, or whether in contrast they are a result of alternative routes.

Patent application WO 99/11258 describes a compound with a structure similar to the one of the present invention. Nevertheless, this document does not specify the configuration of the different chiral centers present in the compound.

Summary of the Invention

Although there is literature on the potential neuroprotective effect of statins, the authors of this invention have found that a derivative of a non-commercial monacolin J, specifically (1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthalenyl-2-ethyl-butyrate (also sometimes identified as NST0037 in this document) is an agent with a surprisingly neuroprotective potential, in addition to having a high capacity of reducing blood lipid (cholesterol and triglycerides) levels, very effectively inhibiting HMGR, and protecting against epilepsy, epileptic seizures and convulsions. Furthermore, and surprisingly, this compound is safer than commercial statins, showing toxicity levels under the levels of the statin showing the highest level of biosafety, simvastatin. Additionally, it refers to a compound which is less expensive to synthesize due to the low cost of the side chain to be added to the monacolin J molecule.

The neuroprotective activity of said compound has been clearly shown against different aggressions which cause neuronal death in human cell lines of cholinergic origin by means of different types of aggressions which cause oxidative stress, reticular stress or apoptosis (Example 2, Figures 1 to 4). Said example clearly shows the potential use of said compound in the prevention and/or treatment of neuronal death associated to neurodegenerative diseases (e.g., Alzheimer's, Parkinson's, multiple sclerosis, amyotrophic lateral sclerosis, *status epilepticus,* Huntington's) or of diseases associated with undesired oxidation or of age-associated pathological processes.

The neuroprotective activity of said compound has been confirmed in a mouse model of Alzheimer's disease, in which this compound exerts a neuroprotective effect against neuronal death in the hippocampus caused by an excitotoxic substance (Example 3, Figure 5). Furthermore, it has been found that said substance restores temporal and spatial memory in mice with neurodegeneration (Example 3, Figures 6 and 7), in addition to preventing death of animals caused by the administration of an excitotoxic substance (Example 3, Figure 8). Said examples clearly show the potential use of this compound in the prevention and/or treatment of neuronal death and of the cognitive deficit associated to neurodegenerative diseases (e.g., Alzheimer's, Parkinson's, multiple sclerosis, amyotrophic lateral sclerosis, *status epilepticus,* Huntington's) or of diseases associated with undesired oxidation or of age-associated pathological processes.

The antiepileptic and anticonvulsant activity of said compound has been clearly shown by means of the determination of the protection against epileptic seizures and convulsions in an epilepsy model in mice (Example 4, Figures 9 and 10). Said example clearly shows the potential use of this compound in the prevention and/or treatment of epilepsy and convulsive seizures or convulsions.

The hypolipidemic activity of said compound has been clearly shown by means of the determination of the HMGR inhibition in comparison to two commercial statins, simvastatin and atorvastatin (Example 5, Figure 11).

The hypolipidemic capacity of said compound has additionally been demonstrated in an endogenous hyperlipidemia model in mice, an effect similar to simvastatin in reducing total plasma cholesterol levels, LDL cholesterol levels, VLDL cholesterol levels and esterified cholesterol fraction levels being observed. In contrast, and like with simvastatin, it alters neither HDL cholesterol levels nor free cholesterol fraction levels, giving it a protective role against cardiovascular diseases (CVD) (Example 5, Figures 12 to 17). Said examples clearly show the potential use of said compound in the prevention and/or treatment of hypercholesterolemia associated to cardiovascular diseases (e.g., myocardial infarction, atherosclerosis, congenital cardiopathy, acquired cardiopathy, ischemic cardiopathy, hypertensive cardiopathy, valvulopathies, cardiomyopathies, blood disorders).

The hypolipidemic activity of said compound has been confirmed in an induced hyperlipidemia model in mice (Example 6, Figures 18 to 23), an effect greater than simvastatin in reducing the total plasma cholesterol levels, LDL cholesterol levels and free and esterified cholesterol fraction levels being observed. In contrast, this compound alters neither HDL cholesterol levels nor VLDL cholesterol levels, giving it a protective role against cardiovascular diseases (CVD). Said examples clearly show the potential use of said compound in the prevention and/or treatment of hypercholesterolemia associated to cardiovascular diseases (e.g., myocardial infarction, atherosclerosis, congenital cardiopathy, acquired cardiopathy, ischemic cardiopathy, hypertensive cardiopathy, valvulopathies, cardiomyopathies, blood disorders).

The biosafety of said compound has been clearly shown by means of the evaluation of its toxicity in a zebrafish embryo model in comparison with a commercial statin, simvastatin, observing that it is less toxic than simvastatin at different concentrations since a lower mortality occurs (Example 7, Figures 24 and 25). Additionally, it has been demonstrated that the lethal dose 50 (LD50) is higher in the case of compound NST0037 than in the case of simvastatin at all the evaluated time points, indicating a higher biosafety of said compound (Example 7, Figures 26). Additionally, it has been demonstrated that this compound causes a higher percentage of healthy larvae at the end of the experiment,

as well as a lower percentage of larvae with malformations or anomalous appearance (Example 7, Figures 27 and 28). Additionally, this compound does not cause a significant variation of the percentage of heartbeats at high concentrations, unlike simvastatin which causes a significant reduction of the cardiac rhythm at high concentrations (Example 7, Figures 29).

Therefore, one aspect of the present invention relates to a compound of formula (I) [also identified on occasions in this document as NST0037]:

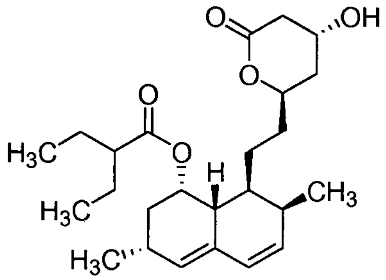

(I)

its hydroxy acid form, the pharmaceutically acceptable salts of said hydroxy acid and pharmaceutically acceptable solvates of the compound and of its hydroxy acid form.

Another aspect of the present invention is a pharmaceutical composition comprising a compound of formula (I) and/or its hydroxy acid form and/or a pharmaceutically acceptable salt of said hydroxy acid and/or a pharmaceutically acceptable solvate of the compound or of its hydroxy acid form, and at least one pharmaceutically acceptable adjuvant, carrier and/or vehicle.

Another aspect of the present invention relates to a compound of formula (I), its hydroxy acid form or a pharmaceutically acceptable salt of said hydroxy acid and/or a pharmaceutically acceptable solvate of the compound or of its hydroxy acid form for its use as medicament.

According to another aspect, the present invention relates to a compound of formula (I), its hydroxy acid form or a pharmaceutically acceptable salt of said hydroxy acid and/or a pharmaceutically acceptable solvate of the compound or of its hydroxy acid form for its use as a neuroprotective agent, in particular in the prevention and/or the treatment of:

a. neurodegenerative diseases (e.g., Alzheimer's, Parkinson's, multiple sclerosis, amyotrophic lateral sclerosis, *status epilepticus,* Huntington's), more specifically, apoptotic processes, oxidative stress or endoplasmic reticulum stress associated to said chronic neurodegenerative diseases,
b. cognitive deterioration,
c. age-associated pathological processes and progeria,
d. epilepsy, epileptic seizures and convulsions.

One aspect of the present invention relates to the use of a compound of formula (I), of its hydroxy acid form, of a pharmaceutically acceptable salt of said hydroxy acid and/or of a pharmaceutically acceptable solvate of the compound or of its hydroxy acid form in the manufacture of a medicament. According to a particular embodiment, the medicament is for being used as a neuroprotective agent, in particular in the prevention and/or the treatment of:

a. neurodegenerative diseases (e.g., Alzheimer's, Parkinson's, multiple sclerosis, amyotrophic lateral sclerosis, *status epilepticus,* Huntington's), more specifically, apoptotic processes, oxidative stress or endoplasmic reticulum stress associated to said chronic neurodegenerative diseases,
b. cognitive deterioration,
c. age-associated pathological processes and progeria,
d. epilepsy, epileptic seizures and convulsions.

The present invention discloses a compound of formula (I), its hydroxy acid form or a pharmaceutically acceptable salt of said hydroxy acid and/or a pharmaceutically acceptable solvate of the compound or of its hydroxy acid form for its use in increasing seladin-1/DHCR24 gene expression.

The present invention discloses a compound of formula (I), its hydroxy acid form or a pharmaceutically acceptable salt of said hydroxy acid and/or a pharmaceutically acceptable solvate of the compound or of its hydroxy acid form for its use in the prevention and/or treatment of diseases related to the seladin-1/DHCR24 gene.

The present invention discloses the use of a compound of formula (I), of its hydroxy acid form, of a pharmaceutically acceptable salt of said hydroxy acid and/or of a pharmaceutically acceptable solvate of the compound or of its hydroxy acid form in the manufacture of a medicament characterized by increasing seladin-1/DHCR24 gene expression.

Brief Description of the Figures

Figure 1 is an XY scatter chart depicting the protective effect of compound NST0037 against death caused by xanthine/xanthine oxidase (XXO). The figure shows the percentage of cell death (taking as 100% the cell death caused by XXO) of the cultures treated with 10 μM xanthine (X) 60 mU/mL xanthine oxidase (XO) and NST0037 at different concentrations, representing the mean±SD of 3 independent experiments in triplicate. *Significant difference with respect to the treatments with XXO alone, according to the Student's t test ($p<0.05$).

Figure 2 is an XY scatter chart depicting the protective effect of compound NST0037 against death caused by tunicamycin (Tm). The figure shows the percentage of cell death (taking as 100% the cell death caused by Tm) of the cultures treated with 24 μM Tm and NST0037 at different concentrations, representing the mean+SD of 3 independent experiments in triplicate. *Significant difference with respect to the treatments with Tm alone, according to the Student's t test ($p<0.05$).

Figure 3 is an XY scatter chart depicting the protective effect of compound NST0037 against death caused by camptothecin (CPT). The figure shows the percentage of cell death (taking as 100% the cell death caused by CPT) of the cultures treated with 20 nM CPT and NST0037 at different concentrations, representing the mean±SD of 3 independent experiments in triplicate. *Significant difference with respect to the treatments with CPT alone, according to the Student's t test ($p<0.05$).

Figure 4 are two bar graphs depicting the protective effect of compound NST0037 against apoptosis caused by camptothecin (CPT) determined by flow cytometry. Figure 4A shows the percentage of inhibition of apoptosis caused by 50 μM CPT and NST0037 at 10, 40 and 100 μM in comparison with the inhibition control, Z-VAD-fmk at 50 μM, representing the mean±SD of 3 independent experiments. *Significant difference with respect to the treatment with CPT alone, according to the Student's t test ($p<0.05$). Figure 4B shows that the pretreatment with NST0037 enhances the antiapoptotic effect of the compound (40 μM NST0037 and 24 hour treatment with 50 μM CPT as an apoptosis inducer), this protection being partially inhibited by adding mevalonate (MEV), a precursor of the cholesterol biosynthesis pathway and a product of the enzymatic reaction catalyzed by the HMG-CoA Reductase enzyme. Figure 4B shows the percentage of inhibition of the apoptosis caused by 50 μμM CPT with a 24 hour pretreament with 40 μM NST0037 alone or together with 100 μM mevalonate (MEV), and the effect of the inhibitor Z-VAD-fmk at 50 μM as a control, representing the means±SD of 3 independent experiments. *Significant difference with respect to the treatment with CPT alone, #significant difference with respect to the treatment with CPT and NST0037, according to the Student's t test ($p<0.05$).

Figure 5 is a micrograph composition showing the hippocampal CA1 and CA2 regions of mice in which the samples have been stained withhematoxylin and eosin. The figure depicts the histopathological analysis of the cell structure of these regions according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 6 is a bar graph in which the state of the temporal memory is analyzed according to the protocol by Dere et al. (Dere, E., Huston, J. P. & De Souza Silva, M. A. Neurobiol Learn Mem 2005; 84: 214-21). The bars represent the means±SEM of the temporal memory expressed in arbitrary units (y-axis), according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 7 is a bar graph in which the state of the spatial memory is analyzed according to the protocol by Dere et al. (Dere, E., Huston, J. P. & De Souza Silva, M. A. Neurobiol Learn Mem 2005; 84: 214-21). The bars represent the means±SEM of the spatial memory expressed in arbitrary units (y-axis), according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 8 is a Kaplan-Meier graph depicting the survival rate of mice according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 9 is a bar graph wherein the mean±SEM of the time is represented in minutes after the inoculation of KA in which the first convulsion (y-axis) occurs according to the pretreatment received (x-axis). The group of pretreatment with PBS is represented in black and the group of treatment with NST0037 at 50 mg/Kg by weight is represented

in gray.

Figure 10 is an XY scatter chart depicting the severity level of the *status epilepticus* observed according to Racine's scale (Racine, Electroencephalogr Clin Neurophysiol 1972; 32[3]:281-94) against the post-inoculation time of the epileptogenic substance (kainic acid or kainate or KA). The chart shows the evolution of the epileptogenic state of the animals according to the treatment received: PBS is represented with black circles and lines and the group of treatment with NST0037 at 50 mg/Kg by weight is represented with gray squares and lines.

Figure 11 is an XY scatter chart depicting the dose-response curves of compound NST0037 in comparison with two commercial statins (simvastatin and atorvastatin) on the HMGR enzyme activity *in vitro*. The chart shows the percentage of HMGR enzyme activity with respect to the control of the compounds at different doses, representing the mean$\pm$SD of at least four independent assays.

Figure 12 is a bar diagram depicting total plasma cholesterol levels of male ApoB100 mice 12 hours after being treated with 50 mg/kg of NST0037 or simvastatin, representing the mean$\pm$SD of each of the groups. *Significant difference with respect to time 0 hours, according to the Student's t test ($p<0.05$).

Figure 13 is a bar diagram depicting the plasma LDL cholesterol (LDL-c) levels of male ApoB100 mice 12 hours after being treated with 50 mg/kg of NST0037 or simvastatin, representing the mean$\pm$SD of each of the groups. *Significant difference with respect to time 0 hours, according to the Student's t test ($p<0.05$).

Figure 14 is a bar diagram depicting the plasma HDL cholesterol levels (HDL-c) of male ApoB100 mice 12 hours after being treated with 50 mg/kg of NST0037 or simvastatin, representing the mean$\pm$SD of each of the groups. *Significant difference with respect to time 0 hours, according to the Student's t test ($p<0.05$).

Figure 15 is a bar diagram depicting the plasma VLDL cholesterol levels (VLDL-c) of male ApoB100 mice 12 hours after being treated with 50 mg/kg of NST0037 or simvastatin, representing the mean$\pm$SD of each of the groups. *Significant difference with respect to time 0 hours according to the Student's t test ($p<0.05$).

Figure 16 is a bar diagram depicting the plasma esterified cholesterol (EC) levels of male ApoB100 mice 12 hours after being treated with 50 mg/kg of NST0037 or simvastatin, representing the mean$\pm$SD of each of the groups. *Significant difference with respect to time 0 hours according to the Student's t test ($p<0.05$).

Figure 17 is a bar diagram depicting the plasma free cholesterol (FC) levels of male ApoB100 mice 12 hours after being treated with 50 mg/kg of NST0037 or simvastatin, representing the mean$\pm$SD of each of the groups. *Significant difference with respect to time 0 hours according to the Student's t test ($p<0.05$).

Figure 18 is a bar diagram depicting the number of times the total cholesterol (TC) in male C57BL6 mice increases after 24 hours of being treated i.p. with 500 mg/kg of Triton 1339 (Tyloxapol) and 50 mg/kg of NST0037 or simvastatin, representing the mean$\pm$SD of each of the groups. *Significant difference with respect to the control group, according to the Student's t test ($p<0.05$).

Figure 19 is a bar diagram depicting the number of times the LDL cholesterol (LDL-c) in male C57BL6 mice increases after 24 hours of being treated i.p. with 500 mg/kg of Triton 1339 (Tyloxapol) and 50 mg/kg of NST0037 or simvastatin, representing the mean$\pm$SD of each of the groups. *Significant difference with respect to the control group, according to the Student's t test ($p<0.05$).

Figure 20 is a bar diagram depicting the number of times the HDL cholesterol (HDL-c) in male C57BL6 mice increases after 24 hours of being treated i.p. with 500 mg/kg of Triton 1339 (Tyloxapol) and 50 mg/kg of NST0037 or simvastatin, representing the mean$\pm$SD of each of the groups.

Figure 21 is a bar diagram depicting the VLDL cholesterol levels (VLDL-c) in male C57BL6 mice after 24 hours of being treated i.p. with 500 mg/kg of Triton 1339 (Tyloxapol) and 50 mg/kg of NST0037 or simvastatin, representing the mean$\pm$SD of each of the groups. *Significant difference with respect to the control group, according to the Student's t test ($p<0.05$).

Figure 22 is a bar diagram depicting the number of times the esterified cholesterol (EC) in male C57BL6 mice increases after 24 hours of being treated i.p. with 500 mg/kg of Triton 1339 (Tyloxapol) and 50 mg/kg of NST0037 or simvastatin, representing the mean$\pm$SD of each of the groups.

Figure 23 is a bar diagram depicting the number of times the free cholesterol (FC) in male C57BL6 mice increases after 24 hours of being treated i.p. with 500 mg/kg of Triton 1339 (Tyloxapol) and 50 mg/kg of NST0037 or simvastatin, representing the mean$\pm$SD of each of the groups.

Figure 24 is a Kaplan-Meier graph depicting the survival rate of the embryos-larvae according to the treatment received: control, NST0037 (at a dose of 2 mg/L) or simvastatin (at a dose of 2 mg/L). *Significant difference with respect to the control, according to the $\chi^2$ test ($p<0.01$).

Figure 25 is a Kaplan-Meier graph depicting the survival rate of the embryos-larvae according to the treatment received: control, NST0037 (at a dose of 0.2 mg/L) or simvastatin (at a dose of 2 mg/L). *Significant difference with respect to the control, according to the $\chi^2$ test ($p<0.01$).

Figure 26 is an XY scatter chart depicting the lethal dose 50 (LD50) of the two compounds (NST0037 or simvastatin) against the time of treatment.

Figure 27 is a bar graph depicting the percentage of healthy larvae which is obtained at the end of the experiment

according to the treatment received (NST0037 or simvastatin) and the doses used (0.02, 0.06 or 0.2 mg/L), and wherein the means ± SD are represented.

Figure 28 is an XY scatter chart depicting the percentage of embryos-larvae with malformations or anomalous appearance according to the treatment received (NST0037 or simvastatin). *Significant difference between the two treatments, according to the Student's t test ($p<0.05$).

Figure 29 is a bar graph depicting the percentage of the cardiac rhythm of the embryos-larvae treated with increasing doses of the compounds NST0037 or simvastatin, at 72 hours post-treatment, representing the means ± SD. The horizontal dotted black line represents the mean value of the cardiac rhythm corresponding to the controls. *Significant difference of the treatments with respect to the control, according to the Student's t test ($p<0.05$).

Figure 30 is a chart showing the antifungal activity of compound NST0037 and of simvastatin. The logarithm of the assayed concentrations (mM) is represented therein against the diameter of the inhibition halos (cm).

Figure 31 is a bar graph showing the increase of seladin-1/DHCR24 gene expression due to treatment with NST0037 in SK-N-MC cells. The relative quantification (RQ) of seladin-1/DHCR24 gene expression is shown, normalized by 18S, and referring to the value of the untreated cells (control), for the treatments for 24 h with NST0037 at 1, 4, 10 and 40 μM. The results of two independent assays in triplicate are shown. *Significant difference with respect to the control, according to the Student's t test ($p<0.05$).

Figure 32 is an XY scatter chart depicting the protective effect of the compound NST0037 against death caused by okadaic acid (OA). The figure shows the percentage of cell death (taking as 100% the cell death caused by OA) of the cultures treated with 20 nM OA and NST0037 at different concentrations, representing the means±SD of 3 independent experiments in triplicate. *Significant difference with respect to the treatments with OA alone, according to the Student's t test ($p<0.05$).

Figure 33 is an XY scatter chart depicting the protective effect of the compound NST0037 against death caused by 3-nitropropionic (3-NP) acid. The figure shows the percentage of cell death (taking as 100% the cell death caused by 3-NP) of the cultures treated with 30 μM 3-NP and NST0037 at different concentrations, representing the means±SD of 3 independent experiments in triplicate. *Significant difference with respect to the treatments with 3-NP alone, according to the Student's t test ($p<0.05$).

Figure 34 is a bar chart depicting the inhibitory effect of the pretreatment of compound NST0037 on the activation of caspase 3/7, induced by camptothecin (CPT). The figure shows the percentage of active caspase 3/7 referred to the control cells, without treatment, produced by 50 μM CPT and pretreatment with NST0037 at 10 and 40 μM, mevalonate at 100 μM and the combination of both compounds, furthermore, the inhibitor Z-VAD-fmk at 50 μM is used as inhibition control, representing the means±SD of 3 independent experiments. *Significant difference with respect to the treatment with CPT alone, #significant difference with respect to the treatment with CPT and NST0037, according to the Student's t test ($p<0.05$).

Figure 35 are two bar graphs showing the percentage of Aβ(1-40) (A) and Aβ(1-42) (B) secreted and quantified by means of ELISA and referred to the control cells at 48 hours. The results of a representative assay in duplicate (mean±SD) are shown. *Significant difference with respect to the control, according to the Student's t test ($p<0.05$).

Figure 36 is a bar graph depicting the effect of mevalonate on the protection by NST0037 against cell death caused by XXO. The figure shows the percentage of cell death (taking as 100% the cell death caused by XXO) of the cultures treated with 10 μM xanthine (X), 60 mU/mL xanthine oxidase (XO) and 40 μM NST0037 and mevalonate at 10, 40 and 100 μM, representing the means±SD of 3 independent experiments in triplicate. *Significant difference with respect to the treatment with XXO alone; #significant difference with respect to the treatment with XXO and NST0037, according to the Student's t test ($p<0.05$).

Figure 37 is a micrograph composition showing the immunohistochemistry of MAP2 in the hippocampus of mice with a magnification of 12.5X and in more detail of the CA1 and CA2-CA3 areas with a magnification of 200X. The figure depicts the histopathological analysis of the neuritic dystrophy according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 38 is a micrograph composition showing: (A) the hippocampal CA2 and CA3 region of mice in which HNE immunohistochemistry, the T.U.N.E.L. technique and GFAP immunohistochemistry have been performed on the samples, all the images have a magnification of 100X. The figure depicts the histopathological analysis of the oxidative damage, apoptosis and astrogliosis in the hippocampus according to the pretreatment (24 and 0.5 hours before the inoculation of the excitotoxic substance [kainic acid or kainate or KA]), the inoculation of the excitotoxic substance (0 days post-inoculation [d.p.i.]) and the subsequent treatment (up to 7 d.p.i.).

Figure 39 is a Kaplan-Meier graph depicting the survival rate of mice according to the treatment (24 and 0.5 hours before the inoculation of the neurotoxic substance [MPTP]), the inoculation of the neurotoxic substance (0 days post-inoculation [d.p.i.]) and the subsequent treatment (up to 7 d.p.i.).

Figure 40 is a bar graph in which the resistance of the animal is analyzed. The bars represent the means±SEM of the ratio between the time of stay of the animals in the cylinder at 7 d.p.i. with respect to the baseline state (Y axis),

according to the treatment (24 and 0.5 hours before the inoculation of the neurotoxic substance [MPTP]), the inoculation of the neurotoxic substance (0 days post-inoculation [d.p.i.]) and the subsequent treatment (up to 7 d.p.i.).

Figure 41 is a bar graph in which the strength in the front extremities of the animal is analyzed. The bars represent the means±SEM of the ratio of the strength of the animals in grams in quintuplicate at 7 d.p.i. with respect to the baseline state (Y axis), according to the treatment (24 and 0.5 hours before the inoculation of the neurotoxic substance [MPTP]), the inoculation of the neurotoxic substance (0 days as post-inoculation [d.p.i.]) and the subsequent treatment (up to 7 d.p.i.).

Figure 42 is a micrograph composition showing the substantia nigra and striatum regions of mice in which the samples have been stained with Fluoro Jade B with a magnification of 100X. The figure represents the histopathological analysis of the neurodegeneration according to the treatment (24 and 0.5 hours before the inoculation of the neurotoxic substance [MPTP]), the inoculation of the neurotoxic substance (0 days post-inoculation [d.p.i.]) and the subsequent treatment (up to 7 d.p.i.).

Figure 43 is a micrograph composition showing the substantia nigra and striatum regions of mice in which the immunohistochemistry against tyrosine-hydroxylase has been performed on the samples with a magnification of 100X. The figure represents the histopathological analysis of the death of dopaminergic neurons of the substantia nigra and of the disappearance of the nerve extensions of the striatum according to the treatment (24 and 0.5 hours before the inoculation of the neurotoxic substance [MPTP]), the inoculation of the neurotoxic substance (0 days post-inoculation [d.p.i.]) and the subsequent treatment (up to 7 d.p.i.).

Figure 44 is an XY scatter chart in which the resistance of the animal is analyzed. The bars represent the means±SEM of the ratio between the time of stay of the animals in the cylinder at 7, 14 and 21 d.p.i. with respect to the baseline state (Y axis) according to the treatment (24 and 0.5 hours before the inoculation of the neurotoxic substance [MPTP]), the inoculation of the neurotoxic substance (0 days post-inoculation [d.p.i.]) and the subsequent treatment (up to 21 d.p.i.).

Figure 45 is a micrograph composition showing the substantia nigra region of mice in which the immunohistochemistry against tyrosine-hydroxylase and HNE has been performed on the samples with a magnification of 100X. The figure represents the histopathological analysis of the death and the lipid peroxidation in dopaminergic neurons of the substantia nigra according to the treatment (24 and 0.5 hours before the inoculation of the neurotoxic substance [MPTP]), the inoculation of the neurotoxic substance (0 days post-inoculation [d.p.i.]) and the subsequent treatment (up to 21 d.p.i.).

Figure 46 is an XY scatter chart depicting the effective permeability expressed as $P_e$ (cm/s) against the BBB passage (%) of atorvastatin, simvastatin and NST0037 by means of *in vitro* determination by the PAMPA method. Verapamil and theophylline, respectively, were used as positive and negative controls.

Figure 47 is a bar diagram depicting the effect of simvastatin and NST0037 on the cholesterol levels in the human cell lines HepG2 and SK-N-MC. The results are expressed as the percentage of reduction of cholesterol with respect to the control in each line after the incubation of the acid compounds for 20 hours in the absence of FBS. The determinations were carried out by enzymatic and fluorometric means and the results are the mean±SD. *Significant difference with respect to the untreated cells, according to the Student's t test ($p<0.05$). In the case of HepG2, 2 independent assays were performed in triplicate and in the case of the SK-N-MC three independent assays were performed in triplicate.

Figure 48 is a set of XY scatter graphs depicting the plasma concentration of cholesterol and its various fractions in addition to the concentration of apoB in 12-week old female apoB100 mice after 7, 21 and 28 days of oral treatment with 50 mg/kg of NST0037 or simvastatin, representing the mean±SEM of each of the groups and the times. *Significant difference with respect to the control, according to the Student's t test ($p<0.05$). +Significant difference with respect to the initial time of that same group.

Figure 49 is a bar diagram depicting the number of times the free and esterified cholesterol increases in 12-week old female apoB100 mice after 7, 21 and 28 days of treatment with 50 mg/kg of NST0037 or simvastatin, representing the mean±SEM of each of the groups. *Significant difference with respect to the control group, according to the Student's t test ($p<0.05$).

Figure 50 is a bar diagram depicting the number of times the oxidation state increases in the plasma of 12-week old female apoB100 mice after 7, 21 and 28 days of treatment with 50 mg/kg of NST0037 or simvastatin, representing the mean±SEM of each of the groups. *Significant difference with respect to the control group, according to the Student's t test ($p<0.05$).

Figure 51 is a set of XY scatter graphs depicting the plasma concentration of cholesterol and its various fractions in 6-month old female apoB100 mice after one, two and three months of oral treatment with 50 mg/kg of NST0037 or simvastatin, representing the mean±SEM of each of the groups. *Significant difference with respect to the control, according to the Student's t test ($p<0.05$). +Significant difference with respect to the initial time of that same group.

Figure 52 is a bar diagram depicting the number of times the free and esterified cholesterol in 6-month old female apoB100 mice increases after one, two or three months of oral treatment with 50 mg/kg of NST0037 or simvastatin,

representing the mean±SEM of each of the groups. *Significant difference with respect to the control group, according to the Student's t test ($p<0.05$).

Figure 53 is a bar diagram depicting the number of times the cholesterol and its various fractions in 11-week old male Zucker rats increase after 7 days of oral treatment with 30 mg/kg of NST0037 or simvastatin, representing the mean±SEM of each of the groups. *Significant difference with respect to the control group, according to the Student's t test ($p<0.05$).

Figure 54 is a bar diagram depicting the number of times the plasma triglycerides in 11-week old male Zucker rats increase after 7 days of oral treatment with 30 mg/kg of NST0037 or simvastatin, representing the mean±SEM of each of the groups. *Significant difference with respect to the control group, according to the Student's t test ($p<0.05$).

Figure 55 is a bar diagram depicting the number of times the plasma redox state in 11-week old male Zucker rats increases after 7 days of oral treatment with 30 mg/kg of NST0037 or simvastatin, representing the mean±SEM of each of the groups. *Significant difference with respect to the control group, according to the Student's t test ($p<0.05$).

Figure 56 is a bar diagram depicting the number of times the seladin-1/DHCR24 gene expression in the brain of wild-type C57BL6 mice increases at 4 hours of the oral administration at 50 mg/kg of NST0037 or simvastatin, representing the mean±SEM of each of the groups. *Significant difference with respect to the control group, according to the Student's t test ($p<0.05$).

Figure 57 is a bar diagram depicting the percentage of healthy larvae (without toxicological problems) after the exposure of compound NST0037 in comparison with simvastatin, representing the mean of the percentage±SEM of healthy animals after the treatment with different doses of NST0037 or of simvastatin. The Y axis determines the percentage of healthy larvae and the X axis the concentrations used of both compounds. The gray bars represent the group of animals treated with NST0037 and the black bars represent the animals treated with simvastatin.

Figure 58 is a bar diagram depicting the percentage of larvae with anomalous appearance (symptomatology) after the exposure of compound NST0037 in comparison with simvastatin, representing the mean of the percentage±SEM of larvae with deformations or an abnormal appearance after the treatment with different doses of NST0037 or of simvastatin. The Y axis determines the percentage of larvae with anomalous appearance and the X axis the different alterations of the symptomatology. The gray bars represent the group of animals treated with NST0037 and the black bars represent the animals treated with simvastatin.

Figure 59 is a bar diagram depicting the variation of the weight of adult zebrafish in a single-dose toxicity assay (24 hours) by the exposure in water of compound NST0037 in comparison with simvastatin. The data are presented as the mean weight of the animals±SD according to the study time and to the treatment group. The white bars indicate the weight of the animals according to the treatment, referring to the baseline study (0 dpt). The gray graphs indicate the weight of the animals according to the treatment, referring to the study at 7 dpt. The black graphs indicate the weight of the animals according to the treatment, referring to the study at 14 dpt. *Statistical comparison with the Student's t-method, in which significant differences in the weight of the animals of one and the same group with respect to the baseline time are determined ($p < 0.05$).

Figure 60 is a micrograph composition showing the histopathological study in different adult zebrafish organs after a single-dose toxicity assay (24 hours) by the exposure in water of compound NST0037 in comparison with simvastatin. The animals were treated with a dose of 2000 mg/Kg, sacrificed at 14 days post-treatment, representative histological sections of the different study groups were made and stained with hematoxylin-eosin. The studied organs which are shown: brain, kidney, pancreas, intestine, eye, gills, ovary, testicle, muscle and liver.

Figure 61 is a micrograph composition showing the histopathological study in the ovary of adult zebrafish after a lethality assay by the constant exposure in water (4 days) of compound NST0037 in comparison with simvastatin. The animals were treated with two doses of 32 and 100 mg/Kg, sacrificed at 4 days post-treatment, representative histological sections of the different study groups were made and stained with hematoxylin-eosin. The ovary was the only studied organ which experienced pathological alterations. A histological section of the ovary of a female from the control group, and ovaries of females from the groups of treatments with NST0037 and simvastatin according to the dose are shown.

## Detailed Description of the Invention

### Definitions

To aid in understanding the invention object of this document, the meaning of some terms and expressions used in the context of the invention is explained below.

The term "neuroprotective agent", as it is used herein, relates to any substance capable of causing the attenuation or disappearance of the effects of neuronal degeneration or death by means of any mechanism known or to be known, for example, necrosis, apoptosis, autophagia, oxidative damage, excitotoxicity, endoplasmic reticulum damage, deposition of byproducts, loss of cell architecture, or to the reduction or disappearance of the side effects thereof.

The term "statin", as it is used herein, relates to an inhibitor of the 3-hydroxy-3-methylglutaryl-coenzyme A reductase (HMGR) enzyme, which catalyzes the limiting step of cholesterol biosynthesis and includes any natural, synthetic or semisynthetic statin. Some statins can be in the closed form (lactone) or in the open form (hydroxy acid). Hydroxy acids (open form) can be prepared from the corresponding lactones by conventional hydrolysis, for example, with sodium hydroxide in methanol, sodium hydroxide in tetrahydrofuran-water and the like. In the open form (hydroxy acid), the statins react to form salts with pharmaceutically acceptable metal and amine cations formed from organic or inorganic bases. The pharmaceutically acceptable salts of the statins can differ from the corresponding free acids in some physical characteristics such as solubility and melting point, but they are considered equivalent to the free acid form for the purposes of this invention. The free open form (hydroxy acid) of the statins can be regenerated from the salt form, if desired, by contacting the salt with a diluted aqueous solution of an acid such as hydrochloric acid and the like. The closed form (lactone) of the statins can be regenerated by dissolving the open form (hydroxy acid) in an inert solvent such as, for example, toluene, benzene, ethyl acetate and the like, at temperatures comprised between approximately 0°C and approximately the boiling point of the solvent, typically (although not necessarily) with simultaneous separation of the resulting water and catalysis with strong acids, e.g., hydrochloric acid and the like. Likewise, the statins can exist in a solvated or non-solvated form and such forms are equivalent to the non-solvated form for the purposes of this invention.

The term "cardioprotective", as it is used herein, relates to any substance capable of causing the attenuation or disappearance of the underlying effects of cardiovascular diseases or cardiopathies or of cardiac damage by means of any mechanism known or to be known, for example, necrosis, apoptosis, ischemia, arrhythmia, deposition of byproducts, loss of cell architecture, or to the reduction or disappearance of the side effects thereof.

The term "hypolipidemic", as it is used herein, relates to any pharmacologically active substance having the property of reducing blood lipid levels or lipid levels in other tissues. The importance of these substances is due to the fact that the excess of some types of lipids (cholesterol or triglycerides) or lipoproteins is one of the main risk factors for cardiovascular diseases.

The term "hypocholesterolemic", as it is used herein, relates to any pharmacologically active substance having the property of reducing blood cholesterol levels or cholesterol levels in other tissues.

The term "hypotriglyceridemic", as it is used herein, relates to any pharmacologically active substance having the property of reducing blood triglyceride levels or triglyceride levels in other tissues.

The term "antiepileptic or anticonvulsant", as it is used herein, relates to the attenuation of epileptic or convulsive seizures, for example, in the duration and/or in the intensity, or to the disappearance of epileptic or convulsive seizures, or to the reduction or disappearance of the side effects thereof.

The term "biosafe" as it is used herein, relates to the absence of toxic effects, generation of tumors, alterations in embryologic development (teratogenesis) or other adverse effects.

The term "neurodegenerative disease", as it is used herein, includes diseases which result from the degeneration or deterioration of nervous tissue, particularly of neurons, leading over time to a dysfunction or to a disability; the term degeneration includes loss of cell viability, loss of cell function and/or loss of the number of cells (neurons or others). Illustrative, non-limiting, examples of neurodegenerative diseases include Alzheimer's disease, Huntington's disease, Parkinson's disease, amyotrophic lateral sclerosis (ALS), multiple sclerosis. In a particular embodiment, said neurodegenerative disease is a disease related to neuronal death caused by a substance which, for example, causes oxidative stress or endoplasmic reticulum stress or apoptosis or excitotoxicity or neuronal death in general.

The term "disease associated with undesired oxidation", as it is used herein, relates to a disease caused by undesired oxidation (e.g., excessive oxidation) or in which said undesired oxidation is a symptom. Said undesired oxidation can be a result of the damage caused by free radicals on proteins, DNA and/or lipids independently from the specific free radical involved or from the target. Undesired oxidation involves an excessive generation of free radicals which can cause a dysfunction in cells, tissues or organs and can therefore form a potential mechanism of a disease. In a particular embodiment, said undesired oxidation can be caused by age (aging) or by a neurodegenerative process and can cause by itself or in combination with other factors the onset of several diseases. In a specific embodiment, said undesired oxidation relates to the oxidative damage caused by a substance which causes oxidative stress.

The term "age-associated pathological process", as it is used herein, relates to any age-related event or combination of events causing loss of cell viability of the nervous tissue or cell sensitization of the nervous tissue, loss of cell function and/or loss of the number of cells (neurons or others), including cell metabolic dysfunction, stress processes, infections by pathogens, genetic alterations, genetic susceptibility, trauma, ischemia, epilepsy.

The term "cardiovascular disease", as it is used herein, relates to any disease or dysfunction or alteration of the heart or of the rest of the cardiovascular system or of the blood.

The term "epilepsy", as it is used herein, relates to a chronic brain syndrome having varied causes, characterized by recurrent seizures due to excessive hypersynchronic discharges of nervous impulses by the brain neurons, associated eventually with several clinical and paraclinical manifestations. The seizures can be convulsive or non-convulsive. Epilepsy can have many causes; in some cases it can be due to different types of brain injuries (e.g., brain traumas,

sequelae of meningitis, tumors); in other cases there is no injury but a genetic predisposition to seizures; in other cases, the etiology of the epilepsy can be environmental, due to pharmacological treatments, due to excitotoxicity, trauma, stress processes, aging, development problems, neurological diseases, psychological crises, problems during gestation, problems during labor.

The term "epileptic or convulsant", as it is used herein, relates to any epileptic seizure or convulsion of any etiology, for example, genetic, environmental, due to pharmacological treatments, due to excitotoxicity, due to trauma, due to stress processes, due to aging, due to development problems, due to neurological diseases, due to psychological crises, due to problems during gestation, due to problems during labor. An epileptic seizure occurs when an abnormal electrical activity in the brain causes an involuntary change of body movement or function, feeling, in the capacity of being alert or in behavior, and can be partial or generalized (convulsive or non-convulsive).

The term "cognitive deterioration", as it is used herein, relates to the loss or alteration of mental functions, such as memory, orientation, language, visual recognition or conduct which interfere with the social activity and interaction of the person affected persistently over time.

The term "fungal or viral infections", as it is used herein, relates to any colonization of a microscopic fungus or virus which is harmful for the normal functioning or for the survival of the colonized organism or host.

The term "subject", as it is used herein, relates to a member of a mammal species and includes but is not limited to domestic animals, primates and humans; preferably, the subject is a male or female human being of any age or race. In a particular embodiment, said subject is a mammal which suffers, or is susceptible of suffering, age-associated pathological processes, such as aging, or a neurodegenerative disease, such as a chronic neurodegenerative disease.

The term "pharmaceutically acceptable", as it is used herein, relates to the fact that the compound is physiologically tolerable and generally does not cause an allergic reaction or a similar unfavorable reaction, such as a gastric disorder, dizziness or the like, when administered to a subject; said term "pharmaceutically acceptable" preferably means approved by a government regulatory agency or listed in the United States Pharmacopoeia or in another generally recognized pharmacopoeia for use in animals (e.g., European Pharmacopoeia).

The term "pharmaceutically acceptable salt", as it is used herein, includes "pharmaceutically acceptable metal salts" as well as "pharmaceutically acceptable amine salts". The term "pharmaceutically acceptable metal salt" contemplates salts formed with sodium, potassium, calcium, magnesium, aluminum, iron or zinc ions. The term "pharmaceutically acceptable amine salt" contemplates salts with ammonia and organic nitrogen bases strong enough to form salts with carboxylic acids. Said pharmaceutically acceptable salts can be obtained by conventional methods known by persons skilled in the art.

The compound (1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthalenyl-2-ethyl-butyrate can be obtained by semisynthesis methods, such as those described for other compounds of the same family in United States patent US 4866090.

A number of assays performed by the inventors have clearly shown the neuroprotective effect of compound NST0037 against the action of a substance causing oxidative stress, as well as its neuroprotective effect against the action of a substance causing endoplasmic reticulum stress, and its neuroprotective effect against the action of a substance causing apoptosis in human cholinergic neurons, as well as its neuroprotective effect against a substance causing excitotoxicity, oxidative damage, apoptosis, hippocampal atrophy, neuronal death, cognitive deterioration, temporal memory loss, spatial memory loss.

The neuroprotective effect against the action of a substance causing oxidative stress, such as xanthine/xanthine oxidase (XXO), is described in Example 2. It is observed in said example that compound NST0037 is capable of significantly and quantitatively reducing neuronal death caused by oxidative stress, which clearly shows the neuroprotective capacity of this compound (Figure 1). For the purpose of better defining the neuroprotective effect of the compound, the inventors analyzed the neurodegenerative process with greater detail by means of the analysis of neuronal death caused by the action of a substance causing endoplasmic reticulum stress (tunicamycin), determining that compound NST0037 is capable of significantly and quantitatively reducing neuronal death caused by endoplasmic reticulum stress, which clearly shows the neuroprotective capacity of said compound (Figure 2). For the purpose of better defining the neuroprotective effect of the compound, the inventors analyzed the neurodegenerative process with greater detail by means of the analysis of neuronal death caused by the action of a substance causing apoptosis (camptothecin), determining that compound NST0037 is capable of significantly and quantitatively reducing neuronal death caused by apoptosis, which clearly shows the neuroprotective capacity of said compound (Figure 3). Likewise, for the purpose of better defining the neuroprotective effect of the compound the inventors analyzed the neurodegenerative process with greater detail by means of flow cytometry analysis of neuronal death caused by apoptosis and its inhibition by said compound in comparison with a specific inhibitor of neuronal death by apoptosis, Z-VAD-fmk, determining that compound NST0037 is capable of significantly and quantitatively inhibiting neuronal death caused by apoptosis and that this neuroprotective effect is enhanced with the pretreatment of compound NST0037 and that it is partially dependent on cholesterol biosynthesis (Figure 4) .

For the purpose of better defining the neuroprotective effect of the compound, the inventors analyzed the

neurodegenerative process with greater detail by means of the analysis of neuronal death caused by an excitotoxic substance (kainate) in the hippocampal neurons of mice, as described in Example 3. It is observed in said example that compound NST0037 is capable of significantly and quantitatively reducing neuronal death caused by an excitotoxic substance, which clearly shows the neuroprotective capacity of said compound (Figure 5). It is demonstrated in said chart that the treatment with NST0037 administered before and after, or only after, the administration of kainate causes protection of the hippocampal neurons, inhibiting neuronal death in the CA1 and CA2 areas. In contrast, the administration of compound NST0037 alone does not cause any considerable histopathological change, showing no noteworthy differences with the control group. Likewise, as is known, the death of hippocampal neurons induces the cognitive deterioration of the animal in some cases, affecting memory processes; for this reason, the inventors analyzed if the neuroprotective effect of the compound was accompanied by a reduction of the temporal and spatial memory loss caused by an excitotoxic substance. It is demonstrated in the obtained results on the temporal memory (Figure 6) that the treatment with NST0037 administered before and after, or only after, the administration of kainate causes protection against the loss of this type of memory. It is demonstrated in the obtained results on the spatial memory (Figure 7) that the treatment with NST0037 administered before and after, or only after, the administration of kainate causes protection against the loss of this type of memory. In contrast, the administration of compound NST0037 alone does not cause any noteworthy change in the cognitive state of the animals, showing no considerable differences with the control group. Likewise, as is known, the administration of an excitotoxic substance and neuronal death induces the death of the animal in some cases; for this reason, the inventors analyzed if the neuroprotective effect of compound NST0037 was accompanied by a reduction of the mortality caused by an excitotoxic substance. It is demonstrated in the obtained results (Figure 8) that the treatment with NST0037 administered before and after, or only after, the administration of kainate causes a higher survival rate, indicating that the treatment with said compound protects the animals from death and against the rest of the organic effects caused by an excitotoxic substance. In contrast, the administration of compound NST0037 alone does not cause any noteworthy variation on the survival rate of the animals, showing no considerable differences with the control group.

Likewise, as is known, the administration of an excitotoxic substance induces convulsive seizures and epilepsy in the animal in some cases; for this reason, the inventors analyzed if the neuroprotective effect of compound NST0037 was accompanied by an antiepileptic and anticonvulsant effect caused by an excitotoxic substance (Example 4), observing that the administration of NST0037 delayed the time of onset of the first convulsion (latency) (Figure 9), furthermore causing a reduction in the severity and frequency of the epileptic symptoms (Figure 10), which demonstrates the antiepileptic or anticonvulsant effect of compound NST0037.

Furthermore, and due to the nature of compound NST0037, the inhibitory capacity of the 3-hydroxy-3-methylglutaryl coenzyme A reductase (HMGR) enzyme was studied. Surprisingly, and as is shown in Example 5, the results demonstrate that compound NST0037 has an evident hypocholesterolemic effect. It can be seen in the obtained results and as shown in Figure 11 that the inhibitory activity of compound NST0037 on the HMGR enzyme is within the range of two of the commercial statins (atorvastatin and simvastatin) commonly used to reduce cholesterol levels in the human population. It is demonstrated here that compound NST0037 exerts an inhibitory effect on said enzyme very similar to the statin with proven higher activity (atorvastatin), and inhibits the HMGR enzyme 7.5-fold more than the safest statin (simvastatin).

For the purpose of better defining the hypocholesterolemic effect of the compound, the inventors analyzed the hypocholesterolemic activity with greater detail by means of the analysis of the variations of the plasma cholesterol fractions in mice with endogenous hypercholesterolemia, as described in Example 5. To that end, the effect of compound NST0037 and of simvastatin was compared in two groups of mice, the total cholesterol levels being determined after the administration of the compounds (Figure 12), in which it was surprisingly observed that both compounds caused a similar hypocholesterolemic effect. For the purpose of better defining the hypocholesterolemic effect of the compound, the cholesterol levels in the LDL, HDL and VLDL fractions were analyzed, being determined that both compounds similarly reduce cholesterol levels in the LDL and VLDL fractions, but not in the HDL fraction (Figures 13 to 15), which demonstrates a hypocholesterolemic and cardioprotective effect. For the purpose of better defining the hypocholesterolemic effect of the compound, the free and esterified cholesterol levels were analyzed, being determined that both compounds similarly reduce the esterified cholesterol levels, but not the free cholesterol levels (Figure 16 to 17), which shows a hypocholesterolemic and cardioprotective effect.

For the purpose of better defining the hypocholesterolemic effect of the compound, the inventors analyzed the hypocholesterolemic activity with greater detail by means of the analysis of the variations of plasma cholesterol fractions in mice with induced hypercholesterolemia, as is described in Example 6. To that end, the effect of compound NST0037 and of simvastatin was compared in two groups of mice, the total cholesterol levels being determined after the administration of the compounds (Figure 18), in which it was surprisingly observed that compound NST0037 had a higher hypocholesterolemic effect than simvastatin. For the purpose of better defining the hypocholesterolemic effect of the compound, the cholesterol levels in the LDL, HDL and VLDL fractions were analyzed, being determined that compound NST0037 more effectively reduces cholesterol levels in the LDL fraction than simvastatin, neither altering cholesterol

levels in the HDL fraction (like simvastatin) or in the VLDL fraction (unlike simvastatin) (Figures 19 to 21), which demonstrates a hypocholesterolemic and cardioprotective effect. For the purpose of better defining the hypocholesterolemic effect of the compound, the free and esterified cholesterol levels were analyzed, being determined that in both cases NST0037 reduces both cholesterol fractions more effectively than simvastatin (Figures 22 and 23), which demonstrates a hypocholesterolemic and cardioprotective effect.

In order for a compound to be administered to the human population, it is necessary that its innocuousness and safety be demonstrated. For this purpose, the inventors analyzed the biosafety of compound NST0037 in a widely used toxicological model, the zebrafish embryo, following the methodology described in the OECD C15 protocol, as described in Example 7. In this model, the inventors compared the effect of the compound with simvastatin at concentrations greater than the doses used in clinical practice for the purpose of causing evident damage in the embryos in order to be able to better define the adverse effects of said compound. Thus, the administration of a high dose of NST0037 caused the mortality of all the embryos of the assay, as occurred with the same dose of simvastatin, although the latter was more toxic, since the reduction of the survival rate started earlier in time (Figure 24). When a dose that was 10 times lower was used, simvastatin caused a significant reduction of the survival rate in comparison with the controls, while NST0037 did not cause a statistically significant reduction of the survival rate of the embryos, indicating its higher safety (Figure 25). For the purpose of better defining the biosafety of the compound, the lethal doses 50 ($LD_{50}$) were studied at different time points with treatments of NST0037 or of simvastatin in a semistatic method, observing that at all times the $LD_{50}$ of simvastatin were lower than those of NST0037, indicating higher biosafety of the latter compound (Figure 26). For the purpose of better defining the biosafety of the compound, the percentage of healthy larvae at the end of the experiment was studied in comparison with simvastatin, a higher number of healthy larvae being observed in the treatments with NST0037 in comparison with simvastatin (Figure 27). For the purpose of better defining the biosafety of the compound, the percentage of larvae with malformations or with an anomalous appearance over time was studied in comparison with simvastatin, a lower number of larvae with malformations or with an anomalous appearance being observed in the treatments with NST0037 in comparison with simvastatin (Figure 28). For the purpose of better defining the biosafety of the compound, the percentage of heartbeats according to the doses used was studied, comparing NST0037 with simvastatin, a higher reduction of the cardiac rhythm being observed in the highest evaluated dose of simvastatin than in that of NST0037, whereas at lower doses only simvastatin caused a statistically significant reduction of the cardiac rhythm (Figure 29), indicating a higher biosafety of compound NST0037.

The antifungal activity of compound NST0037 was also studied by means of bioassay against statins (lovastatin, atorvastatin and simvastatin). The obtained results showed that compound NST0037 was the only one capable of causing inhibition halos in the entire assayed concentration range, even at the lowest concentrations (Figure 30), indicating a higher antifungal activity.

In addition, the capacity of compound NST0037 to increase seladin-1/DHCR24 gene expression was studied since the neuroprotective effects of the increased expression of this gene against Alzheimer's disease have been demonstrated (Cechi et al., J. Cell. Mol. Med. 2008; 12: 1990-2002). It has been described (Greeve et al., J. Neurosci. 2000; 20: 7345-52) that the product of the seladin-1/DHCR24 gene exerts its neuroprotective power by means of the antiapoptotic effect by inhibition of caspase-3, and regulating cholesterol synthesis from desmosterol, which determinates the generation of a barrier against the neurotoxic injuries and prevents the production of β-amyloid. These mechanisms of action indicate that the increase of seladin-1/DHCR24 gene expression has a general neuroprotective effect, therefore those drugs that cause an increase of the expression of this gene can potentially be used in the prevention and/or treatment of neuronal death associated to neurodegenerative diseases (e.g., Alzheimer's, Parkinson's, multiple sclerosis, amyotrophic lateral sclerosis, *status epilepticus,* Huntington's) or of diseases associated with undesired oxidation or of age-associated pathological processes. The capacity of increasing seladin-1/DHCR24 gene expression by means of the administration of NST0037 is described in Example 9, in comparison with memantine (one of the drugs normally used to treat AD). It is observed in said example that compound NST0037 is capable of increasing quantitatively, significantly and in a dose-dependent manner the seladin-1/DHCR24 gene expression, which is demonstrated both by means of gene expression analysis using microarray technology and by means of relative expression analysis using real time quantitative PCR. Said results clearly show the neuroprotective capacity of compound NST0037 (Figure 31). Additionally, the increase of seladin-1/DHCR24 gene expression and the increase in the amount of the protein coded by this gene have also been corroborated in a parallel study using simvastatin. The results indicate that this statin is also capable of increasing the expression of this gene, thus explaining its neuroprotective capacity, and indicating that it refers to a general mechanism of the statin family which is capable of increasing seladin-1/DHCR24 gene expression.

Additionally, the neuroprotective effect in human neuronal cultures of compound NST0037 against aggressions which mimic some neurodegenerative diseases, such as AD by means of the inhibition of protein phosphatase 1 (Figure 32) or Huntington's disease by means of the inhibition of succinate dehydrogenase (Figure 33), was studied. Furthermore, the effect on the modulation of effector caspases 3/7 was studied, it being determined that compound NST0037 prevents the activation thereof, and that this effect is related to the cholesterol biosynthesis pathway (Figure 34). Furthermore, it was determined that compound NST0037 is capable of reducing Aβ(1-40) and Aβ(1-42) levels in a human neuronal cell

model which overexpresses APP protein (Figure 35). Furthermore, it was determined that the neuroprotective effect of NST0037 against death caused by oxidative stress is modulated by mevalonate, which is a precursor of the cholesterol biosynthesis pathway and the product of the enzymatic reaction catalyzed by the HMG-CoA reductase enzyme (Figure 36).

For the purpose of better defining the neuroprotective effect of the compound, the inventors analyzed the neurodegenerative process with greater detail by means of the analysis of the histopathological signs associated with neuronal death caused by an excitotoxic substance (kainate) in the hippocampal neurons of mice, as described in Example 15. In said example it is observed that compound NST0037 is capable of preventing or improving neuritic dystrophy (Figure 37) as well as oxidative damage, apoptosis and astrogliosis (Figure 38) caused by the administration of an excitotoxic substance.

For the purpose of better defining the neuroprotective effect of the compound, the inventors analyzed the potential therapeutic effect of the treatment with NST0037 in a model of acute Parkinson's disease in mice as described in Example 16. To that end, and after the acute administration of a dopaminergic neuron-specific parkinsonian neurotoxin (MPTP), it was observed that NST0037 was capable of modifying the deleterious effects caused by the neurotoxin such as mortality (Figure 39), the locomotor deficit in relation to the parameters of resistance (Figure 40) and strength (Figure 41), neurodegeneration (Figure 42) in addition to the loss of dopaminergic neurons (Figure 43) in regions involved in Parkinson's disease such as the substantia nigra or the striatum.

For the purpose of better defining the neuroprotective effect of the compound, the inventors analyzed the potential therapeutic effect of the treatment with NST0037 in a model of subchronic Parkinson's disease in mice as described in Example 17. To that end, and after the subchronic administration of a dopaminergic neuron-specific parkinsonian neurotoxin (MPTP), it was observed that NST0037 was capable of modifying the deleterious effects caused by the neurotoxin such as the locomotor deficit in relation to the motor resistance (Figure 44), neuronal death or oxidative damage associated with lipid peroxidation (Figure 45) in the substantia nigra.

For the purpose of better defining the blood-brain barrier passage of compound NST0037, the inventors analyzed different parameters such as the theoretical lipophilicity, the percentage of passage and the effective permeability (Figure 46, Table I) as described in Example 18.

For the purpose of better defining the hypocholesterolemic effect of the compound, the inventors analyzed the hypocholesterolemic activity with greater detail by means of the analysis of the reductions of cholesterol in two human cells lines of hepatic and neuronal origin (Figure 47), as described in Example 19. Furthermore, the oral treatment for 28 days of compound NST0037 in mice with familial hyperlipidemia caused a decrease of the total cholesterol, ApoB, LDL-c, VLDL-c, HDL-c (Figure 48), free and esterified cholesterol (Figure 49) levels and of the plasma oxidation state (Figure 50), as described in Example 20. Furthermore, the oral treatment for 3 months of compound NST0037 in mice with familial hyperlipidemia caused a decrease of the total cholesterol, LDL-c levels and an increase of HDL-c (Figure 51), and a decrease of the free and esterified cholesterol levels (Figure 52), as described in Example 21.

For the purpose of better defining the hypocholesterolemic effect of the compound, the inventors analyzed the hypocholesterolemic activity with greater detail by means of reductions of cholesterol and of the associated fractions (Figure 53), of triglycerides (Figure 54), and of the plasma redox state in Zucker rats with endogenous hyperlipidemia (Figure 55), as described in Example 22.

For the purpose of better defining the modulation of the seladin-1/DHCR24 gene by compound NST0037, the inventors analyzed its regulation in the brain of mice treated orally with NST0037 (Figure 56), as described in Example 18, demonstrating that at 4 hours of the administration of NST0037 there is an increase in the expression of this neuroprotective gene.

For the purpose of better defining the innocuousness and safety of compound NST0037 and to corroborate the studies performed in Example 7 with zebrafish embryo, the inventors decided to analyzed the biosafety of compound NST0037 in larvae as described in Example 24. In this model, the inventors compared the effect of the compound in comparison with simvastatin by making increasing concentration curves, which revealed a high safety of the compound since there was no mortality with any of the two treatments (Table II), and where simvastatin produced a smaller percentage of healthy larvae than NST0037 (Figure 57) and a larger percentage of larvae with anomalous appearance (Figure 58).

For the purpose of better defining the innocuousness and safety of compound NST0037 and to corroborate the studies performed in previous examples, the inventors decided to analyze the biosafety of NST0037 after treatment in adult fish in comparison with simvastatin, as described in Example 25. The results indicated that while simvastatin causes a significant weight loss in the animals, NST0037 does not significantly vary said parameter (Figure 59). Furthermore, while simvastatin causes histopathological variations in the treated animals, NST0037 cause fewer deleterious effects (Figure 60).

For the purpose of better defining the innocuousness and safety of the compound NST0037 and to corroborate the studies performed in previous examples, the inventors decided to analyze the biosafety of NST0037 after treatment in adult fish in comparison with simvastatin, as described in Example 26. The results indicated that while simvastatin

caused a significant mortality at 4 days of treatment, the mortality associated with NST0037 was residual (Table III). Furthermore, while simvastatin caused clear histopathological variations in the ovary of the animals treated with 100 mg/Kg, NST0037 did not cause any deleterious effect at this dose (Figure 63).

The pharmaceutical composition provided by this invention can contain compound NST0037, and/or its hydroxy acid form and/or a pharmaceutically acceptable salt of said hydroxy acid and/or a pharmaceutically acceptable solvate of the compound or of its hydroxy acid form together with one or more pharmaceutically acceptable adjuvants, vehicles or excipients.

The term pharmaceutically acceptable "salt or solvate" relates to any pharmaceutically acceptable salt, solvate or any other compound which is capable of providing (directly or indirectly) a compound as has been described in the present invention in its administration to the recipient. Nevertheless, pharmaceutically unacceptable salts also fall within the scope of the invention, since the latter can be useful for the preparation of pharmaceutically acceptable salts. The salts can be prepared by means of methods known in the state of the art.

The compounds according to the invention can be in a crystalline form or as free compounds or as solvates (for example, hydrates) and it is intended that both forms are within the scope of the present invention. Solvation methods are generally known in the state of the art. In a particular embodiment the solvate is a hydrate.

The pharmaceutical compositions containing compound NST0037, or a hydroxy acid form thereof or a pharmaceutically acceptable salt of said hydroxy acid, can be formulated in any pharmaceutical dosage form suitable for its administration by the chosen administration route, e.g., oral, parenteral (subcutaneous, intramuscular, intravenous, intraperitoneal route), topical, rectal route. By way of a non-limiting illustration, the pharmaceutical compositions provided by this invention can be formulated in a solid pharmaceutical dosage form administered by the oral route (e.g., granules, tablets, capsules), in a liquid pharmaceutical dosage form administered by the oral route (e.g., solutions, suspensions, emulsions), in a pharmaceutical dosage form administered by the parenteral route (e.g., solutions, suspensions, emulsions). To that end, in each case, the suitable pharmaceutically acceptable vehicles and excipients will be chosen for the chosen pharmaceutical dosage form and route of administration, for example, binding agents, diluents, disintegrating agents, lubricants, wetting agents, for the formulation of solid pharmaceutical dosage forms, and buffers, surfactants, for the formulation of liquid pharmaceutical dosage forms. Said vehicles and excipients must be pharmaceutically acceptable and pharmacologically tolerable and have to be able to be combined with other components of the formulation without exerting any adverse effect on the subject treated. Information on said vehicles and excipients, as well as on said pharmaceutical dosage forms of said active ingredient can be found in Galenic Pharmacy treatises. A review of the different pharmaceutical dosage forms of drugs, in general, and of their methods of preparation can be found in the book "Treated de Farmacia Galénica" ("Galenic Pharmacy Treatise"), by C. Faulí i Trillo, 1st Edition, 1993, Luzán 5, S.A. de Ediciones.

The pharmaceutical composition provided by this invention comprises, compound NST0037, or a hydroxy acid form thereof or a pharmaceutically acceptable salt of said hydroxy acid, in a therapeutically effective amount. In the way used in this description, the expression "therapeutically effective amount" relates to the amount of compound calculated to cause the desired effect. The dose of compound NST0037, or a hydroxy acid form thereof or a pharmaceutically acceptable salt of said hydroxy acid, to be administered to a subject can vary within a wide range depending on a number of factors, including the characteristics of the compound used, e.g., its biological half-life and activity, the concentration of the compound in the pharmaceutical composition, the clinical situation of the subject, the severity of the pathology, the chosen pharmaceutical dosage form. The pharmaceutical composition provided by this invention can be administered one or more times a day for preventive or therapeutic purposes or, alternatively, others administration regimens can be followed, not necessarily daily but also at precise times, weekly.

If desired, the pharmaceutical composition provided by this invention can be used together with other drugs, for example, drugs useful in the treatment of neurodegenerative diseases, cognitive deterioration, diseases associated with undesired oxidation, age-associated pathological processes and progeria, epilepsy, epileptic seizures, convulsions, cardiovascular diseases, or fungal or viral infections for the purpose of increasing the efficacy of the pharmaceutical composition provided by this invention, a combination therapy thus being generated. Said additional drugs can form part of the same pharmaceutical composition or, alternatively, can be provided as a separate pharmaceutical composition for its administration at the same time (simultaneous administration) as the pharmaceutical composition provided by this invention or at different times (sequential administration) with respect to the administration of the pharmaceutical composition provided by this invention.

The following examples serve to illustrate the invention.

## EXAMPLE 1

**Synthesis of (1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthalenyl-2-ethyl-butyrate**

The compound identified as NST0037 was prepared following the methodology described in Hoffman, et al. (J. Med. Chem., 1986, 29, 849-852) for similar compounds.

1.1. Purification of lovastatin

Lovastatin was purified from an extract of natural origin by column chromatography using a hexane and ethyl acetate gradient as eluent.

1.2. Obtaining monacolin J

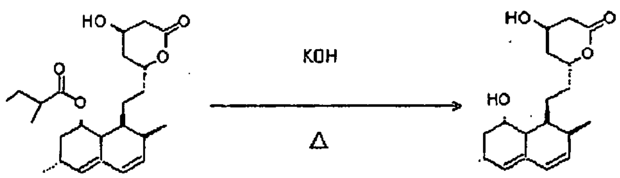


A solution of 0.7 g of potassium hydroxide in 0.5 ml of water is prepared and 3 ml of methanol are added little by little. 0.5 g of Lovastatin are subsequently added and the solution is placed under reflux for 21 hours. After the treatment of the reaction, a 50% mixture of monacolin J and the opened product is obtained.

1.3. Preparation of protected derivative

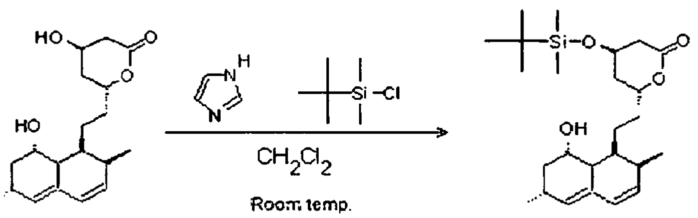


A solution of 0.5 g of Monacolin J in 10 ml of dichloromethane is prepared. 0.4345 g of imidazol are added and it is stirred until dissolution. Then 0.4835 g of tert-butyl-dimethylsilane chloride dissolved in 5 ml of dichloromethane are added, and stirring is continued for 24 hours. The reaction is followed by TLC using dichloromethane-methanol (10:1) as eluent. Yield: 96 %.

1.4. Preparation of the acylated derivative

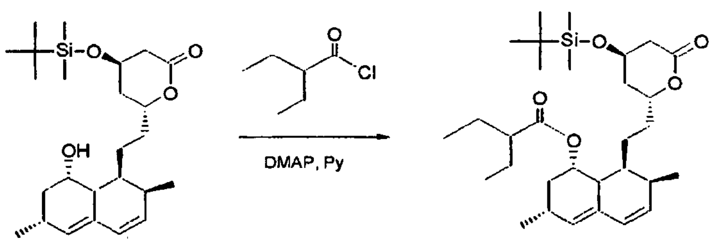

0.3 g of the protected derivative previously obtained are dissolved in a flask with inert atmosphere in 2 ml of pyridine. 0.06 g of DMAP dissolved in 2 mL of pyridine are subsequently added. The reaction flask is placed in an ice bath and 0.378 ml of 2-ethylbutyryl chloride are added. Then it is stirred for one hour at 0°C and at room temperature for 18 hours. The reaction is followed by TLC using hexane-ethyl acetate (2:1) as eluent. Yield: 95%.

1.5. Synthesis of the final compound

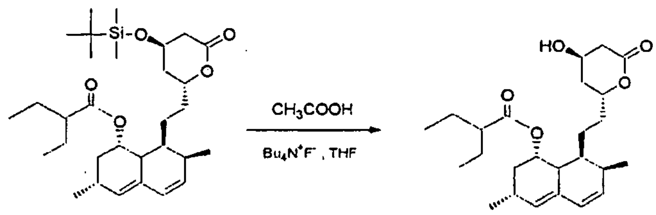


0.368 g of the derivative previously obtained are dissolved in 2 ml of THF. Then a solution of 0.16 ml of acetic acid and 2.16 ml of 1M tetrabutylammonium fluoride is added to the reaction medium. The reaction mixture is stirred at room temperature for 16 hours. The reaction is followed by TLC using dichloromethane-acetone (6:1) as eluent. Yield: 75 %.

**EXAMPLE 2**

**Protection by NST0037 against neuronal death induced by different aggressions: oxidative stress, endoplasmic reticulum stress and apoptosis**

2.1. Protection by NST0037 against neuronal death induced by oxidative stress

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 of cell death caused by treatment with xanthine/xanthine oxidase which generates oxidative damage (causes free radicals such as hydrogen peroxide, superoxide anion, hydroxyl radical), which triggers cell death, was analyzed. These cells, not exceeding 15 passages, were seeded on 96-well plates treated for adherent cells with a cell concentration of $5x10^4$ cells/well; 3 wells of the plate were seeded for each condition of the assay.

After 24 hours of cell incubation at 37°C and 5% $CO_2$, the cell treatments were performed with 100 μl of total volume for the following conditions:

- Control: culture medium (medium)
- Xanthine/xanthine oxidase (XXO): medium plus 10 μM xanthine / 60 mU/mL xanthine oxidase, causing the death of 50% of the cells.
- XXO plus NST0037: medium plus XXO (10 μM / 60 mU/mL) plus NST0037 at 1, 4, 10, 20, 40 or 100 μM.

The cells were incubated (at 37°C and 5% $CO_2$) with these treatments for 22 hours, after which time the WST-1 reagent (Roche) was added. The Test WST-1 is based on the measurement of metabolic activity. Cell damage causes the loss of the ability of cells to obtain the energy necessary to maintain their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are shown, as can be seen in Figure 1, as the percentage of cell death for each treatment relating to death caused by XXO. Protection against death was observed at from 1 to 100 μM of NST0037, the differences with respect to XXO being statistically significant, according to the Student's t-test, for all the concentrations evaluated,

reaching maximum protection of 78% at 20 μM. These results indicate that compound NST0037 shows a protective effect against the death of human cells of neuronal origin caused by oxidative stress.

2.2. Protection by NST0037 against neuronal death induced by endoplasmic reticulum stress

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 of cell death caused by treatment with tunicamycin generating reticular stress was analyzed. Tunicamycin is an inhibitor of protein N-glycosylation, causing abnormal protein folding in the endoplasmic reticulum, therefore said proteins are accumulated and cause stress, resulting in cell death. These cells, not exceeding 15 passages, were seeded on 96-well plates treated for adherent cells with a cell concentration of $5x10^4$ cells/well; 3 wells of the plate were seeded for each condition of the assay.

After 24 hours of cell incubation at 37°C and 5% $CO_2$, the cell treatments were performed with 100 μl of total volume for the following conditions:

- Control: culture medium (medium)
- Tunicamycin (Tm): medium plus tunicamycin 24 μM, causing the death of 50% of the cells.
- Tm plus NST0037: medium plus Tm (24 μM) plus NST0037 at 1, 4, 10, 40 or 100 μM.

The cells were incubated (at 37°C and 5% $CO_2$) with these treatments for 22 hours, after which time WST-1 reagent (Roche) was added. The WST-1 Test is based on the measurement of metabolic activity. Cell damage causes the loss of the ability of cells to obtain the energy necessary to maintain their metabolic functions and cell growth; therefore the metabolically active (live) cells reduce tetrazolium salt to formazan by means of the succinate-tetrazolium reductase system (of the mitochondrial respiratory chain). The formazan that is formed can be detected colorimetrically since it has an absorbance of 440 nm. The reading was taken in a plate reader at 440 nm 2 hours after adding the reagent.

The obtained results are shown, as can be seen in Figure 2, as the percentage of cell death for each treatment relating to death caused by Tm. Protection against death was observed at from 1 to 100 μM of NST0037, reaching 55% at 40 μM. These results indicate that compound NST0037 shows a protective effect against the death of human cells of neuronal origin caused by endoplasmic reticulum stress.

2.3. Protection by NST0037 against neuronal death induced by apoptosis

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 of cell death caused by treatment with camptothecin generating cell cycle arrest was analyzed. Camptothecin is an inhibitor of Topoisomerase I, it therefore prevents DNA duplication and triggers cell cycle arrest and death due to apoptosis. These cells, not exceeding 15 passages, were seeded on 96-well plates treated for adherent cells with a cell concentration of $5x10^4$ cells/well; 3 wells of the plate were seeded for each condition of the assay.

After 24 hours of cell incubation at 37°C and 5% $CO_2$, the cell treatments were performed with 100 μl of total volume for the following conditions:

- Control: culture medium (medium)
- Camptothecin (CPT): medium plus camptothecin 20 nM, causing death of 50% of the cells.
- Camptothecin plus NST0037: medium plus camptothecin (20 nM) plus NST0037 at 1, 4, 10, 20, 40 or 100 μM.

The cells were incubated (at 37°C and 5% $CO_2$) with these treatments for 22 hours, after which time the WST-1 reagent (Roche) was added. The Test WST-1 is based on the measurement of metabolic activity. Cell damage causes the loss of the ability of cells to obtain the energy necessary to maintain their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are shown, as can be seen in Figure 3, as the percentage of cell death for each treatment relating to death caused by camptothecin. Protection against death was observed at from 4 to 100 μM of NST0037, reaching 18%, 28%, 27% and 27% at 4, 10, 40 and 100 μM, respectively. These results indicate that compound NST0037 shows a protective effect of the death of human cells of neuronal origin caused by cell cycle arrest.

2.4. Determination of the inhibition of apoptosis by means of flow cytometry

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 against apoptosis (programmed cell death) caused by treatment with camptothecin which inhibits the enzyme Topoisomerase I, which prevents DNA duplication and triggers apoptotic cell death, was analyzed. These cells, not exceeding 15 passages, were seeded on 6-well plates treated for adherent cells with a cell concentration of $8x10^5$ cells/well and $7x10^5$ cells/well for the assay with pretreatment; 2 wells of the plate were seeded for each condition of the assay.

After 24 hours of cell incubation at 37°C and 5% $CO_2$, the cell treatments were performed with 2 ml of total volume for the following conditions:

- Control: culture medium (medium)
- Camptothecin: medium plus camptothecin 50 μM.
- Camptothecin plus Z-VAD-fmk: medium plus 50 μM camptothecin plus 50 μM Z-VAD-fmk, as positive inhibition control.
- Camptothecin plus NST0037: medium plus 50 μM camptothecin plus NST0037 at 10, 40 or 100 μM.

In the assay with pretreatment, the cells were previously treated with 40 μM of NST0037, 100 μM of mevalonate or both together for 24 hours and then they were treated with 50 μM camptothecin, the rest of the procedure was similar to the assay without pretreatment.

The cells were incubated (at 37°C and 5% $CO_2$) with these treatments for 6 hours, after which time they were collected along with their culture medium and centrifuged at 300xg for 5 minutes. The medium was removed, a washing was performed with PBS and the cells were fixed for 2 minutes with 500 μl of 70% ethanol at -20°C. Once fixed, they were centrifuged at 400xg for 5 minutes, washed with PBS and 0.05 mg/ml propidium iodide, diluted in cycling buffer (0.1% sodium citrate, 0.3% Nonidet P-40 and 0.02 mg/ml RNase) were added and they were incubated for 1 hour at 37°C. After this time they were analyzed by flow cytometry, comparing the fluorescence of propidium iodide against the amount of DNA. The percentage of apoptosis was measured on the sub-G1 region of each of the conditions

The obtained results are shown, as can be seen in Figure 4A and B, as the percentage of the inhibition of apoptosis of each treatment relating to apoptosis caused by camptothecin. Maximum protection of 18% was observed at 40 and 100 μM of NST0037 (Figure 4A), therefore this compound shows a protective effect of apoptosis in human cells of neuronal origin. Z-VAD-fmk, a specific caspase inhibitor, specifically inhibited the apoptosis caused by camptothecin. There results were corroborated by means of experiments of pretreatment of NST0037 at 40 μM (Figure 4B), which caused an increase of the percentage of protection (reaching up to 45%). Additionally, it was determined that the protection exerted by NST0037 is partially inhibited by adding mevalonate to the medium, which indicates that the neuroprotective effect of NST0037 is related to cholesterol biosynthesis or to one of the precursors of the pathway.

**EXAMPLE 3**

**Protection by NST0037 against neuronal death in the hippocampus, against cognitive deficit and against death caused by an excitotoxic substance**

3.1. Protective effect of NST0037 against neuronal death in the hippocampus of mice caused by an excitotoxic substance

Based on the results of Example 2, the inventors decided to investigate if the neuroprotective effect of NST0037 demonstrated in human cholinergic neurons was corroborated in a model of sporadic Alzheimer's disease in mice by means of the administration of kainate (KA).

All the animals included for the experimental process were 12-week old males of the FVB/NHan strain. The experiments were conducted strictly following the Guidance on the Operation of Animals (Scientific Procedures, Act. 1986*)*. The animals had their respective quarantine period and were treated with maximum precaution to minimize

possible contaminations for inoculations and handling.

Twenty-eight animals were used in this assay and the treatments were all conducted by intraperitoneal (i.p.) route with a volume of 100 μl, according to the following regimens:

i) PBS+PBS+PBS regimen: 4 animals were initially treated with PBS with a daily dose for 2 days, on the second day the animals were inoculated with a new dose of PBS and for the next 7 days with a daily dose of PBS.
ii) PBS+KA+PBS regimen: 6 animals were initially treated with PBS with a daily dose for 2 days, on the second day the animals were inoculated with a dose of kainate of 25 mg/Kg and for the next 7 days with a daily dose of PBS.
iii) PBS+KA+NST0037 regimen: 7 animals were initially treated with PBS with a daily dose for 2 days, on the second day the animals were inoculated with a dose of kainate at 25 mg/Kg and for the next 7 days with a daily dose of NST0037 at 50 mg/kg.
iv) NST0037+KA+NST0037 regimen: 6 animals were initially treated with a daily dose for 2 days with NST0037 at 50 mg/kg, on the second day the animals were inoculated with a dose of kainate at 25 mg/Kg and for the next 7 days with a daily dose of NST0037 at 50 mg/kg.
v) NST0037+PBS+NST0037 regimen: 5 animals were initially treated with a daily dose for 2 days with NST0037 at 50 mg/kg, on the second day the animals were inoculated with a dose of PBS and for the next 7 days with a daily dose of NST0037 at 50 mg/kg.

Once the treatment time of 7 days ended, the animals were sacrificed and the brains were dissected. The brain samples were processed and included in paraffin. Hematoxylin and eosin stain was used in 5 μm thick sections to analyze the cell architecture of the hippocampus.

As shown in Figure 5, the PBS+KA+PBS administration regimen caused severe neuronal damage in the CA1 and CA2 regions of the hippocampus in mice. In the samples of said group, an absence of neurons, evidence of necrosis and a number of pycnotic nuclei were observed, which demonstrates neuronal death. However, the samples of the NST0037+KA+NST0037 group showed a cell structure identical to the controls (PBS+PBS+PBS group) without evidence of cell damage in the CA1 and CA2 regions. Furthermore and surprisingly, the samples of the PBS+KA+NST0037 group showed several signs of damage and neuronal death in the hippocampus. However, comparing *de visu* the samples of the PBS+KA+PBS group with those of the PBS+KA+NST0037 group, a higher number of neurons of the hippocampus without damage was observed in the group with the NST0037 treatment, indicating its neuroprotective effect. Finally, the samples of the NST0037+PBS+NST0037 group showed a pattern identical to that observed in the animals of the PBS+PBS+PBS group.

In summary, the pre-treatment with NST0037 (NST0037+KA+NST0037 group) completely protected against neuronal death caused by KA in the hippocampal CA1 and CA2 regions. Furthermore, the beginning of treatment with NST0037 after the inoculation of KA qualitatively reduced neuronal death and damage in this region. It was also demonstrated that the daily administration of NST0037 for 8 days was not neurotoxic for the mice.

<u>3.2. Protective effect of NST0037 against episode-type memory deterioration in mice caused by an excitotoxic substance</u>

The excitotoxicity caused by KA induces, a few days after its inoculation in mice, episode-type memory deterioration, affecting the temporal memory and causing a severe spatial memory deficit. As a result the inventors decided to investigate if the neuroprotective effect of NST0037 was accompanied by a protection of the memory which deteriorates due to the effect of an excitotoxic substance.

All the animals included for the experimental process were 12-week old males of the FVB/NHan strain. The experiments were conducted strictly following the Guidance on the Operation of Animals (Scientific Procedures, Act. 1986*)*. The animals had their respective quarantine period and were treated with maximum precaution to minimize possible contaminations for inoculations and handling.

Twenty-eight animals were used in this assay and the treatments were all conducted by intraperitoneal (i.p.) route with a volume of 100 μl, according to the following regimens:

i) PBS+PBS+PBS regimen: 4 animals were initially treated with PBS with a daily dose for 2 days, on the second day the animals were inoculated with a new dose of PBS and for the following 3 days with a daily dose of PBS.
ii) PBS+KA+PBS regimen: 6 animals were initially treated with PBS with a daily dose for 2 days, on the second day the animals were inoculated with a dose of kainate at 25 mg/Kg and for the following 3 days with a daily dose of PBS.
iii) PBS+KA+NST0037 regimen: 7 animals were initially treated with PBS with a daily dose for 2 days, on the second day the animals were inoculated with a dose of kainate at 25 mg/Kg and for the following 3 days with a daily dose of NST0037 at 50 mg/kg.
iv) NST0037+KA+NST0037 regimen: 6 animals were initially treated with a daily dose for 2 days with NST0037 at 50 mg/kg, on the second day the animals were inoculated with a dose of kainate at 25 mg/Kg and for the following

3 days with a daily dose of NST0037 at 50 mg/kg.

v) NST0037+PBS+NST0037 regimen: 5 animals were initially treated with a daily dose for 2 days with NST0037 at 50 mg/kg, on the second day the animals were inoculated with a dose of PBS and for the following 3 days with a daily dose of NST0037 at 50 mg/kg.

Three days after inoculation of KA, an object recognition test called integral memory test was performed on the animals. Figure 6 shows the results of the relation in the time of the exploration of old objects compared to new objects (temporal memory). It was observed that the group with the NST0037+KA+NST0037 treatment regimen prevented the reduction in the temporal memory capacity in comparison with the mice of the PBS+KA+PBS group. Furthermore, the animals of the PBS+KA+NST0037 group also showed an improvement of the state of the temporal memory with respect to those treated in the PBS+KA+PBS group. The data also showed that the group of mice with the NST0037+PBS+NST0037 regimen also showed an improvement of the temporal memory with respect to the PBS+PBS+PBS group.

Figure 7 shows the results of the relation over time of the exploration of the displaced old object compared with the non-displaced old object (spatial memory). The group of mice with the NST0037+KA+NST0037 or NST0037+PBS+NST0037 treatment regimens showed a relation of the exploration of objects demonstrating that the spatial memory is intact since no differences were observed with respect to the PBS+PBS+PBS group. However, the mice of the PBS+KA+PBS group showed severe deterioration of spatial memory. The PBS+KA+NST0037 treatment group showed an improvement of this type of memory with respect to those that were treated in the PBS+KA+PBS group.

These studies indicate that the treatment before and after (or only after) the damage with an excitotoxic substance with compound NST0037 has a protective effect on episodic (temporal and spatial) memory which degenerates after the administration of an excitotoxic substance.

3.3. Protective effect of NST0037 against death caused by an excitotoxic substance

It is known that the administration of an excitotoxic substance to animals induces, in some cases, death of the animal. As a result, the inventors decided to investigate if the neuroprotective effect of NST0037 was accompanied by a reduction of the mortality caused by an excitotoxic substance.

All the animals included for the experimental process were 12-week old males of the FVB/NHan strain. The experiments were conducted strictly following the Guidance on the Operation of Animals (Scientific Procedures, Act. 1986*)*. The animals had their respective quarantine period and were treated with maximum precaution to minimize possible contaminations for inoculations and handling.

Twenty-eight animals were used in this assay and the treatments were all conducted by intraperitoneal (i.p.) route with a volume of 100 $\mu$l, according to the following regimen:

vi) PBS+PBS+PBS regimen: 4 animals were initially treated with PBS with a daily dose for 2 days, on the second day the animals were inoculated with a new dose of PBS and for the following 3 days with a daily dose of PBS.

vii) PBS+KA+PBS regimen: 6 animals were initially treated with PBS with a daily dose for 2 days, on the second day the animals were inoculated with a dose of kainate at 25 mg/Kg and for the following 3 days with a daily dose of PBS.

viii)PBS+KA+NST0037 regimen: 7 animals were initially treated with PBS with a daily dose for 2 days, on the second day the animals were inoculated with a dose of kainate at 25 mg/Kg and for the following 3 days with a daily dose of NST0037 at 50 mg/kg.

ix) NST0037+KA+NST0037 regimen: 6 animals were initially treated with a daily dose for 2 days with NST0037 at 50 mg/kg, on the second day the animals were inoculated with a dose of kainate at 25 mg/Kg and for the following 3 days with a daily dose of NST0037 at 50 mg/kg.

x) NST0037+PBS+NST0037 regimen: 5 animals were initially treated with a daily dose for 2 days with NST0037 at 50 mg/kg, on the second day the animals were inoculated with a dose of PBS and for the following 3 days with a daily dose of NST0037 at 50 mg/kg.

The deaths were recorded during the entire time the assay lasted, and the results are shown in Figure 8 by means of Kaplan-Meier survival curves. The results of the study of the mortality caused by KA showed that the group with the NST0037+KA+NST0037 treatment regimen was protected against death associated to the damage caused by an excitotoxic substance. Furthermore, and more importantly, the survival of the animals in group with the PBS+KA+NST0037 treatment regimen increases in comparison with that of the group with the PBS+KA+PBS regimen.

These studies indicate that the treatment before and after (or only after) of the damage with an excitotoxic substance with compound NST0037 has a protective effect against mortality caused by the administration of an excitotoxic substance.

## EXAMPLE 4

**Antiepileptic effect of NST0037 against the action of an excitotoxic substance**

It is known that the administration of an excitotoxic substance to animals induces, in some cases, epileptic seizures and convulsions in the animals. As a result, the inventors decided to investigate if the neuroprotective effect of NST0037 was accompanied by an antiepileptic effect caused by an excitotoxic substance.

All the animals included for the experimental process were 12-week old males of the FVB/NHan strain. The experiments were conducted strictly following the Guidance on the Operation of Animals (Scientific Procedures, Act. 1986*).* The animals had their respective quarantine period and were treated with maximum precaution to minimize possible contaminations for inoculations and handling.

The animals were intraperitoneally inoculated with 25 mg/kg of kainate (KA) dissolved in PBS. Thirteen animals were pre-treated 24 and 0.5 hours before inoculation with KA by means of intraperitoneal injection with PBS (PBS+KA administration regimen) and 6 were pre-treated 24 and 0.5 hours before inoculation with KA by means of intraperitoneal injection with NST0037 at a dose of 50 mg/kg (NST0037+KA administration regimen). After the inoculation, the animals were individually housed in trays to monitor them. The maximum epilepsy level of the animals was recorded during the observation according to the Racine scale every ten minutes and for at least 120 minutes post-inoculation (m.p.i.).

Comparative studies of the epileptogenic phenomena between the treatment with PBS (PBS+KA) and with NST0037 (NST0037+KA) were subsequently conducted. Differences were observed in the percentage of animals that entered in *status epilepticus,* i.e., they showed tonic-clonic seizures, among the NST0037+KA group which was 33.3% (2 out of 6) compared to the PBS+KA treatment regimen group which was 76.9% (10 out of 13), which demonstrates that compound NST0037 is antiepileptic and anticonvulsant. Differences over time were also observed in which the first convulsions occurred (*latency period*), in which the latency of the NST0037+KA group (103.3 $\pm$ 13.1 minutes) was greater than with the PBS+KA treatment regimen (74.6 $\pm$ 8.6 minutes) as shown in Figure 9, which demonstrates an antiepileptic and anticonvulsant effect of compound NST0037. Based on these results the inventors decided to investigate if the antiepileptic and anticonvulsant effect was confirmed with the epilepsy level and by the severity of the symptoms, observing that the animals of the group with the PBS+KA regimen reached epilepsy level 4 on the Racine scale, whereas the NST0037+KA regimen did not cause at any time an epilepsy level 3 on said scale, indicating that compound NST0037 is antiepileptic and anticonvulsant. Furthermore, based on the 100 m.p.i. the NST0037+KA regimen caused the disappearance of the epileptogenic symptoms, whereas the PBS+KA regimen showed severe seizures and spasms.

These studies indicate that treatment with compound NST0037 has an antiepileptic and anticonvulsant effect due to the administration of an excitotoxic substance.

## EXAMPLE 5

**Inhibitory activity of HMGR and hypocholesterolemic effect of NST0037 in an endogenous hyperlipidemia model in mice**

5.1. Inhibitory effect of NST0037 on the HMGR enzyme activity

Due to the nature of the compound, the degree of inhibition of the HMGR enzyme by compound NST0037 was determined since this enzyme is key in cellular cholesterol synthesis and in the physiological regulation of said synthesis. The effect of this compound was compared with that of two known statins, atorvastatin and simvastatin, compounds for which a potent inhibitory effect on HMGR has already been described. For the reaction, HMGR uses NADPH as a reducing agent and 3-hydroxy-3-methylglutaryl coenzyme A (HMGCoA) as a substrate, producing mevalonic acid, CoASH and NADP+. The reduction of the absorbance at which NADPH (340 nm) is absorbed is a direct measurement of the catalytic activity of HMGR and serves to calculate the inhibition percentages of different compounds. In order to take the HMGR inhibition, the assay was performed on a 96-well plate, with a total reaction volume of 200 $\mu$l. The reaction buffer (50 mM $KH_2PO_4$, 1 M KCl, 2 mg/ml Bovine Serum Albumin [BSA] and 5 mM DTT, at pH=7.3) was prepared at the time the reaction was conducted and was maintained at 37°C. The following was included in the assays:

- A blank: Lacking HMGR which reports on the stability of NADPH for the reaction.
- A control: Contains all the components of the reaction but lacks the test compound.
- Test compounds: NST0037, Atorvastatin and Simvastatin at several concentrations. The acid form of the compounds is used in all the cases.

Five independent assays were conducted with NST0037, four independent assays with atorvastatin and six independent assays with simvastatin, the inhibition potency being determined in a range of concentrations which allowed

defining the 50% inhibition constants ($IC_{50}$) by means of spectrophotometric determination of the drop of NADPH at 37°C, and as is shown in Figure 11. The reduction of absorbance at 340 nm allows calculating the percentage of HMGR enzyme activity with respect to the control. The $IC_{50}$ were determined by means of the Trimmed Spearman-Karber method (Version 1.5). The results indicate that the inhibition potency of NST0037 is surprisingly close to that of more potent statin, atorvastatin, and is 7.5 times more potent than simvastatin, which demonstrates a clear inhibitory effect of HMGR.

5.2. Hypocholesterolemic effect of NST0037 in an endogenous hyperlipidemia model (familial)

Based on the results of the previous point, the inventors decided to investigate if the inhibitory activity of HMGR enzyme by compound NST0037 corresponds with a variation of total cholesterol and of its fractions in an endogenous hyperlipidemia model in mice.

All the animals included for the experimental process were 46-week old males of the ApoB100 strain, which strain has a deficiency in removing cholesterol from the blood which causes an abnormally high increase of plasma cholesterol. The experiments were conducted strictly following the Guidance on the Operation of Animals (Scientific Procedures, Act. 1986*)*. The animals had their respective quarantine period and were treated with maximum precaution to minimize possible contaminations for inoculations and handling.

In order to conduct the experiment on the fasting animals, blood was extracted by ocular puncture. The plasma was obtained from said blood, in which plasma total cholesterol levels and levels of its fractions were determined prior to administration of the substances under study. Immediately after that, the animals were intraperitoneally inoculated with 50 mg/kg of each of the test compounds. Four animals were treated with the vehicle and were considered the control group. A second group of five mice received 50 mg/Kg of simvastatin. Finally, a third group of five mice received 50 mg/Kg of NST0037. Twelve hours after the treatments, blood was again extracted from the fasting animals and the plasma was obtained from said blood. Figure 12 shows the plasma cholesterol levels at the initial time (t0) and twelve hours after the administration of the vehicle, simvastatin or NST0037 in the different groups of mice (t12), demonstrating that both compounds have a significant hypocholesterolemic effect. In relation to the different cholesterol fractions, both compounds considerably reduced the low density lipoprotein cholesterol (LDL-c) and very low density lipoprotein cholesterol (VLDL-c) levels without changing the high density lipoprotein cholesterol (HDL-c) levels, as shown in Figures 13 to 15. In relation to the free cholesterol (FC) and esterified cholesterol (EC) levels, both compounds reduced the EC levels in a similar manner without changing FC levels, as shown in Figures 16 and 17.

The results shown in this section demonstrate that compound NST0037 shows an effect that is surprisingly similar to simvastatin, significantly reducing TC, LDL-c, VLDL-c and EC levels without changing HDL-c or FC levels.

**EXAMPLE 6**

**Hypocholesterolemic effect of NST0037 in an induced hyperlipidemia model in mice**

Based on the results obtained in the experiment with ApoB100 animals, the inventors decided to investigate if compound NST0037 also showed a hypocholesterolemic effect in an induced acute hyperlipidemia model.

All the animals included for the experimental process were wild-type 6-week old male mice of the C57BL6 strain. The experiments were conducted strictly following the Guidance on the Operation of Animals (Scientific Procedures, Act. 1986*)* . The animals had their respective quarantine period and were treated with maximum precaution to minimize possible contaminations for inoculations and handling.

In order to conduct the experiment on the fasting animals, blood was extracted by ocular puncture. The plasma was obtained from said blood, in which plasma total cholesterol levels and levels of its fractions were determined prior to administration of the substances under study. Immediately after that, the animals were intraperitoneally inoculated with 500 mg/kg of Triton 1339, also known as Tyloxapol. After thirty minutes, the animals were intraperitoneally inoculated with 50 mg/kg of each of the test compounds. Seven animals were treated with the vehicle alone and were considered the control group. A second group of six mice received 50 mg/Kg of simvastatin. Finally, a third group of seven mice received 50 mg/Kg of NST0037. Twenty-four hours after the treatments, blood was again extracted from the fasting animals and the plasma was obtained from said blood. Figure 18 shows the number of times the total cholesterol increases twenty-four hours after the administration of Triton 1339 followed by the administration of the vehicle, simvastatin or NST0037 in the different groups of mice, demonstrating that both test compounds have a hypocholesterolemic effect, being statistically significant only in the case of treatment with NST0037. In relation to the different cholesterol fractions, both compounds reduced the low density lipoprotein cholesterol (LDL-c) levels, said reduction being statistically significant only in the case of NST0037. Only simvastatin reduced the very low density lipoprotein cholesterol (VLDL-c) levels, the high density lipoprotein cholesterol (HDL-c) levels not being changed with either of the two compounds, as shown in Figures 19 to 21. Unlike simvastatin, NST0037 reduced the esterified cholesterol (EC) levels, as shown in

Figure 22. In relation to the free cholesterol (FC) levels, both compounds reduced the levels of this fraction in a similar manner, as shown in Figure 23.

The results shown in this section surprisingly demonstrate that compound NST0037 has a hypocholesterolemic effect identical to or greater than simvastatin, reducing TC and LDL-c levels in a statistically significant manner. These results confirm the hypocholesterolemic effect of NST0037 observed in the apoB100 mice model.

**EXAMPLE 7**

**Biosafety of NST0037 in zebrafish embryo**

7.1. Analysis of the survival rate of compound NST0037 in comparison with simvastatin

To evaluate the biosafety of compound NST0037, its toxicological effects on the zebrafish embryo model were analyzed by means of determining the safety parameters of the OECD C15 protocol.

The fertilized eggs were obtained by natural mating of the zebrafish (Danio rerio, AB strain). A total of 8-10 pairs were used for each cross and a total of 200-250 eggs were generated on average per pair. The eggs were collected immediately after spawning and were washed with dilution water ($CaCl_2$•$2H_2O_2$ 0.29 g/L, $MgSO_4$•$7H_2O_2$ 0.12 g/L, $NaHCO_3$ 0.065 g/L, KCl 0.006 g/L), the pH being adjusted to 7.8 $\pm$ 0.2, in accordance with EC Regulation 440/2008, method C.1, and were deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25 $\pm$ 1°C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:

- 10 control animals, treated with dilution water and with daily replacement for the 9 days that the assay lasted.
- 30 animals treated with compound NST0037 (10 embryos in triplicate) diluted in dilution water and with daily replacement for the 9 days that the assay lasted.
- 30 animals treated with the compound simvastatin (10 embryos in triplicate) diluted in dilution water and with daily replacement for the 9 days that the assay lasted.

The mortality of the embryos-larvae was recorded for the entire duration of the assay, the results being shown in Figures 24 and 25 by means of Kaplan-Meier curves. The results of the study of the mortality induced by the two substances under study showed that compound NST0037 is surprisingly safer than simvastatin at the evaluated doses, the survival curves being statistically different when comparing both treatments.

7.2. Analysis of the lethal dose 50s over time of compound NST0037 in comparison with simvastatin

Based on the results of the previous section, the inventors decided to investigate if the higher biosafety of compound NST0037 in comparison with simvastatin was corroborated by means of the analysis of the lethal dose 50s ($LD_{50}$) over time.

The fertilized eggs were obtained by natural mating of the zebrafish (*Danio rerio*, AB strain). A total of 8-10 pairs were used for each cross and a total of 200-250 eggs were generated on average per pair. The eggs were collected immediately after spawning and were washed with dilution water ($CaCl_2$•$2H_2O_2$ 0.29 g/L, $MgSO_4$•$7H_2O_2$ 0.12 g/L, $NaHCO_3$ 0.065 g/L, KCl 0.006 g/L) the pH being adjusted to 7.8 $\pm$ 0.2, in accordance with EC Regulation 440/2008, method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25 $\pm$ 1°C) and under a regimen of 12 hours light/12 hours darkness. Each experiment was performed in triplicate.

The studies were conducted in a total of 9 days post-fertilization, in which the treatment was renewed every

day, thus conducting a semi-static assay. The treatments conducted followed the following regimen:

- ➢ 10 control animals, treated with dilution water and with daily replacement for the 9 days that the assay lasted.
- ➢ 30 animals treated with compound NST0037 (10 embryos in triplicate) diluted in dilution water and with daily replacement for the 9 days that the assay lasted.
- ➢ 30 animals treated with the compound simvastatin (10 embryos in triplicate) diluted in dilution water and with daily replacement for the 9 days that the assay lasted.

For the duration of the study, the number of dead embryos/larvae was recorded at 24-hour intervals and the $LD_{50}$ was calculated at each time point of the experiment, as shown in Figure 26. The results show that in the first days posttreatment (up to day 2) the $LD_{50}$ of both compounds is above the maximum dose evaluated. Nevertheless, from the third day and up to the end of the experiment, simvastatin shows an $LD_{50}$ lower than that of compound NST0037, indicating its higher toxicity. After day 2 of treatment, the $LD_{50}$ of both compounds progressively reduced throughout the entire experiment, although simvastatin always showed an $LD_{50}$ under that of compound NST0037, indicating that NST0037 has a higher degree of biosafety than simvastatin.

<u>7.3. Analysis of the percentage of healthy larvae at the end of the experiment of compound NST0037 in comparison with simvastatin over time</u>

Based on the results of the previous sections, the inventors decided to investigate if the higher biosafety of compound NST0037 in comparison with simvastatin was corroborated by means of the analysis of the percentage of healthy larvae at the end of the experiment.

The fertilized eggs were obtained by natural mating of the zebrafish (*Danio rerio*, AB strain). A total of 8-10 pairs were used for each cross and a total of 200-250 eggs were generated on average per pair. The eggs were collected immediately after spawning and were washed with dilution water ($CaCl_2$•$2H_2O_2$ 0.29 g/L, $MgSO_4$•$7H_2O_2$ 0.12 g/L, $NaHCO_3$ 0.065 g/L, KCl 0.006 g/L) the pH being adjusted to 7.8 $\pm$ 0.2, in accordance with EC Regulation 440/2008, method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25$\pm$1 °C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:

- ➢ 10 control animals, treated with dilution water and with daily replacement for the 9 days that the assay lasted.
- ➢ 30 animals treated with compound NST0037 (10 embryos in triplicate) diluted in dilution water and with daily replacement for the 9 days that the assay lasted.
- ➢ 30 animals treated with the compound simvastatin (10 embryos in triplicate) diluted in dilution water and with daily replacement for the 9 days that the assay lasted.

The results at the end of the experiment allowed collecting the percentage of healthy larvae which reach the end of the experiment, defined as the number of live larvae and without symptoms of external physiopathological anomalies (morphological and/or behavioral), this being a parameter determining the biosafety of a substance under study and is complementary to the survival rates and to the $LD_{50}$. As shown in Figure 27, evident differences were observed between the percentages of healthy larvae between the two treatments evaluated at the higher doses under study (0.06 and 0.2 mg/L), more healthy larvae reaching the end of the experiment with the NST0037 treatment, indicating its higher biosafety.

<u>7.4. Analysis of the percentage of larvae with malformations or anomalous appearance at the end of the experiment of compound NST0037 in comparison with simvastatin over time</u>

Based on the results of the previous sections, the inventors decided to investigate if the higher biosafety of compound NST0037 in comparison with simvastatin was corroborated by means of the analysis of the percentage of larvae with malformations or anomalous appearance, collecting the number of larvae showing bodily and/or pigmentary anomalies, as well as the yolk sac reabsorption phase at suitable intervals (every 24 hours). The anomalies recorded

in the study are described below:

- Intracranial thrombosis: a clot inside the blood vessel, in this case of any cerebral vessel.
- Cardiac thrombosis: occurs in a cardiac vessel.
- Cardiac edema (or hydrops): accumulation of fluid in the intercellular tissue space and also in the cavities of the organism. In the case of the heart, it causes the accumulation of fluid in the pericardial sac.
- Malformation: a noticeable difference in the shape of the body or part of the body, or organ of the body (internal or external) compared with the average shape of the part in question. The malformations observed in this study are usually of the yolk sac.

The fertilized eggs were obtained by natural mating of the zebrafish (*Danio rerio*, AB strain). A total of 8-10 pairs were used for each cross and a total of 200-250 eggs were generated on average per pair. The eggs were collected immediately after spawning and were washed with dilution water ($CaCl_2$•$2H_2O_2$ 0.29 g/L, $MgSMO_4$•$7H_2O_2$ 0.12 g/L, $NaHCO_3$ 0.065 g/L, KCl 0.006 g/L) the pH being adjusted to 7.8 ± 0.2, in accordance with EC Regulation 440/2008, method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls) or with different concentrations of the treatments. The embryos were incubated without additional aeration, at the suitable temperature (25±1 °C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:

➢ 10 control animals, treated with dilution water and with daily replacement for the 9 days that the assay lasted.
➢ 30 animals treated with compound NST0037 (10 embryos in triplicate) diluted in dilution water and with daily replacement for the 9 days that the assay lasted.
➢ 30 animals treated with the compound simvastatin (10 embryos in triplicate) diluted in dilution water and with daily replacement for the 9 days that the assay lasted.

The results indicate that the percentage of larvae with malformations or anomalous appearance is dependent on time and on the treatment, as shown in Figure 28, observing that at the evaluated dose (0.2 mg/L) simvastatin induced in the embryos-larvae a significant percentage of anomalies or toxicological problems, whereas compound NST0037 did not show significant toxicological effects, indicating its higher biosafety. Treatment with simvastatin caused intracranial and cardiac thrombosis and pericardial edemas. The data of the chart also shows that the animals recovered from the problems induced by simvastatin over time, indicating that the treatment with 0.2 mg/L of this substance was not toxic enough to induce the death of the animal, with the subsequent improvement of said animals.

These results indicate that compound NST0037 is safer than simvastatin, since the latter induces a significantly higher percentage of more severe toxicological problems.

7.5. Analysis of the variation of the cardiotoxicity of compound NST0037 in comparison with simvastatin dependent on the dose

Based on the results of the previous sections, the inventors decided to investigate if the higher biosafety of compound NST0037 in comparison with simvastatin was corroborated by means of the analysis of the cardiotoxicity after the treatment with the different compounds and at various doses. The study of this parameter (cardiotoxicity) not only determines the cardiac rhythm of the animals but it furthermore allows observing heartbeat alterations (tachycardia, bradycardia, etc) or heart development anomalies (pericarditis, atrophy, hypertrophy, etc.).

The fertilized eggs were obtained by natural mating of the zebrafish (*Danio rerio,* AB strain). A total of 8-10 pairs were used for each cross and a total of 200-250 eggs were generated on average per pair. The eggs were collected immediately after spawning and were washed with dilution water ($CaCl_2$•$2H_2O_2$ 0.29 g/L, $MgSO_4$•$7H_2O_2$ 0.12 g/L, $NaHCO_3$ 0.065 g/L, KCl 0.006 g/L) the pH being adjusted to 7.8 ± 0.2, in accordance with EC Regulation 440/2008, method C.1, and deposited in a Petri dish.

To assure exposure in the earliest stages of development, the eggs were quickly transferred (a minimum of 50 per condition) to another Petri dish with solutions of the compounds to be tested. Subsequently, only the fertilized eggs (10 per experimental condition) were transferred from the Petri dishes to the exposure chambers (M24 microtiter plates) by means of pipettes and exposed to the substances to be tested (NST0037 or simvastatin) with dilution water (controls)

or with different concentrations of the treatments. The embryos were incubated without additional aeration, at the suitable temperature (25±1 °C) and under a regimen of 12 hours light/12 hours darkness. Each experiment was performed in triplicate.

The studies were conducted in a total of 9 days post-fertilization, in which the treatment was renewed every day, thus conducting a semi-static assay. The treatments conducted followed the following regimen:

- ➢ 10 control animals, treated with dilution water and with daily replacement for the 9 days that the assay lasted.
- ➢ 30 animals treated with compound NST0037 (10 embryos in triplicate) diluted in dilution water and with daily replacement for the 9 days that the assay lasted.
- ➢ 30 animals treated with the compound simvastatin (10 embryos in triplicate) diluted in dilution water and with daily replacement for the 9 days that the assay lasted.

The cardiac rhythm of the zebrafish embryos-larva was determined by means of visual analysis in stereomicroscopy, determining the heartbeat with the aid of a manual counter for a period of one minute. The different treatments varied the cardiac rhythm of the animals, as shown in Figure 29, observing that the larvae treated with simvastatin showed a statistically significant reduction with respect to the controls after the dose of 0.06, which became very evident at the dose of 2 mg/L. In contrast, the reduction of the cardiac rhythm with compound NST0037 was statistically significant only at the dose of 2 mg/L in comparison with the controls, whereas at lower concentrations it showed no differences with the controls. Furthermore, at the maximum dose used of both compounds (2 mg/L), the cardiac rhythm of the animals treated with simvastatin was statistically lower than those treated with NST0037, demonstrating again that compound NST0037 has a higher biosafety than simvastatin. Therefore, NST0037 is a more heart-safe compound than simvastatin.

**EXAMPLE 8**

**Fungicidal activity of NST0037**

The fungicidal activity of NST0037 was analyzed by means of bioassay against *Candida albicans.* Solutions of the β-hydroxy acid form of NST0037, lovastatin, atorvastatin and simvastatin were prepared for this purpose. The assayed concentrations were the following: 2, 1.5, 1, 0.5, 0.25, 0.125, 0.06, 0.025 and 0.01 mM. Plates of MA medium (containing per liter: 20 g malt extract, 20 g glucose, 1 g mycopeptone and 10 g agar) previously inoculated with a culture of *Candida albicans* CECT (Spanish Type-Culture Collection) 1002 were prepared. With aid of a die (6 mm in diameter) various wells were prepared in which 40 μL of the previous solutions were deposited. The study was performed in triplicate and triplicates for each compound and concentration were included in each plate. The plates were kept at 4°C for 1 hour and were subsequently incubated at 28°C overnight. The presence of antifungal activity was determined by means of the formation of *C. albicans* growth inhibition halos.

The obtained results showed that compound NST0037 has a higher antifungal activity than the statins included in the assay. As shown in Figure 30, this behavior was observed for the entire range of concentrations. In the case of higher concentrations (2-0.25 mM) compound NST0037 showed values slightly greater than those of simvastatin, the statin which showed the highest fungicidal activity. However, when using lower concentrations (0.06 and 0.025 mM), compound NST0037 was the only one capable of causing inhibition halos.

**EXAMPLE 9**

**Induction of the cell expression of the 24-dehydrocholesterol reductase gene (Seladin-1/DHCR24) due to treatment with NST0037**

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The expression of the seladin-1/DHCR24 gene (NCBI Reference Sequence: NM_014762.3) was analyzed by means of real-time quantitative RT-PCR in comparison with memantine (a drug normally used to treat Alzheimer's disease). The SK-N-MC cells were treated for 24 hours in the absence (control) or presence of NST0037 or of memantine at 1, 4, 10 or 40 μM. The total RNA was extracted by means of the High Pure RNA Isolation kit (Roche) and the amount and quality of the RNA were analyzed by means of spectrophotometry (Infinite 200 with NanoQuant, Tecan) and viewing of the 18S and 28S bands by means of electrophoresis. The RT-PCR was performed by means of two steps, first, the

mRNA was changed to cDNA using the RNA to cDNA kit (Applied Biosystem) and the gene expression was subsequently analyzed by means of TaqMan probes using the validated probes Hs00207388_m1 for seladin-1/DHCR24 and Hs99999901_s1 for 18S (used to normalize the results) in the 7500 Fast Real-Time PCR System equipment (Applied Biosystem). Two independent assays were performed in triplicate. The relative amount of the gene expression was determined by using the ΔΔCt method with the SDS v2.1.1 software (Applied Biosystem); the expression of 18S was used to normalize the measurement.

The obtained results, as can be seen in Figure 31, show the increase of seladin-1/DHCR24 gene expression with the treatment with NST0037 at the concentrations assayed, but not with the treatment with memantine, which indicates that the treatment with NST0037 causes the specific increase of seladin-1/DHCR24 gene expression, which is related to cholesterol biosynthesis and to anti-apoptotic capacity by means of the inhibition of caspase 3.

**EXAMPLE 10**

**Protection by NST0037 against neuronal death induced by the inhibition of protein phosphatase 1 (PP1)**

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 of cell death caused by the treatment with okadaic acid (OA) which inhibits the activity of protein phosphatase 1 (PP1) was analyzed. OA is one of the most used drugs for studying the phosphorylation mechanism of the tau protein, involved in the pathogenesis and progression of AD. The inhibition of PP1 causes cytoskeleton alterations and mitochondrial damage. These cells, not exceeding 15 passages, were seeded on 96-well plates treated for adherent cells with a cell concentration of $5x10^4$ cells/well; 3 wells of the plate were seeded for each condition of the assay.

After 24 hours of cell incubation at 37°C and 5% $CO_2$, the cell treatments were performed with 100 μl of total volume for the following conditions:

- Control: culture medium (medium)
- Okadaic acid (OA): medium plus 20 nM OA, causing the death of 50% of the cells.
- OA plus NST0037: medium plus OA (20 nM) plus NST0037 at 1, 4, 10, 40 or 100 μM.

The cells were incubated (at 37°C and 5% $CO_2$) with these treatments for 22 hours, after which time the WST-1 reagent (Roche) was added. The Test WST-1 is based on the measurement of metabolic activity. Cell damage causes the loss of the ability of cells to obtain the energy necessary to maintain their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are shown, as can be seen in Figure 32, as the percentage of cell death for each treatment relating to death caused by OA. Protection against death was observed at from 4 to 40 μM of NST0037, reaching 57% at 40 μM. These results indicate that compound NST0037 shows a protective effect against the death of human cells of neuronal origin caused by inhibition of PP1.

**EXAMPLE 11**

**Protection by NST0037 against neuronal death induced by the inhibition of succinate dehydrogenase**

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The inhibition caused by compound NST0037 of cell death caused by treatment with 3-Nitropropionic (3-NP) acid was analyzed. 3-NP is an irreversible inhibitor of the succinate dehydrogenase enzyme which in animal models causes oxidative stress and apoptotic cell death in the striatum, which mimics neurochemical and anatomical changes associated with Huntington's disease (HD). These cells, not exceeding 15 passages, were seeded on 96-well plates treated for adherent cells with a cell concentration of $5x10^4$ cells/well; 3 wells of the plate were seeded for each condition

of the assay.

After 24 hours of cell incubation at 37°C and 5% $CO_2$, the cell treatments were performed with 100 μl of total volume for the following conditions:

- Control: culture medium (medium)
- 3-Nitropropionic (3-NP): medium plus 30 μM 3-NP, causing the death of 50% of the cells.
- 3-NP plus NST0037: medium plus 3-NP (30 μM) plus NST0037 at 0.1, 1, 4, 10, 40, 100 or 200 μM.

The cells were incubated (at 37°C and 5% $CO_2$) with these treatments for 22 hours, after which time the WST-1 reagent (Roche) was added. The Test WST-1 is based on the measurement of metabolic activity. Cell damage causes the loss of the ability of cells to obtain the energy necessary to maintain their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are shown, as can be seen in Figure 33, as the percentage of cell death for each treatment relating to death caused by 3-NP. Protection against death was observed at from 1 to 100 μM of NST0037, reaching 41% at 40 μM. These results indicate that compound NST0037 shows a protective effect against the death of human cells of neuronal origin caused by inhibition of the succinate dehydrogenase enzyme.

## EXAMPLE 12

**Inhibition by NST0037 of the activation of caspase 3/7 in a cell apoptosis model**

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum.

The induction of apoptosis on the cells in culture was performed by means of incubation with camptothecin (CPT), which triggers cell apoptosis activation. The apoptosis was determined by means of the measurement of the activation of effector caspases, caspase 3 or caspase 7, for which a kit for the fluorometric detection of active caspase 3/7 (Apo-ONE® Homogeneous Caspase-3/7, Promega) was used. The active caspase 3 or 7 of the cells cause the rupture of a substrate, which leads to fluorescence emission, which is read by means of a fluorometer. The effect of the pretreatment of compound NST0037 at 10 and 40 μM on the activation of caspase 3/7 caused by 50 μM of camptothecin (CPT) in the SK-N-MC cells was thus analyzed. These cells, not exceeding 15 passages, were seeded on 96-well plates treated for adherent cells with a cell concentration of $5x10^4$ cells/well, 3 wells of the plate were seeded for each condition of the assay. After 24 hours of cell incubation at 37°C and 5% $CO_2$, the pretreatment with NST0037 at 10 and 40 μM was performed.

After 24 hours, the cell treatments were performed with 100 μl of total volume for the following conditions:

- Control: culture medium (medium)
- Camptothecin (CPT): medium with CPT at 50 μM
- Camptothecin (CPT): medium with CPT at 50 μM, with different pretreatments with NST0037 (10 or 40 μM) and/or mevalonate (MEV; at 100 μM) or Z-VAD-fmk (50 μM).

The cells were incubated (at 37°C and 5% $CO_2$) with these treatments for 6 hours, after which the cell lysis buffer and the caspase substrate were added at the concentrations specified by the manufacturer; they were incubated at room temperature for 30 minutes and then frozen at -20°C overnight. On the following day, fluorescence was measured (499/521 nm, Exci/Emi).

Figure 34 shows the percentage of activation of caspase 3/7 with respect to the control cells of the different treatments. An inhibition of caspase 3/7 with respect to camptothecin of 26 and 33% at 10 and 40 μM of NST0037, respectively, was observed, which demonstrates a functional anti-apoptotic effect of this compound. In addition, mevalonate reverted this inhibition, therefore it is demonstrated that the effect of NST0037 on caspase 3/7 is related to cholesterol biosynthesis or to the biosynthesis of one of the precursors of the biosynthesis pathway thereof. Z-VAD-fmk, as a caspase inhibitor, completely inhibited the activation of caspase 3/7.

**EXAMPLE 13**

**Compound NST0037 reduces the production of β-amyloid peptide in a cell model**

The assay was performed on human neuroblastoma SK-N-MC cells in culture from the American Type Culture Collection (ATCC), stably transfected with a construct carried by the APP gene (beta-amyloid precursor protein, Gene ID: 351) in isoform 695, which is the most frequent variant expressed in neurons, whereby overexpression of the APP protein is achieved. In all cases strict rules of sterility were followed and the manipulation was performed in class II biological safety cabinets following European standard EN 12469. The cells were maintained in the following culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum, the expression of APP is selected by means of adding the antibiotic hygromycin B at 0.16 mg/mL.

These cells, not exceeding 15 passages, were seeded on 6-well plates treated for adherent cells with a cell concentration of $8x10^5$ cells/well, 2 wells of the plate were seeded for each condition of the assay. After 24 hours of cell incubation at 37°C and 5% $CO_2$, the cell treatments were performed with 2 ml of total volume for the following conditions:

- Control: culture medium (medium)
- NST0037: medium plus NST0037 at 1, 4, 10 ó 40 μM.

The cells were incubated (at 37°C and 5% $CO_2$) with these treatments for 48 hours, 500 μl of conditioned medium then being collected to perform the measurements. The cell remains were removed from the conditioned medium by means of centrifugation at 300xg and protease inhibitors (Mini EDTA-free, Roche) were then added. The two most frequent Aβ species, 1-40 and 1-42, were measured. For Aβ(1-42) it was necessary to concentrate the medium 5 times to perform the measurements, using the Amicon Ultra filtration system (Millipore). The amount of Aβ(1-40) and Aβ(1-42) of the conditioned medium was analyzed for each treatment by means of ELISA (Enzyme-linked immunosorbent assay), using the "beta amyloid 40 ELISA" and "beta amyloid 42 ELISA" kit (Biosource), following the recommendations of the manufacturer. The results were quantified by using an increasing concentration curve with synthetic peptides. The baseline values of secreted Aβ in these cells are 1340±141 pg/ml for Aβ(1-40) and 20±3 pg/mL for Aβ(1-42).

The results obtained for the treatments are shown in Figure 35, which shows the percentage of Aβ(1-40) (A) and Aβ(1-42) (B) with respect to the control cells at 48 hours. For Aβ(1-40) a reduction is obtained after the treatment with 10, 40 and 100 μM of NST0037. For Aβ(1-42) a reduction is obtained both at 10 and at 40 μM of NST0037.

From these results it is concluded that NST0037 reduces the secretion of Aβ in human neuroblastoma cells which overexpress APP. The production of Aβ has been related to cell death both in vitro and in vivo and is furthermore associated with the senile plaques present in patients with Alzheimer's disease.

**EXAMPLE 14**

**Effect of mevalonate on the protection by NST0037 against neuronal death induced by oxidative stress**

The assay was performed on human neuroblastoma SK-N-MC cells in culture from the American Type Culture Collection (ATCC), in all cases strict rules of sterility were followed and the manipulation was performed in class II biological safety cabinets following European standard EN 12469. The cells were maintained in the following culture medium culture medium: Minimum Essential Medium Eagle (MEM) supplemented with 1 mM sodium pyruvate, 2 mM L-glutamine, 0.1 mM non-essential amino acids, 0.05 mg/ml gentamicin and 10% fetal bovine serum

The effect of mevalonate on the protection caused by compound NST0037 against cell death caused by the treatment with xanthine/xanthine oxidase generating oxidative damage and cell death was analyzed. These cells, not exceeding 15 passages, were seeded on 96-well plates treated for adherent cells with a cell concentration of $5x10^4$ cells/well; 3 wells of the plate were seeded for each condition of the assay.

After 24 hours of cell incubation at 37°C and 5% $CO_2$, the cell treatments were performed with 100 μl of total volume for the following conditions:

- Control: culture medium
- Xanthine/xanthine oxidase (XXO): medium plus 10 μM xanthine/60 mU/mL xanthine oxidase, causing the death of 50% of the cells.
- XXO plus NST0037: medium plus XXO (10 μM/60 mU/mL) plus NST0037 at 40 μM.
- XXO plus mevalonate: medium plus XXO (10 μM/60 mU/mL) plus mevalonate at 10, 40 or 100 μM.
- XXO plus NST0037 plus mevalonate: medium plus XXO (10 μM/60 mU/mL) plus NST0037 at 40 μM plus mevalonate at 10, 40 or 100 μM.

The cells were incubated (at 37°C and 5% $CO_2$) with the treatments for 22 hours, after which time the WST-1 reagent (Roche) was added. The Test WST-1 is based on the measurement of metabolic activity. Cell damage causes the loss of the ability of cells to obtain the energy necessary to maintain their metabolic functions and cell growth, therefore the metabolically active (live) cells reduce tetrazolium salt to formazan by means of the succinate-tetrazolium reductase system (of the mitochondrial respiratory chain). The formazan which is formed can be detected colorimetrically since it has an absorbance of 440 nm. The reading was taken in a plate reader at 440 nm 2 hours after adding the reagent.

The obtained results are shown, as can be seen in Figure 36, as the percentage of cell death for each treatment relating to death caused by XXO. It was observed that mevalonate alone does not protect against death by XXO, nor does it cause increased toxicity, whereas NST0037 at 40 μM protected 57% against death and this effect was inhibited by adding mevalonate at 10, 40 and 100 μM, the differences being statistically significant, according to the Student's t test, for 10 and 100 μM. These results indicate that the observed protection of compound NST0037 is related to cholesterol biosynthesis or to the biosynthesis of one of the precursors of the pathway.

**EXAMPLE 15**

**Protection by NST0037 of the neuropathological signs associated with neuronal death in the hippocampus**

15.1. Protective effect of NST0037 against neuritic dystrophy caused by an excitotoxic substance in the hippocampus

Based on the neuroprotection results, the inventors decided to investigate if the neuroprotective effect of NST0037 in the hippocampus demonstrated in a model of sporadic Alzheimer's disease in mice by means of the administration of kainate (KA) was accompanied by the protection of another sign of neuronal damage such as neuritic dystrophy.

All the animals included for the experimental process were 12-week old males of the FVB/NHan strain. The experiments were conducted strictly following the Guidance on the Operation of Animals (Scientific Procedures, Act. 1986*).* The animals had their respective quarantine period and were treated with maximum precaution to minimize possible contaminations during inoculations and handling.

Twenty-five animals were used in this assay and the treatments were all conducted by intraperitoneal (i.p.) route with a volume of 100 μl, according to the following regimens:

i) PBS+PBS+PBS regimen: 4 animals were initially treated with PBS with a daily dose for 2 days, on the second day the animals were inoculated with a new dose of PBS and for the next 7 days with a daily dose of PBS.

ii) NST0037+KA+NST0037 regimen: 7 animals were initially treated with a daily dose for 2 days with NST0037 at 50 mg/kg, on the second day the animals were inoculated with a dose of kainate at 25 mg/Kg and for the next 7 days with a daily dose of NST0037 at 50 mg/kg.

iii) PBS+KA+PBS regimen: 7 animals were initially treated with PBS with a daily dose for 2 days, on the second day the animals were inoculated with a dose of kainate at 25 mg/Kg and for the next 7 days with a daily dose of PBS.

iv) PBS+KA+NST0037 regimen: 7 animals were initially treated with PBS with a daily dose for 2 days, on the second day the animals were inoculated with a dose of kainate at 25 mg/Kg and for the next 7 days with a daily dose of NST0037 at 50 mg/kg.

Once the treatment time of 7 days ended, the animals were sacrificed and the brains were dissected. The brain samples were processed and included in paraffin. For the analysis of neuritic dystrophy in the hippocampus, bright field immunochemistry against microtubule associated protein type 2 (MAP2) was performed in 5 μm thick coronal sections.

As shown in Figure 37, the PBS+KA+PBS administration regimen causes a decrease of MAP2 labeling, especially in the CA1, CA3 and dentate gyrus areas of the hippocampus, with respect to the PBS+PBS+PBS administration regimen showing a uniform labeling. However, the samples of the NST0037+KA+NST0037 group showed a staining pattern identical to the controls (PBS+PBS+PBS group) without evidence of neuritic dystrophy in these regions of the hippocampus. Furthermore, it is surprisingly observed that the treatment with NST0037 after the pretreatment with PBS and the inoculation of KA clearly reduces the loss of MAP2 labeling in the hippocampus.

In summary, the treatment with NST0037, both before and after the inoculation of KA, protects against the neuritic dystrophy caused by the latter in the hippocampus.

15.2. Protective effect of NST0037 against oxidative damage caused by an excitotoxic substance in the hippocampus

Based on the neurodegeneration and neuritic dystrophy protection results, the inventors decided to investigate if the neuroprotective effect of NST0037 in the hippocampus demonstrated in a model of sporadic Alzheimer's disease in mice by means of the administration of kainate (KA) was accompanied by the protection from another sign of neuronal

damage such as oxidative damage.

All the animals included for the experimental process were 12-week old males of the FVB/NHan strain. The experiments were conducted strictly following the Guidance on the Operation of Animals (Scientific Procedures, Act. 1986*)*. The animals had their respective quarantine period and were treated with maximum precaution to minimize possible contaminations during inoculations and handling.

Twenty-five animals were used in this assay and the treatments were all conducted by intraperitoneal (i.p.) route with a volume of 100 μl, according to the following regimens:

i) PBS+PBS+PBS regimen: 4 animals were initially treated with PBS with a daily dose for 2 days, on the second day the animals were inoculated with a new dose of PBS and for the next 7 days with a daily dose of PBS.
ii) NST0037+KA+NST0037 regimen: 7 animals were initially treated with a daily dose for 2 days with NST0037 at 50 mg/kg, on the second day the animals were inoculated with a dose of kainate at 25 mg/Kg and for the next 7 days with a daily dose of NST0037 at 50 mg/kg.
iii) PBS+KA+PBS regimen: 7 animals were initially treated with PBS with a daily dose for 2 days, on the second day the animals were inoculated with a dose of kainate at 25 mg/Kg and for the next 7 days with a daily dose of PBS.
iv) PBS+KA+NST0037 regimen: 7 animals were initially treated with PBS with a daily dose for 2 days, on the second day the animals were inoculated with a dose of kainate at 25 mg/Kg and for the next 7 days with a daily dose of NST0037 at 50 mg/kg.

Once the treatment time of 7 days ended, the animals were sacrificed and the brains were dissected. The brain samples were processed and included in paraffin. For the analysis of oxidative damage due to lipid peroxidation in the hippocampus, bright field immunochemistry against 4-hydroxynonenal (HNE) was performed in 5 μm thick coronal sections.

As shown in Figure 38, the PBS+KA+PBS administration regimen causes an increase of the HNE signal in the hippocampal CA3 region with respect to the PBS+PBS+PBS administration regimen which does not show this lipid peroxidation labeling. However, the samples of the NST0037+KA+NST0037 group showed a staining pattern identical to the controls (PBS+PBS+PBS group) without HNE-labeled neurons in these regions of the hippocampus. Furthermore, it is surprisingly observed that the treatment with NST0037 after the pretreatment with PBS and the inoculation of KA reduces the number of HNE-labeled neurons in the hippocampus.

In summary, the treatment with NST0037, both before and after the inoculation of KA, protects against the oxidative damage induced by the latter in the hippocampus.

<u>15.3. Protective effect of NST0037 against apoptosis caused by an excitotoxic substance in the hippocampus</u>

Based on the previous results, the inventors decided to investigate if the neuroprotective effect of NST0037 in the hippocampus demonstrated in a model of sporadic Alzheimer's disease in mice by means of the administration of kainate (KA) was accompanied by the protection against death by apoptosis, a mechanism which is associated with the disease.

All the animals included for the experimental process were 12-week old males of the FVB/NHan strain. The experiments were conducted strictly following the Guidance on the Operation of Animals (Scientific Procedures, Act. 1986*)*. The animals had their respective quarantine period and were treated with maximum precaution to minimize possible contaminations during inoculations and handling.

Twenty-five animals were used in this assay and the treatments were all conducted by intraperitoneal (i.p.) route with a volume of 100 μl, according to the following regimens:

i) PBS+PBS+PBS regimen: 4 animals were initially treated with PBS with a daily dose for 2 days, on the second day the animals were inoculated with a new dose of PBS and for the next 7 days with a daily dose of PBS.
ii) NST0037+KA+NST0037 regimen: 7 animals were initially treated with a daily dose for 2 days with NST0037 at 50 mg/kg, on the second day the animals were inoculated with a dose of kainate at 25 mg/Kg and for the next 7 days with a daily dose of NST0037 at 50 mg/kg.
iii) PBS+KA+PBS regimen: 7 animals were initially treated with PBS with a daily dose for 2 days, on the second day the animals were inoculated with a dose of kainate at 25 mg/Kg and for the next 7 days with a daily dose of PBS.
iv) PBS+KA+NST0037 regimen: 7 animals were initially treated with PBS with a daily dose for 2 days, on the second day the animals were inoculated with a dose of kainate at 25 mg/Kg and for the next 7 days with a daily dose of NST0037 at 50 mg/kg.

Once the treatment time of 7 days ended, the animals were sacrificed and the brains were dissected. The brain samples were processed and included in paraffin. For the analysis of the presence of neurons in apoptosis in the

hippocampus, the T.U.N.E.L. (TdT-mediated dUTP nick end labeling) fluorescence technique was performed in 5 μm thick coronal sections.

The PBS+KA+PBS administration regimen causes neuronal death by apoptosis in the hippocampus and especially in the CA1, CA3 (Figure 38) and dentate gyrus regions. However, the samples of the NST0037+KA+NST0037 group did not show neurons positive for T.U.N.E.L., showing a pattern identical to the controls (PBS+PBS+PBS group). It is additionally observed that the treatment with NST0037 after the pretreatment with PBS and the inoculation of KA reduces the number of neurons in apoptosis to a few isolated cells in the hippocampal CA3 region.

In summary, the treatment with NST0037, both before and after the inoculation of KA, protects against the oxidative damage and against the apoptosis induced by the latter in hippocampal neurons.

<u>15.4. Protective effect of NST0037 against astrogliosis caused by an excitotoxic substance in the hippocampus</u>

Based on the previous results, the inventors decided to investigate if the antioxidant and antiapoptotic effect shown by NST0037 in the hippocampus demonstrated in a model of sporadic Alzheimer's disease in mice by means of the administration of kainate (KA) was accompanied by the reduction of reactive astrogliosis, which is another histopathological sign which is detected in the brain of patients with Alzheimer's disease.

All the animals included for the experimental process were 12-week old males of the FVB/NHan strain. The experiments were conducted strictly following the Guidance on the Operation of Animals (Scientific Procedures, Act. 1986*)*. The animals had their respective quarantine period and were treated with maximum precaution to minimize possible contaminations during inoculations and handling.

Twenty-five animals were used in this assay and the treatments were all conducted by intraperitoneal (i.p.) route with a volume of 100 μl, according to the following regimens:

i) PBS+PBS+PBS regimen: 4 animals were initially treated with PBS with a daily dose for 2 days, on the second day the animals were inoculated with a new dose of PBS and for the next 7 days with a daily dose of PBS.

ii) NST0037+KA+NST0037 regimen: 7 animals were initially treated with a daily dose for 2 days with NST0037 at 50 mg/kg, on the second day the animals were inoculated with a dose of kainate at 25 mg/Kg and for the next 7 days with a daily dose of NST0037 at 50 mg/kg.

iii) PBS+KA+PBS regimen: 7 animals were initially treated with PBS with a daily dose for 2 days, on the second day the animals were inoculated with a dose of kainate at 25 mg/Kg and for the next 7 days with a daily dose of PBS.

iv) PBS+KA+NST0037 regimen: 7 animals were initially treated with PBS with a daily dose for 2 days, on the second day the animals were inoculated with a dose of kainate at 25 mg/Kg and for the next 7 days with a daily dose of NST0037 at 50 mg/kg.

Once the treatment time of 7 days ended, the animals were sacrificed and the brains were dissected. The brain samples were processed and included in paraffin. The analysis of reactive astrogliosis was performed by means of bright field immunochemistry against Glial fibrillary acidic protein (GFAP) in 5 μm thick coronal sections, which protein is present in astrocytes.

The PBS+KA+PBS administration regimen causes a significant increase of the activation and propagation of astrocytes in the neuropil of the hippocampus (Figure 38). In contrast, the samples of the NST0037+KA+NST0037 group showed an astrocyte pattern very similar to that of the controls (PBS+PBS+PBS group). Surprisingly, it is also observed that the treatment with NST0037 after the pretreatment with PBS and the inoculation of KA reduces the number of astrocytes and the intensity of the GFAP labeling in the hippocampus.

In summary, the treatment with NST0037, both before and after the inoculation of KA, protects against oxidative damage, against apoptosis and prevents the reactive astrogliosis induced by the latter in the hippocampal region.

**EXAMPLE 16**

**<u>Protection by NST0037 against clinical symptoms, locomotor deficits, neurodegeneration and neuronal death caused by a parkinsonian neurotoxin administered in an acute manner</u>**

Based on the neuroprotection results demonstrated by the compound NST0037 in a model of sporadic Alzheimer's disease in mice, the investigators decided to evaluate the neuroprotective capacity of the compound in a model of Parkinson's disease based on the death of dopaminergic neurons induced by a neurotoxic substance such as 1-methyl-4-phenyl-1,2,3,6-tetrahydropyridine (MPTP). The systemic injection of MPTP in mice induces a massive death of the dopaminergic neurons of the substantia nigra, which reduces the concentration of dopamine in this region and in others such as the striatum that are highly linked to locomotor activity in humans. Due to these phenomena, this model has been widely used to study Parkinson's disease since it reproduces one of the central events in said disease such

as motor deterioration. This example presents the results of the analysis of the effect of NST0037 against neurodegeneration and the symptoms induced by MPTP in an acute administration.

16.1. Protective effect of NST0037 against death caused by the acute administration of a neurotoxic substance inducing the death of dopaminergic neurons

All the animals included for the experimental process were 12-week old males of the CD1 strain. The experiments were conducted strictly following the Guidance on the Operation of Animals (Scientific Procedures, Act. 1986*).* The animals had their respective quarantine period and were treated with maximum precaution to minimize possible contaminations during inoculations and handling.
Forty animals were used in this assay and the treatments were conducted with a volume of 100 μl, according to the following regimens:

i) PBS+PBS+PBS regimen: 10 animals were initially treated with PBS with a daily dose for 2 days by subcutaneous (s.c.) route, on the second day the animals were inoculated with 4 doses of PBS, at intervals of 2 hours by intraperitoneal (i.p.) route. For the next 7 days they were administered a daily dose of PBS by s.c. route.
ii) PBS+MPTP+PBS regimen: 10 animals were initially treated with a daily dose for 2 days of PBS by s.c. route, on the second day the animals were inoculated with 4 doses of MPTP at 20 mg/Kg, at intervals of 2 hours by i.p. route. For the next 7 days they were administered a daily dose of PBS by s.c. route.
iii) NST0037+MPTP+NST0037 regimen: 10 animals were initially treated with a daily dose for 2 days of NST0037 at 50 mg/kg by s.c. route, on the second day the animals were inoculated with 4 doses of MPTP at 20 mg/Kg, at intervals of 2 hours by i.p. route. For the next 7 days they were administered a daily dose of NST0037 at 50 mg/kg by s.c. route.

The deaths in the different treatment groups were recorded during the entire experimental procedure, with these data the corresponding survival curves presented in Figure 39 were made.
The PBS+MPTP+PBS administration regimen causes a statistically significant increase ($p<0.05$) of the mortality in the mice. However, the treatment with NST0037 surprisingly promotes the survival of the animals after the acute administration of MPTP since no significant differences were observed between the NST0037+MPTP+NST0037 group and the PBS+PBS+PBS group ($p>0.05$).

16.2. Protective effect of NST0037 against the decline in motor resistance caused by the acute administration of a neurotoxic substance inducing the death of dopaminergic neurons

All the animals included for the experimental process were 12-week old males of the CD1 strain. The experiments were conducted strictly following the Guidance on the Operation of Animals (Scientific Procedures, Act. 1986*).* The animals had their respective quarantine period and were treated with maximum precaution to minimize possible contaminations during inoculations and handling.
Forty animals were used in this assay and the treatments were conducted with a volume of 100 μl, according to the following regimens:

i) PBS+PBS+PBS regimen: 10 animals were initially treated with PBS with a daily dose for 2 days by subcutaneous (s.c.) route, on the second day the animals were inoculated with 4 doses of PBS, at intervals of 2 hours by intraperitoneal (i.p.) route. For the next 7 days they were administered a daily dose of PBS by s.c. route.
ii) PBS+MPTP+PBS regimen: 10 animals were initially treated with a daily dose for 2 days of PBS by s.c. route, on the second day the animals were inoculated with 4 doses of MPTP at 20 mg/Kg, at intervals of 2 hours by i.p. route. For the next 7 days they were administered a daily dose of PBS by s.c. route.
iii) NST0037+MPTP+NST0037 regimen: 10 animals were initially treated with a daily dose for 2 days of NST0037 at 50 mg/kg by s.c. route, on the second day the animals were inoculated with 4 doses of MPTP at 20 mg/Kg, at intervals of 2 hours by i.p. route. For the next 7 days they were administered a daily dose of NST0037 at 50 mg/kg by s.c. route.

Before starting the treatments and once the treatment time of 7 days ended, the animals were subjected to a locomotor resistance test referred to as the Rotarod test for 2 minutes, recording the time the animals resisted walking over a cylinder which increased its speed from 4 to 40 r.p.m. Figure 40 shows the ratio between the end of the assay and the baseline state, of the time that the animals of the different groups remain walking over the cylinder.
The PBS+MPTP+PBS administration regimen causes a statistically significant decrease of the motor resistance in the mice from their baseline state to 7 days after the acute administration of MPTP ($p<0.05$). However, the treatment

with NST0037 surprisingly prevents the motor deterioration of the animals, maintaining the resistance of the mice at the level that they showed before the administration of MPTP ($p>0.05$), a pattern which is identical to that of the mice not injected with MPTP and treated with PBS ($p>0.05$).

In summary, the treatment with NST0037 protects against the loss of resistance induced by the acute administration of MPTP.

16.2. Protective effect of NST0037 against the decrease of strength caused by the acute administration of a neurotoxic substance inducing the death of dopaminergic neurons

All the animals included for the experimental process were 12-week old males of the CD1 strain. The experiments were conducted strictly following the *Guidance on the Operation of Animals (Scientific Procedures, Act. 1986).* The animals had their respective quarantine period and were treated with maximum precaution to minimize possible contaminations during inoculations and handling.

Forty animals were used in this assay and the treatments were conducted with a volume of 100 μl, according to the following regimens:

i) PBS+PBS+PBS regimen: 10 animals were initially treated with PBS with a daily dose for 2 days by subcutaneous (s.c.) route, on the second day the animals were inoculated with 4 doses of PBS, at intervals of 2 hours by intraperitoneal (i.p.) route. For the next 7 days they were administered a daily dose of PBS by s.c. route.
ii) PBS+MPTP+PBS regimen: 10 animals were initially treated with a daily dose for 2 days of PBS by s.c. route, on the second day the animals were inoculated with 4 doses of MPTP at 20 mg/Kg, at intervals of 2 hours by i.p. route. For the next 7 days they were administered a daily dose of PBS by s.c. route.
iii) NST0037+MPTP+NST0037 regimen: 10 animals were initially treated with a daily dose for 2 days of NST0037 at 50 mg/kg by s.c. route, on the second day the animals were inoculated with 4 doses of MPTP at 20 mg/Kg, at intervals of 2 hours by i.p. route. For the next 7 days they were administered a daily dose of NST0037 at 50 mg/kg by s.c. route.

Before starting the treatments and once the treatment time of 7 days ended, the animals were subjected to a strength test in the so-called Grip-strength test, recording the strength of gripping a bar in grams exerted by the animals with the front paws in triplicate. Figure 41 shows the ratio between the strength shown by the animals at the end of the assay with respect to that of the baseline state.

The PBS+MPTP+PBS administration regimen causes a statistically significant decrease of the strength exerted by the mice upon gripping wit the front paws from their baseline state to 7 days after the acute administration of MPTP ($p<0.05$). Furthermore, the treatment with NST0037 prevents the loss of strength induced by MPTP and after 7 days of treatment the mice of the NST0037+MPTP+NST0037 group show strength levels identical to those before the administration of MPTP ($p>0.05$), a pattern which is very similar to that of mice not injected with MPTP and treated with PBS ($p>0.05$).

In summary, the treatment with NST0037 protects against the loss of resistance and strength induced by the acute administration of MPTP.

16.3. Protective effect of NST0037 against the neurodegeneration caused by the acute administration of a neurotoxic substance inducing the death of dopaminergic neurons

Based on the previous results, the inventors wished to determine if the protection shown by compound NST0037 against the deterioration of the psychomotor state caused by MPTP would be accompanied by the neurodegeneration in brain regions involved in locomotor activity and related to Parkinson's disease such as the substantia nigra and the striatum.

All the animals included for the experimental process were 12-week old males of the CD1 strain. The experiments were conducted strictly following the Guidance on the Operation of Animals (Scientific Procedures, Act. 1986*).* The animals had their respective quarantine period and were treated with maximum precaution to minimize possible contaminations during inoculations and handling.

Forty animals were used in this assay and the treatments were conducted with a volume of 100 μl, according to the following regimens:

i) PBS+PBS+PBS regimen: 10 animals were initially treated with PBS with a daily dose for 2 days by subcutaneous (s.c.) route, on the second day the animals were inoculated with 4 doses of PBS, at intervals of 2 hours by intraperitoneal (i.p.) route. For the next 7 days they were administered a daily dose of PBS by s.c. route.
ii) PBS+MPTP+PBS regimen: 10 animals were initially treated with a daily dose for 2 days of PBS by s.c. route, on

the second day the animals were inoculated with 4 doses of MPTP at 20 mg/Kg, at intervals of 2 hours by i.p. route. For the next 7 days they were administered a daily dose of PBS by s.c. route.

iii) NST0037+MPTP+NST0037 regimen: 10 animals were initially treated with a daily dose for 2 days of NST0037 at 50 mg/kg by s.c. route, on the second day the animals were inoculated with 4 doses of MPTP at 20 mg/Kg, at intervals of 2 hours by i.p. route. For the next 7 days they were administered a daily dose of NST0037 at 50 mg/kg by s.c. route.

Once the treatment time of 7 days ended, the animals were sacrificed and the brains were dissected. The brain samples were processed and included in paraffin. For the analysis of neurodegeneration in the substantia nigra and the striatum, fluorescent Fluoro Jade B (FJB) staining was performed in 5 μm thick sagittal sections. Figure 42 shows a representative photograph of each group and in both areas under study.

The PBS+MPTP+PBS administration regimen causes a clear increase of the cells positive for FJB both in the substantia nigra and in the striatum with respect to the samples of the PBS+PBS+PBS group. In contrast, the samples of the NST0037+MPTP+NST0037 group had a number of cells in degeneration and positive for FJB significantly lower than those of the PBS+MPTP+PBS group.

In summary, the treatment with NST0037 protects against psychomotor deterioration and against neurodegeneration, induced by the acute administration of MPTP, in dopaminergic neurons of the substantia nigra, as well as of the nerve endings innerving the striatum.

<u>16.4. Protective effect of NST0037 against the loss of dopaminergic neurons by the acute administration of a neurotoxic substance</u>

Based on the previous results, the inventors wished to determine if the protection shown by compound NST0037 against the neurodegeneration caused by MPTP was accompanied by the protection of dopaminergic neurons in deteriorated brain regions in Parkinson's disease such as the substantia nigra and the striatum.

All the animals included for the experimental process were 12-week old males of the CD1 strain. The experiments were conducted strictly following the Guidance on the Operation of Animals (Scientific Procedures, Act. 1986*)* *.* The animals had their respective quarantine period and were treated with maximum precaution to minimize possible contaminations during inoculations and handling.

Forty animals were used in this assay and the treatments were conducted with a volume of 100 μl, according to the following regimens:

i) PBS+PBS+PBS regimen: 10 animals were initially treated with PBS with a daily dose for 2 days by subcutaneous (s.c.) route, on the second day the animals were inoculated with 4 doses of PBS, at intervals of 2 hours by intraperitoneal (i.p.) route. For the next 7 days they were administered a daily dose of PBS by s.c. route.

ii) PBS+MPTP+PBS regimen: 10 animals were initially treated with a daily dose for 2 days of PBS by s.c. route, on the second day the animals were inoculated with 4 doses of MPTP at 20 mg/Kg, at intervals of 2 hours by i.p. route. For the next 7 days they were administered a daily dose of PBS by s.c. route.

iii) NST0037+MPTP+NST0037 regimen: 10 animals were initially treated with a daily dose for 2 days of NST0037 at 50 mg/kg by s.c. route, on the second day the animals were inoculated with 4 doses of MPTP at 20 mg/Kg, at intervals of 2 hours by i.p. route. For the next 7 days they were administered a daily dose of NST0037 at 50 mg/kg by s.c. route.

Once the treatment time of 7 days ended, the animals were sacrificed and the brains were dissected. The brain samples were processed and included in paraffin. For the analysis of the presence of dopaminergic neurons in the substantia nigra and the striatum, the immunohistochemistry against the tyrosine-hydroxylase (TH) protein was performed in 5 μm thick sagittal sections. Figure 43 shows a representative photograph of each group and in both areas under study.

The PBS+MPTP+PBS administration regimen causes an evident decrease of the amount of dopaminergic neurons since a clear absence of TH labeling is observed in comparison with the PBS+PBS+PBS group, both in the neuronal bodies of the substantia nigra and in the nerve extensions in the striatum. In contrast, the samples of the NST0037+MPTP+NST0037 group had a larger number of TH-positive cells in the substantia nigra as well as a higher labeling intensity in the striatum than those of the PBS+MPTP+PBS group.

In summary, the treatment with NST0037 protects against the psychomotor deterioration of the mice, in addition to the neurodegeneration and the neuronal death induced by the acute administration of MPTP, in the substantia nigra and the striatum.

## EXAMPLE 17

**Protection by NST0037 against locomotor deficits, against neuronal death and against oxidative damage caused by parkinsonian neurotoxin administered in a subchronic manner**

Due to the results obtained and presented in Example 16, the inventors decided to analyze the effect of NST0037 on clinical symptoms and on the neuropathology induced in the substantia nigra by the subchronic administration of MPTP in mice.

17.1. Protective effect of NST0037 against the decline in motor resistance caused by the subchronic administration of a neurotoxic substance inducing the death of dopaminergic neurons

All the animals included for the experimental process were 12-week old males of the CD1 strain. The experiments were conducted strictly following the *Guidance on the Operation of Animals (Scientific Procedures, Act. 1986).* The animals had their respective quarantine period and were treated with maximum precaution to minimize possible contaminations during inoculations and handling.

Twenty animals were used in this assay and the treatments were all conducted by intraperitoneal (i.p.) route with a volume of 100 μl, according to the following regimens:

i) PBS+MPTP+PBS regimen: 10 animals were initially treated with a daily dose for 2 days of PBS, from the second day onwards the animals were inoculated with 1 daily dose of MPTP at 30 mg/Kg for 5 days, at intervals of 2 hours by i.p. route. For the next 21 days they were administered 3 weekly doses of PBS.

ii) NST0037+MPTP+NST0037 regimen: 10 animals were initially treated with a daily dose for 2 days of NST0037 at 50 mg/kg, from the second day onwards the animals were inoculated with 1 daily dose of MPTP at 30 mg/Kg for 5 days, at intervals of 2 hours by i.p. route. For the next 21 days they were administered 3 weekly doses of NST0037 at 50 mg/kg.

Seven days before starting the treatments and at 7, 14 and 21 days post-inoculation of MPTP, the animals were subjected to a locomotor resistance test referred to as the Rotarod test for 2 minutes, recording the time the animals resisted walking over a cylinder which increased its speed from 4 to 40 r.p.m. Figure 44 shows the ratio between the different time points of the assay and the baseline state, of the time that the animals of the different groups remain walking over the cylinder.

The PBS+MPTP+PBS administration regimen causes a statistically significant decrease of the motor resistance in the mice from their baseline state starting from 14 days after the subchronic administration of MPTP ($p<0.05$). However, the treatment with NST0037 surprisingly prevents the motor deterioration of the animals, maintaining the resistance of the mice at the level that they showed before the administration of MPTP ($p>0.05$).

In summary, the treatment with NST0037 protects against the loss of resistance induced by the subchronic administration of MPTP.

17.2. Protective effect of NST0037 against the loss of dopaminergic neurons due to the subchronic administration of a neurotoxic substance

Based on the previous results, the inventors wished to determine if the protection shown by compound NST0037 against the deterioration of the psychomotor state caused by MPTP was accompanied by neurodegeneration in one of the most important brain regions which is involved in Parkinson's disease such as the substantia nigra.

All the animals included for the experimental process were 12-week old males of the CD1 strain. The experiments were conducted strictly following the Guidance on the Operation of Animals (Scientific Procedures, Act. 1986*).* The animals had their respective quarantine period and were treated with maximum precaution to minimize possible contaminations during inoculations and handling.

Twenty animals were used in this assay and the treatments were all conducted by intraperitoneal (i.p.) route with a volume of 100 μl, according to the following regimens:

i) PBS+MPTP+PBS regimen: 10 animals were initially treated with a daily dose for 2 days of PBS, from the second day onwards the animals were inoculated with 1 daily dose of MPTP at 30 mg/Kg for 5 days, at intervals of 2 hours by i.p. route. For the next 21 days they were administered 3 weekly doses of PBS.

ii) NST0037+MPTP+NST0037 regimen: 10 animals were initially treated with a daily dose for 2 days of NST0037 at 50 mg/kg, from the second day onwards the animals were inoculated with 1 daily dose of MPTP at 30 mg/Kg for 5 days, at intervals of 2 hours by i.p. route. For the next 21 days they were administered 3 weekly doses of NST0037

at 50 mg/kg.

Once the treatment time of 21 days ended, the animals were sacrificed and the brains were dissected. The brain samples were processed and included in paraffin. For the analysis of the presence of dopaminergic neurons in the substantia nigra, the immunohistochemistry against the tyrosine-hydroxylase (TH) protein was performed in 5 μm thick sagittal sections. Figure 45 shows a representative photograph of each group.

The PBS+MPTP+PBS administration regimen causes an evident loss of dopaminergic neurons since a clear absence of TH labeling is observed in some substantia nigra regions. In contrast, the samples of the NST0037+MPTP+NST0037 group had a larger number of TH-positive cells in the same substantia nigra region.

In summary, the treatment with NST0037 protects against the psychomotor deterioration of the mice, in addition to the neuronal death induced in the substantia nigra by the subchronic administration of MPTP.

<u>17.3. Protective effect of NST0037 against oxidative damage induced by the subchronic administration of a neurotoxic substance for dopaminergic neurons</u>

Based on the previous results, the inventors wished to determine if the neuroprotection shown by compound NST0037 against the neurodegeneration caused by MPTP in the substantia nigra was accompanied by the reduction of another histopathological sign of neuronal damage such as oxidative damage.

All the animals included for the experimental process were 12-week old males of the CD1 strain. The experiments were conducted strictly following the Guidance on the Operation of Animals (Scientific Procedures, Act. 1986*).* The animals had their respective quarantine period and were treated with maximum precaution to minimize possible contaminations during inoculations and handling.

Twenty animals were used in this assay and the treatments were all conducted by intraperitoneal (i.p.) route with a volume of 100 μl, according to the following regimens:

i) PBS+MPTP+PBS regimen: 10 animals were initially treated with a daily dose for 2 days of PBS, from the second day onwards the animals were inoculated with 1 daily dose of MPTP at 30 mg/Kg for 5 days, at intervals of 2 hours by i.p. route. For the next 21 days they were administered 3 weekly doses of PBS.

ii) NST0037+MPTP+NST0037 regimen: 10 animals were initially treated with a daily dose for 2 days of NST0037 at 50 mg/kg, from the second day onwards the animals were inoculated with 1 daily dose of MPTP at 30 mg/Kg for 5 days, at intervals of 2 hours by i.p. route. For the next 21 days they were administered 3 weekly doses of NST0037 at 50 mg/kg.

Once the treatment time of 21 days ended, the animals were sacrificed and the brains were dissected. The brain samples were processed and included in paraffin. For the analysis of lipid peroxidation in the neurons of the substantia nigra, bright field immunohistochemistry against 4-hydroxynonenal (HNE) was performed in 5 μm thick sagittal sections. Figure 45 shows a photograph of each group.

The PBS+MPTP+PBS administration regimen induces the presence of labeling against HNE in a number of neurons of the substantia nigra. In contrast, the samples of the NST0037+MPTP+NST0037 group have a modest number of isolated neurons with HNE-positive labeling in the substantia nigra.

In summary, the treatment with NST0037 protects against the psychomotor deterioration of the mice, in addition to neuronal death and oxidative damage due to lipid peroxidation induced in the substantia nigra by the subchronic administration of MPTP.

## EXAMPLE 18

**<u>study of the blood-brain barrier passage of the acid form of NST0037 in an *in vitro* assay (PAMPA)</u>**

The object of the assay was to predict if compound NST0037, in comparison with simvastatin and atorvastatin, in the active forms thereof, was capable of crossing the blood-brain barrier (BBB), for which said barrier was mimicked in an *in vitro* system which allowed evaluating the compound without using cells. To that end, the assay referred to as PAMPA (Parallel Artificial Membrane Permeation Assay), which uses a sandwich system contemplating the passage of compounds by means of passive diffusion, was carried out. Verapamil, a compound with high permeability, was used as positive control, whereas theophylline, a compound which does not cross the BBB, was used as negative control.

To mimic the BBB, a commercial mixture of lipids derived from pig brain with a composition of phospholipids very similar to that which forms the barrier, and which is referred to as PBL (Porcine Polar Brain Lipid), was used. Verapamil, theophylline and atorvastatin were prepared at 10 mM in DMSO, whereas NST0037 and simvastatin were prepared in water and activated with 0.1 N NaOH at 4°C for 12 hours.

At the time of the assay, 1.5 mL of the compounds to be evaluated and of the controls were prepared, in all cases at 100 μM from the stocks in a phosphate buffer at pH 7.4 which contained monobasic sodium phosphate (0.41 M) and dibasic potassium phosphate (0.287 M). To that end, a 1/100 dilution of the compounds was carried out, equaling the content of DMSO at 1% in all cases.

In a 96-well filter plate, with a PVDF membrane, with a 45 μm pore size (MAIPN4550, Millipore), 5 μL of PBL at 20 μg/mL were added. After two minutes, 300 μL of the phosphate buffer used to dilute the compounds were added. This plate was considered the acceptor plate and was placed in the upper part of the sandwich. On another 96-well plate which was perfectly assembled with the previous one (MATRNP550 from Millipore), 300 μL of the compounds at 100 μM were added in triplicate. Furthermore, a blank which only included 1% DMSO in the phosphate buffer used was included. This plate was referred to as donor plate. The acceptor plate was carefully placed on the donor plate, forming the sandwich system. The compounds object of study diffused from the wells of the donor plate to the corresponding wells of the acceptor plate during the 18 hours in which was the system remained intact. The remaining compound prepared was preserved in the same conditions of humidity, temperature and darkness as the sandwich system formed by the plates. After this time, 100 μL of the wells of the donor plates and of the acceptor plates were transferred to a special 96-well plate for UV reading. Furthermore, 100 pL, in triplicate, of the compounds prepared to perform the assay were transferred and preserved in the same manner as the plates (baseline wells). The UV plate was introduced in a spectrophotometer in which a scan was carried out in UV from 230 to 498 nm, with readings every 4 nm. Based on the spectrophotometer data, the percentage of barrier passage as well as the effective permeability ($P_e$) were calculated. The following formula was applied to calculate $P_e$:

$$Pe = C \times -Ln\left(1 - \frac{[Drug]_{acceptor}}{[Drug]_{equilibrium}}\right)$$

Where:

$$C = \frac{V_D \times V_A}{(V_D \times V_A) \times Area \times Time}$$

$[Drug]_{acceptor}$ = Absorbance of the acceptor well
$[Drug]_{equilibrium}$ = Absorbance of the mean of the baseline wells/2
$V_A$= Volume of the acceptor well = 0.3 $cm^3$
$V_D$= Volume of the donor well = 0.3 $cm^3$
Area = 0.24 $cm^2$
Time = 64.000 s

The theoretical calculation of lipophilicity of a compound can be obtained by calculating the logarithm of the octanol/water partition coefficient, which is given by cLogP. cLogP was theoretically calculated by means of the CLOGP program (OSIRIS Property Explorer), entering the chemical structures into the software. The n for each compound is indicated in the table together with the numerical results of the mean±SD of the values obtained in the assay.

Table I

| | % BBB Passage | $P_e$ (cm/s) | n | cLogP |
|---|---|---|---|---|
| Simvastatin | 25.6±9.0 | $4.0\pm1.7x10^{-6}$ | 21 | 4.61 |
| Atorvastatin | 4.9±2.0 | $0.5\pm0.2x10^{-6}$ | 9 | 5.55 |
| NST0037 | 22.5±5.5 | $3.1\pm1.2x10^{-6}$ | 33 | 4.55 |

As can be observed in Table I and in Figure 46, both simvastatin and NST0037 had a very similar BBB passage level. In both cases, an intermediate BBB passage between verapamil ($P_e$=10±2 x $10^{-6}$ cm/s) and theophylline ($P_e$=0.2±0.1 x $10^{-6}$ cm/s) is expected for these compounds. These BBB passage data are confirmed by the lipophilicity range, shown by both compounds, and having a very similar cLogP. However, despite having a high cLogP, atorvastatin hardly crosses the BBB, doing so in the same range as the negative control, theophylline.

## EXAMPLE 19

**Effect of NST0037 on intercellular total cholesterol levels in human hepatic and neuronal cell lines**

The object of the assay was to determine to what extent compound NST0037, in comparison with simvastatin, was capable of modifying the total cholesterol levels in cell lines of neuronal and hepatic origin. To carry out this assay, the compounds were activated with NaOH until the lactone form was completely opened.

Both the Hep G2 (human hepatocarcinoma) line and the SK-N-MC (human neuroblastoma) line were obtained from the American Type Culture Collection (ATCC no. HB-8065 and HTB-10 respectively). In all cases strict rules of sterility were followed and the manipulation was performed in class II biological safety cabinets following European standard EN 12469. The cells maintained in passage were seeded in 96-well plates in MEM supplemented with 10% FBS, 2 mM L-glutamine, 1 mM sodium pyruvate, 0.1 mM non-essential amino acids and 0.05 mg/mL gentamicin. In the case of HepG2, $4x10^4$ cells/well were seeded and in the case of SK-N-MC $5x10^4$ cells/well were seeded. After 24 hours had elapsed from the seeding, the cells were deprived of FBS for 8 hours. The cells were then incubated with the treatments at various concentrations for 20 hours. After the incubation period, the cells were washed with PBS and lysed with a phosphate buffer with 0.5% Triton X-100. The lysis was completed by means of a double freezing-thawing.

The total cholesterol was quantified by means of enzymatic and fluorometric techniques.2 5 μL of each lysate were transferred to a 96-well plate together with a standard cholesterol curve starting from 10 μg/mL to 0.08 μg/mL. 75 μL of the reactive mixture prepared based on 0.05 M MES (pH 6.5) containing cholesterol oxidase (0.5 U/mL), cholesterol esterase (0.8 U/mL), horseradish peroxidase (4 U/mL) and ampliflu red (20 μg/mL) were added on the samples and incubation was performed for 15 minutes at 37°C. The fluorescence intensity was determined at 530 nm of excitation and 580 nm of emission by means of a fluorometer. The results were normalized by protein, which was determined by means of the BCA technique.

Figure 47 shows how NST0037 had a hypocholesterolemic effect in both cell types, although it was in the neuronal cells where the reductions of cholesterol were more drastic. In the case of HepG2, NST0037 was more potent than simvastatin, whereas in SK-N-MC, the reductions of cholesterol with both compounds were similar.

## EXAMPLE 20

**Hypocholesterolemic effect of NST0037 administered orally for 28 days in an endogenous hyperlipidemia model (familial)**

Based on the results previously presented on the hypocholesterolemic character of compound NST0037, the investigators decided to evaluate the effect of the oral administration over time of NST0037 in the same familial endogenous hyperlipidemia model (apoB100 mice) which was used in Example 5.2. The objective of this new study was to analyze if prolonged oral administration is effective for reducing cholesterol levels over time.

All the animals included for the experimental process were 12-week old female mice. The experiments were conducted strictly following the Guidance on the Operation of Animals (Scientific Procedures, Act. 1986*).* The animals had their respective quarantine period and were treated with maximum precaution to minimize possible contaminations for inoculations and handling.

The mice were divided into three groups: control group (n =8); simvastatin group (n = 8); NST0037 group (n = 8). After a conditioning to the diet, 50 mg/kg of simvastatin or NST0037 (in the lactone forms thereof) were orally administered to the mice at the same time (15.00 to 18.00h) for 28 consecutive days, using in both cases 0.25% carboxy methylcellulose in water as a vehicle. The control group received this same vehicle. Before starting the treatments and at 7, 21 and 28 days of starting them, retro-orbital blood extractions after fasting for 16 hours were carried out. The plasma was obtained from said blood, in which the total cholesterol (TC), free cholesterol (FC), cholesterol (EC), HDL-c, LDL-c, VLDL-c and apoB100 levels were evaluated by enzymatic and spectrophotometric techniques. In order to check if the effect at the lipid level involved an alteration of the oxidative stress, the redox state in the plasmas obtained was furthermore evaluated by means of the TEAC (Trolox Antioxidant Capacity Assay) technique. This technique determines the antioxidant capacity in vitro, giving an idea of the redox state of the sample analyzed by means of the absorbance capacity by electron transfer. The different components of the reaction, myoglobin, ABTS (2,2-azino-bis-(3-ethylbenzthiazoline-sulfonic acid), hydrogen peroxide and $H_2O_2$ were added to the plasmas diluted 1/10 in PBS (10 μL) in a 96-well plate. After 3 minutes of incubation, the absorbance was measured at 405 nm (with a reference of 600 nm).

Figure 48 shows that both treatments are capable of significantly reducing the plasma total cholesterol levels. This fact is mainly shown by a reduction of VLDL and HDL in addition to a smaller increase of LDL. Furthermore, the apolipoprotein B (apoB) levels were visibly reduced by the treatments. Figure 49 furthermore shows the reduction of the FC and EC levels after the treatments with compound NST0037 in comparison with simvastatin, this reduction being more pronounced after 21 days of treatment. As shown in Figure 50, it was additionally demonstrated that the treatments

with NST0037 and with simvastatin decrease the redox state of the plasmas of the animals.

In summary, the treatment with NST0037 for 28 days by means of the oral administration to mice with congenital hyperlipidemia causes a reduction of total cholesterol, esterified cholesterol, free cholesterol levels, of all the lipoprotein fractions associated with cholesterol, of the ApoB levels and of the redox state of the animals.

**EXAMPLE 21**

**Hypocholesterolemic effect of NST0037 administered orally for three months in an endogenous hyperlipidemia model (familial)**

Based on the results previously presented on the hypocholesterolemic character of compound NST0037, the investigators decided to evaluate the effect of the oral administration for 3 months of NST0037 in the same familial endogenous hyperlipidemia model (apoB100 mice).

All the animals included for the experimental process were 6-month old female mice. The experiments were conducted strictly following the Guidance on the Operation of Animals (Scientific Procedures, Act. 1986*).* The animals had their respective quarantine period and were treated with maximum precaution to minimize possible contaminations for inoculations and handling.

The animals were divided into three groups: control group (n =8); simvastatin group (n = 6); NST0037 group (n = 8). After a conditioning to the diet, 50 mg/kg of NST0021 or NST0037 were orally administered to the mice for 3 months, three times a week, using in both cases 0.25% carboxy methylcellulose in physiological saline as a vehicle. The control group received this same vehicle. Before starting the treatments and at one, two and three months of starting them, retro-orbital blood extractions after fasting for 16 hours were carried out. The plasma was obtained from said blood, in which the total cholesterol (TC), free cholesterol (FC), cholesterol (EC), HDL-c, LDL-c, VLDL-c levels were evaluated by enzymatic and spectrophotometric techniques.

Figure 51 shows how both treatments reduced the different cholesterol fractions. Nevertheless, NST0037 surprisingly reduced to a greater extent the LDL-c levels and increase the HDL-c levels, thus showing a better cardioprotective profile than simvastatin. These results correlated with the effects of the treatments on the FC and EC levels (Figure 52), in which it was seen that NST0037 causes a significant reduction of both FC and EC.

In summary, the treatment with NST0037 for 3 months by means of oral administration to mice with congenital hyperlipidemia causes a reduction of total cholesterol, esterified cholesterol, free cholesterol levels, as well as a reduction of the LDL-c levels and an increase of HDL-c, which demonstrates its cardioprotective power.

**EXAMPLE 22**

**Hypolipidemic effect of NST0037 in genetically obese Zucker rats**

Based on the results previously presented on the hypocholesterolemic character of compound NST0037 in mice, the investigators decided to evaluate the effect of the oral administration for 7 days of NST0037 in an endogenous hyperlipidemia model in genetically obese Zucker rats with abnormally high lipid levels.

All the animals included for the experimental process were 11 week-old male Zucker rats. The experiments were conducted strictly following the Guidance on the Operation of Animals (Scientific Procedures, Act. 1986*)* . The animals had their respective quarantine period and were treated with maximum precaution to minimize possible contaminations for inoculations and handling.

The animals were divided into three groups: control group (n =7); simvastatin group (n = 7); NST0037 group (n = 7). After a conditioning to the diet, 30 mg/kg of NST0021 or NST0037 were orally administered to the mice at the same time (15.00 to 18.00h) for 7 consecutive days, using in both cases 0.5% carboxy methylcellulose in water as a vehicle. The control group received this same vehicle. Before starting the treatments and at 7 days therefrom, blood was extracted by lingual puncture under anesthesia after fasting for 16 hours. The plasma was obtained from said blood, in which the total cholesterol, HDL-c, LDL-c, VLDL-c, and triglyceride (TG) levels were evaluated by enzymatic and spectrophotometric techniques. In order to check if the effect at the lipid level involved an alteration of the oxidative stress, the redox state in the plasmas obtained was furthermore evaluated by means of the TEAC (Trolox Antioxidant Capacity Assay) technique. This technique determines the antioxidant capacity *in vitro*, giving an idea of the redox state of the sample analyzed by means of the absorbance capacity by electron transfer. The different components of the reaction, myoglobin, ABTS (2,2-azino-bis-(3-ethylbenzthiazoline-sulfonic acid) and hydrogen peroxide were added to the plasmas diluted 1/10 in PBS (10 μL) in a 96-well plate. After 3 minutes of incubation, the absorbance was measured at 405 nm (with a reference of 600 nm).

Figure 53 shows that both simvastatin and NST0037 were capable of equally reducing the plasma LDL levels, a fact directly related to the inhibition of the HMGR activity, which leads to an increase of LDL receptor expression. In

this case, the effect of NST0037 reached statistical significance. Furthermore, both compounds caused an increase of HDL, which involves a cardioprotective effect. Figure 54 shows how the compounds reduced the plasma triglyceride levels, which indicates a hypotriglyceridemic effect. Furthermore, while in the control rats there was an increase of oxidative stress (Figure 55), determined by means of the redox state in plasma by means of TEAC, in the rats treated with simvastatin and NST0037 there was a drastic reduction, which indicates an antioxidant effect. In this case, the results were only statistically significant in the case of NST0037.

In summary, the treatment with NST0037 for 7 days by means of oral administration to genetically obese Zucker rats with endogenous hyperlipidemia caused a reduction of the LDL-c levels, of triglycerides, and of the redox state, which demonstrates its cardioprotective power.

## EXAMPLE 23

**Effect of NST0037 on the expression of the 24-dehydrocholesterol reductase (Seladin-I/DHCR24) gene in wild-type mice**

Based on the results presented in Example 9 on the induction of the seladin-1/DHCR24 gene in human neuronal lines, the investigators decided to analyze if NST0037 modulated said gene in brains of mice treated with this compound.

In order to carry out this study, 3-month old male C57BL6 mice (n=3/group) were orally treated with 50 mg/kg of simvastatin or NST0037. A control group received only the vehicle, which consisted of 0.25% carboxy methylcellulose in physiological saline. 4 hours after the oral administrations, the animals were sacrificed under gas anesthesia with isoflurane and the brains, previously perfused with PBS, were removed and immediately afterwards frozen in liquid nitrogen. Subsequently, the total RNA of the brain was extracted by means of the High Pure RNA Isolation kit (Roche) and the amount and quality of the RNA were analyzed by means of spectrophotometry (Infinite 200 with NanoQuant, Tecan) and viewing of the 18S and 28S bands by means of electrophoresis. The RT-PCR was performed by means of two steps, first, the mRNA was changed to cDNA using the RNA to cDNA kit (Applied Biosystem) and the gene expression was subsequently analyzed by means of TaqMan probes using the validated probes Mn00519071_ml for seladin-1/DHCR24 and Hs99999901_s1 for 18S (used to normalize the results) in the 7500 Fast Real-Time PCR System equipment (Applied Biosystem). The relative amount of the gene expression was determined by using the ΔΔCt method with the SDS v2.1.1 software (Applied Biosystem); the expression of 18S was used to normalize the measurement.

Figure 56 shows how both simvastatin and NST0037 increased the gene expression at 4 hours of the administration thereof to the same extent. These results confirm the previous data in human neurons, in which an increase of seladin-1 expression as a neuroprotective mechanism of NST0037 was observed. Furthermore, these results confirmed the blood-brain barrier passage presented in previous examples.

In summary, the oral treatment with NST0037 in wild-type mice causes an increase of the seladin-1/DHCR24 neuroprotective gene, which furthermore confirms that the compound crosses the blood-brain barrier.

## EXAMPLE 24

**Comparative analysis of the survival rate of two compounds in zebrafish larvae**

24.1. Analysis of the survival rate of compound NST0037 in comparison with simvastatin in zebrafish larvae. Acute toxicity assay.

To evaluate the biosafety of compound NST0037, its toxicological effects on the zebrafish larvae model were analyzed by means of determining the safety parameters of the OECD C15 protocol.

The fertilized eggs were obtained by natural mating of the zebrafish (Danio rerio, AB strain). A total of 8-10 pairs were used for each cross and a total of 200-250 eggs were generated on average per pair. The eggs were collected immediately after spawning and were washed with dilution water ($CaCl_2 \cdot 2H_2O_2$ 0.29 g/L, $MgSO_4 \cdot 7H_2O_2$ 0.12 g/L, $NaHCO_3$ 0.065 g/L, KCl 0.006 g/L), the pH being adjusted to 7.8 ± 0.2, in accordance with EC Regulation 440/2008, method C.1, and were deposited in a Petri dish.

The embryos were developed normally until 96 hours post-fertilization in their growth medium. At said time, the larvae were transferred from the Petri dishes to the exposure chambers (M24 microtiter plates) by means of pipettes and exposed to the substances to be tested (NST0037 or simvastatin) with dilution water (controls) or with different concentrations of the treatments. The larvae were incubated without additional aeration, at the suitable temperature (25 ± 1°C) and under a regimen of 12 hours light/12 hours darkness. Each experiment was performed in triplicate.

The studies were conducted for 24 hours of treatment, thus performing an acute toxicity assay. The treatments conducted followed the following regimen:

➢ 10 control animals, treated with dilution water for a period of 24 hours.
➢ 30 animals treated with compound NST0037 (10 larvae in triplicate) diluted in dilution water for a period of 24 hours.
➢ 30 animals treated with the compound simvastatin (10 embryos in triplicate) diluted in dilution water for a period of 24 hours.

After the time of exposure of the substances under study, the record of the mortality of the larvae was determined. Table II shows the analysis of the survival rate of compound NST0037 in comparison with simvastatin in zebrafish larvae. The table shows the number of larvae used for the experiments, the dose in mg/Kg (of weight of the animal), the treatment time and the number of dead larvae with respect to the total.

Table II

| Group (NST0037) | Larvae | Dose (mg/Kg) | Time (Days) | Mortality |
|---|---|---|---|---|
| 1 | 10 | Vehicle[a] | 1 | 0/10 |
| 2 | 30 | 10 | 1 | 0/30 |
| 3 | 30 | 30 | 1 | 0/30 |
| 4 | 30 | 100 | 1 | 0/30 |
| 5 | 30 | 300 | 1 | 0/30 |
| 6 | 30 | 1000 | 1 | 0/30 |
| Group (simvastatin) | Larvae | Dose (mg/Kg) | Time (Days) | Mortality |
| 1 | 10 | Vehicle[a] | 1 | 0/10 |
| 2 | 30 | 10 | 1 | 0/30 |
| 3 | 30 | 30 | 1 | 0/30 |
| 4 | 30 | 100 | 1 | 0/30 |
| 5 | 30 | 300 | 1 | 0/30 |
| 6 | 30 | 1000 | 1 | 0/30 |

[a]Embryo water

The results of the study showed that both compounds had a similar toxicological profile in terms of the parameter of induction of mortality of zebrafish larvae, no death being observed at any of the doses used.

<u>24.2. Analysis of the percentage of healthy larvae after the exposure of compound NST0037 in comparison with simvastatin in larvae. Acute toxicity assay.</u>

Based on the previous results, the inventors wished to determine if the absence of mortality recorded by the compound NST0037 was associated with a percentage of healthy larvae equal to or less than that of the treatment with simvastatin. The percentage of healthy larvae was defined as the number of live larvae without symptoms of external physiopathological anomalies (morphological and/or behavioral), the latter being a parameter determining the biosafety of a substance under study and is complementary to the survival rates and to $LD_{50}$. As shown in Figure 58, differences in the percentages of healthy larvae were observed between the two treatments evaluated at the highest study dose (1000 mg/Kg), reaching $67.9 \pm 3.3$ for compound NST0037, being $53.3 \pm 8.8$ for simvastatin, which surprisingly indicates a higher biosafety of compound NST0037 in the zebrafish larva model.

<u>24.3. Analysis of the percentage of larvae with anomalous appearance (symptomatology) after the exposure of compound NST0037 in comparison with simvastatin in larvae. Acute toxicity assay.</u>

Based on the results of the previous sections, the inventors decided to investigate if the higher biosafety of compound NST0037 in comparison with simvastatin was corroborated by means of the analysis of the percentage of larvae with malformations or anomalous appearance, collecting the percentage of larvae showing bodily and/or pigmentary anomalies. The anomalies recorded in the study are described below:

- Thrombosis in the yolk sac: it is a clot inside a blood vessel, in this case of any vessel irrigating the yolk.
- Cardiac thrombosis: occurs in a cardiac vessel.
- Cardiac edema (or hydrops): accumulation of fluid in the intercellular tissue space and also in the cavities of the organism. In the case of the heart, it causes the accumulation of fluid in the pericardial sac.

The results indicate that the percentage of larvae with malformations or anomalous appearance is very similar between both compounds, nevertheless, and as shown in Figure 58, it is surprisingly observed that simvastatin induced a larger percentage of larvae with toxicological symptomatology than compound NST0037, which indicates a higher biosafety of the latter.

In summary, the treatment with NST0037 in zebrafish larvae in a wide range of concentrations does not cause any mortality, in addition to presenting a higher percentage of healthy larvae and a smaller number of larvae with malformations or anomalous appearance than simvastatin.

**EXAMPLE 25**

25.1. Study of the variation of the weight of adult zebrafish in a single-dose toxicity assay (24 hours) by the exposure in water of compound NST0037 in comparison with simvastatin

Based on the results of previous examples, the inventors decided to investigate if the higher biosafety of compound NST0037 in comparison with simvastatin was corroborated by means of the analysis of the variation of the weight of adult zebrafish, by means of a single-dose assay included in the European Medicines Agency (EMEA). This study of the EMEA is about the quality and the amount of the toxic phenomenon caused by the single administration of a substance or combination of substances, in relation to time. These studies report on information of the possible effects of overdoses occurring in humans and can be useful for designing toxicity studies in which repeated doses of administration of the treatment are required.

The experiments were conducted strictly following the Guidance on the Operation of Animals (Scientific Procedures, Act. 1986*).* The assay developed has the following study parameters:

- ✓ Duration of the treatment: 24 hours by exposure in water.
- ✓ Post-treatment time: 14 days.
- ✓ Number of animals: 7 per concentration.
- ✓ Assay vessels: fish tanks with a capacity of 9 liters, filling them a volume of 5 liters with filtering rack recirculation water.
- ✓ Animal load: mean weight of the fish, between 0.54-0.60 g.
- ✓ Assay concentration: 2000 mg/kg.
- ✓ Light: photoperiods of 12/12 hours of light/darkness.
- ✓ Temperature: 25 ± 1 °C.
- ✓ Food: the animals are not fed 24 hours before the treatment or during the 24 hours that the assay lasts. Subsequently they were fed like the rest of the animals.

Weight of the animals: they were weighed at time 0 (before the treatment), at 7 and at 14 days post-treatment.

In this case, the weight of the animals of the different study groups at three established times: before starting the treatments (0 dpt), at day 7 post-treatment and at day 14 post-treatment, was used as a toxicity parameter. Figure 59 shows the average of the weights of the animals according to the treatment and to the post-exposure time of the substances. The results indicated that while the weight of the fish without treatment increased throughout the study, the weight of those treated with simvastatin was significantly reduced. In turn, the treatment with NST0037 surprisingly did not modify the weight during the assay, which indicates that it has a better safety profile than simvastatin.

25.2. Histopathological study in adult zebrafish after a single-dose toxicity assay (24 hours) by the exposure in water of compound NST0037 in comparison with simvastatin

Based on the results of the previous studies, the inventors decided to investigate if there were differences in the histopathological study of the different treatment groups by means of staining the samples with hematoxylin-eosin, which allows distinguishing pathological marks in the organs or tissues of the animals under study. To that end, the fish were sacrificed at 14 days post-treatment (previously anesthetized) and the samples (whole fish) were included in paraffin. The hematoxylin-eosin staining was subsequently performed in order to conduct the histopathological studies, obtaining a minimum of 6 longitudinal sections per animal, for the purpose of collecting different study areas of the same organ. The following organs were studied: brain, kidney, pancreas, intestine, eye, gills, ovary, testicle, muscle and liver. Figure 60 shows representative images of the histopathological study conducted in the animals treated with the dose of 2000 mg/Kg. Of all the organs studied, the only ones having distinguishable pathological features were the intestine and the ovary. Atrophy signs in the intestinal villi were detected in the intestine, being more pronounced in the animals treated with simvastatin than in those treated with NST0037. In the ovary of the females treated with simvastatin atrophy was detected in tertiary oocytes. Furthermore, the liver of the animals treated with both compounds had discrete inflammation areas.

In summary, the treatment with NST0037 in adult zebrafish did not modify the weight thereof during a 14-day experiment, whereas with simvastatin a significant reduction of the weight of the animals was detected. Furthermore, simvastatin caused greater histopathological effects than NST0037, demonstrating that the latter compound is safer.

**EXAMPLE 26**

26.1. Lethality study by the constant exposure in water (4 days) of NST0037 in comparison with simvastatin in adult zebrafish.

Based on the results of previous examples, the inventors decided to investigate if the higher biosafety of compound NST0037 in comparison with simvastatin was corroborated by means of the analysis of the lethality by the constant exposure in water for 4 days according to the acute toxicity test for fish of the OECD (Draft Revised Guideline 203). The object of this assay is to determine the acute lethal toxicity of a substance on freshwater fish. Acute toxicity is the distinguishable adverse effect induced in an organism as a result of the exposure to a given substance for a short time period (days). In the present assay, acute toxicity is expressed as mean lethal dose ($LD_{50}$), i.e., the dose which in water causes the death of 50% of the fish of a batch subjected to assay for a continuous exposure period.

The experiments were conducted strictly following the Guidance on the Operation of Animals (Scientific Procedures, Act. 1986*)*. The assay developed has the following study parameters:

- ✓ Static method: a toxicity assay, during which there was no replacement of the assay solution, was conducted.
- ✓ A control (or check sample) is made without assay substance in addition to the actual assay concentrations.
- ✓ Duration: 4 days.
- ✓ Number of animals: 7 per concentration.
- ✓ Vessels: 9 liter tanks, filling them with a volume of 5 liters, with filtering rack recirculation water.
- ✓ Load: mean weight of the fish: 0.46 g ± 0.03, the load being 0.49 g/liter.
- ✓ Assay concentrations: 5 concentrations (1, 3.2, 10, 32 and 100 mg/kg).
- ✓ Light: photoperiods of 12/12 hours of light/darkness.
- ✓ Temperature: 25±1 °C.
- ✓ Food: none.

The deaths of the animals under study during the entire time of the assay were recorded as a toxicity parameter.

Table III shows the analysis of the survival rate of compound NST0037 in comparison with simvastatin in adult zebrafish treated for 4 days with the compounds in water. The table shows the number of animals used for the experiments, the dose in mg/Kg (of weight of the animal), the treatment time and the number of dead animals with respect to the total.

Table III

| Group (NST0037) | Animals | Dose (mg/kg) | Time (Days) | Mortality |
|---|---|---|---|---|
| 1 | 7 | Vehicle[a] | 4 | 0/7 |
| 2 | 7 | 1 | 4 | 0/7 |
| 3 | 7 | 3.2 | 4 | 0/7 |
| 4 | 7 | 10 | 4 | 0/7 |
| 5 | 7 | 32 | 4 | 0/7 |
| 6 | 7 | 100 | 4 | 1/7 |
| Group (simvastatin) | Animals | Dose (mg/kg) | Time (Days) | Mortality |
| - | - | - | - | - |
| 1 | 7 | 1 | 4 | 0/7 |
| 2 | 7 | 3.2 | 4 | 0/7 |
| 3 | 7 | 10 | 4 | 0/7 |
| 4 | 7 | 32 | 4 | 0/7 |
| 5 | 7 | 100 | 4 | 6/7 |

[a] System water.

As shown in Table III, the mortality caused by both compounds in this acute toxicity assay surprisingly demonstrate that simvastatin was significantly more toxic than compound NST0037 ($\chi^2$, p-value< 0.001), since at day 4 post-treatment, 14% (1/7) of the animals treated with NST0037 died, the percentage of mortality observed for the group of treatment with simvastatin being 86% (6/7). According to what is observed in this study, the $LD_{50}$ of compound NST0037 was greater than 100 mg/Kg after 4 days of continuous treatment with the compound. Simvastatin had an $LD_{50}$ of 60.55 mg/Kg after 4 dpt; it was again surprisingly more toxic than compound NST0037, since simvastatin reached the $LD_{50}$ with fewer doses than NST0037 and in the same time.

26.2. Histopathological study in adult zebrafish after the lethality assay by the constant exposure in water (4 days) of NST0037 in comparison with simvastatin in adult zebrafish

Based on the results of the previous studies, the inventors decided to investigate if there were differences in the histopathological study of the different treatment groups by means of staining the samples with hematoxylin-eosin, which allows distinguishing pathological marks in the organs or tissues of the animals under study. To that end, the fish that survived the treatment were sacrificed at 4 days post-treatment (previously anesthetized) and the samples (whole fish) were included in paraffin. The hematoxylin-eosin staining was subsequently performed in order to conduct the histopathological studies, obtaining a minimum of 6 longitudinal sections per animal, for the purpose of collecting different study areas of the same organ. The following organs were studied: brain, kidney, pancreas, intestine, eye, gills, ovary, testicle, muscle and liver.

Figure 61 shows representative images of the histopathological study conducted in the animals treated. It shows representative images of the histopathological study conducted. The only doses which induced toxicological problems were those of 32 and 100 mg/Kg, the pathological alterations being restricted to the ovaries. While the control females showed normal ovarian follicles in different maturation stages, the females treated with 100 mg/Kg of both compounds presented ovarian damage, a massive degeneration of the secondary and tertiary oocytes being observed, in addition to stromal alteration. In addition, the group of females treated with 30 mg/kg of simvastatin also presented histopathological damage in the ovary, it surprisingly not being observed in the group of females treated with the same dose of compound NST0037, which indicates that compound NST0037 is safer than simvastatin in this experimental model.

In summary, the treatment with NST0037 in adult zebrafish for 4 days at 100 mg/Kg presented a residual mortality, which was extended in the same conditions with simvastatin. Furthermore, the histopathological damage found was restricted to the ovaries of the animals and were detected at the doses of 32 and 100 mg/Kg for simvastatin, whereas with NST0037 the ovarian damage was only detected at the highest dose, which demonstrates that the latter compound is safer.

## Claims

1. A compound of formula (I)

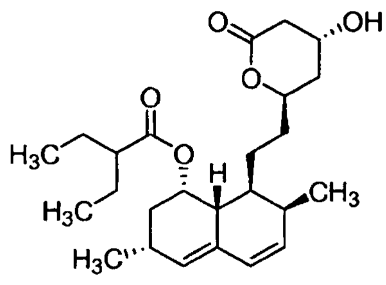

(I)

its hydroxy acid form, the pharmaceutically acceptable salts of said hydroxy acid and pharmaceutically acceptable solvates of the compound and of its hydroxy acid form.

2. A pharmaceutical composition comprising a compound of formula (I) according to claim 1 and/or its hydroxy acid form and/or a pharmaceutically acceptable salt of said hydroxy acid and/or a pharmaceutically acceptable solvate of the compound or of its hydroxy acid form, and at least one pharmaceutically acceptable adjuvant, carrier and/or vehicle.

3. A compound of formula (I) according to claim 1, its hydroxy acid form or a pharmaceutically acceptable salt of said

hydroxy acid and/or a pharmaceutically acceptable solvate of the compound or of its hydroxy acid form for its use as a medicament.

**4.** A compound of formula (I) according to claim 1, its hydroxy acid form or a pharmaceutically acceptable salt of said hydroxy acid and/or a pharmaceutically acceptable solvate of the compound or of its hydroxy acid form for its use in the prevention and/or the treatment of:

- neurodegenerative diseases,
- cognitive deterioration,
- age-associated pathological processes that relates to any age-related event or combination of events causing loss of cell viability of the nervous tissue or cell sensitization of the nervous tissue, loss of cell function and/or loss of the number of cells; and progeria, or
- epilepsy, epileptic seizures and convulsions.

**5.** A compound of formula (I), its hydroxy acid form or a pharmaceutically acceptable salt of said hydroxy acid and/or a pharmaceutically acceptable solvate of the compound or of its hydroxy acid form according to claim 4, wherein the neurodegenerative diseases are: Alzheimer' s disease, Huntington's disease, Parkinson's disease, amyotrophic lateral sclerosis (ALS) or multiple sclerosis.

**6.** Use of a compound of formula (I) according to claim 1, of its hydroxy acid form, of a pharmaceutically acceptable salt of said hydroxy acid and/or of a pharmaceutically acceptable solvate of the compound or of its hydroxy acid form in the manufacture of a medicament for the prevention and/or the treatment of:

- neurodegenerative diseases,
- cognitive deterioration,
- age-associated pathological processes that relates to any age-related event or combination of events causing loss of cell viability of the nervous tissue or cell sensitization of the nervous tissue, loss of cell function and/or loss of the number of cells; and progeria, or
- epilepsy, epileptic seizures and convulsions.

## Patentansprüche

**1.** Verbindung der Formel (I)

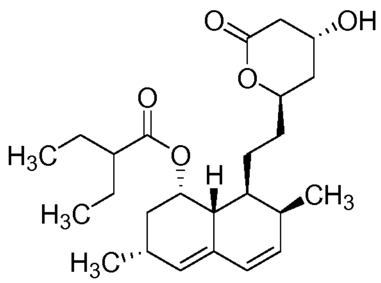

(I)

ihre Hydroxysäureform, die pharmazeutisch zulässigen Salze der Hydroxysäure und pharmazeutisch zulässige Solvate der Verbindung und ihrer Hydroxysäureform.

**2.** Pharmazeutische Zusammensetzung, welche eine Verbindung der Formel (I) nach Anspruch 1 und/oder ihre Hydroxysäureform und /oder ein pharmazeutisch zulässiges Salz der Hydroxysäure und/oder ein pharmazeutisch zulässiges Solvat der Verbindung oder ihrer Hydroxysäureform und mindestens ein pharmazeutisch zulässiges Hilfsmittel, Trägermittel und/oder Bindemittel umfasst.

**3.** Verbindung der Formel (I) nach Anspruch 1, ihre Hydroxysäureform oder ein pharmazeutisch zulässiges Salz der Hydroxysäure und/oder ein pharmazeutisch zulässiges Solvat der Verbindung oder ihrer Hydroxysäureform für deren Verwendung als Medikament.

4. Verbindung der Formel (I) nach Anspruch 1, ihre Hydroxysäureform oder ein pharmazeutisch zulässiges Salz der Hydroxysäure und/oder ein pharmazeutisch zulässiges Solvat der Verbindung oder ihrer Hydroxysäureform zu deren Verwendung bei der Verhinderung und/oder der Behandlung von:

- neurodegenerativen Erkrankungen,
- kognitivem Abbau,
- altersbedingten pathologischen Prozessen, die einen altersbedingten Vorfall oder eine Kombination von Vorfällen betreffen, die Verlust der Lebensfähigkeit von Zellen des Nervengewebes oder Zellensensibilisierung des Nervengewebes, Verlust der Zellenfunktion und/oder Verlust der Anzahl von Zellen hervorrufen; und Progerie oder
- Epilepsie, epileptischen Anfällen und Krampfanfällen.

5. Verbindung der Formel (I), ihre Hydroxysäureform oder ein pharmazeutisch zulässiges Salz der Hydroxysäure und/oder ein pharmazeutisch zulässiges Solvat der Verbindung oder ihrer Hydroxysäureform nach Anspruch 4, wobei die neurodegenerativen Erkrankungen sind: Alzheimersche Krankheit, Chorea Huntington, amyotrophe Lateralsklerose (ALS) oder Multiple Sklerose.

6. Verwendung einer Verbindung der Formel (I) nach Anspruch 1, ihrer Hydroxysäureform, eines pharmazeutisch zulässigen Salzes der Hydroxysäure und/oder eines pharmazeutisch zulässigen Solvats der Verbindung oder ihrer Hydroxysäureform bei der Herstellung eines Medikaments zur Verhinderung und/oder Behandlung von:

- neurodegenerativen Erkrankungen,
- kognitivem Abbau,
- altersbedingten pathologischen Prozessen, die einen altersbedingten Vorfall oder eine Kombination von Vorfällen betreffen, die Verlust der Lebensfähigkeit von Zellen des Nervengewebes oder Zellensensibilisierung des Nervengewebes, Verlust der Zellenfunktion und/oder Verlust der Anzahl von Zellen hervorrufen; und Progerie oder
- Epilepsie, epileptischen Anfällen und Krampfanfällen.

**Revendications**

1. Composé de formule (I)

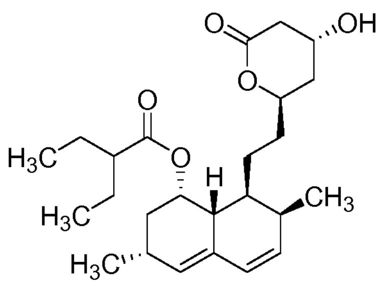

(I)

sa forme hydroxyacide, les sels pharmaceutiquement acceptables dudit hydroxyacide et les solvates pharmaceutiquement acceptables du composé et de sa forme hydroxyacide.

2. Composition pharmaceutique comprenant un composé de formule (I) selon la revendication 1 et/ou sa forme hydroxyacide et/ou un sel pharmaceutiquement acceptable dudit hydroxyacide et/ou un solvate pharmaceutiquement acceptable du composé ou de sa forme hydroxyacide, et au moins un adjuvant, un support et/ou un véhicule pharmaceutiquement acceptable.

3. Composé de la formule (I) selon la revendication 1, sa forme hydroxyacide ou un sel pharmaceutiquement acceptable dudit hydroxyacide et/ou un solvate pharmaceutiquement acceptable du composé ou de sa forme hydroxyacide pour son utilisation en tant que médicament.

**4.** Composé de formule (I) selon la revendication 1, sa forme hydroxyacide ou un sel pharmaceutiquement acceptable dudit hydroxyacide et/ou un solvate pharmaceutiquement acceptable du composé ou de sa forme hydroxyacide pour son utilisation dans la prévention et/ou le traitement de :

- maladies neurodégénératives,
- détérioration cognitive,
- processus pathologiques liés à l'âge qui concernent n'importe quel évènement lié à l'âge ou une combinaison d'événements provoquant une perte de la viabilité des cellules du tissu nerveux ou une sensibilisation des cellules du tissu nerveux, une perte d'une fonction cellulaire et/ou une perte du nombre de cellules ; et la progéria, ou
- épilepsie, crises et convulsions épileptiques.

**5.** Composé de formule (I), sa forme hydroxyacide ou un sel pharmaceutiquement acceptable dudit hydroxyacide et/ou un solvate pharmaceutiquement acceptable du composé ou de sa forme hydroxyacide selon la revendication 4, dans lequel les maladies neurodégénératives sont : la maladie d'Alzheimer, la maladie de Huntington, la maladie de Parkinson, la sclérose latérale amyotrophique (SLA) ou la sclérose en plaques.

**6.** Utilisation d'un composé de formule (I) selon la revendication 1, de sa forme hydroxyacide, d'un sel pharmaceutiquement acceptable dudit hydroxyacide et/ou d'un solvate pharmaceutiquement acceptable du composé ou de sa forme hydroxyacide dans la fabrication d'un médicament destiné à la prévention et/ou au traitement de :

- maladies neurodégénératives,
- détérioration cognitive,
- processus pathologiques liés à l'âge qui concernent n'importe quel évènement lié à l'âge ou une combinaison d'événements provoquant une perte de la viabilité des cellules du tissu nerveux ou une sensibilisation des cellules du tissu nerveux, une perte d'une fonction cellulaire et/ou une perte du nombre de cellules ; et la progéria, ou
- épilepsie, crises et convulsions épileptiques.

Figure 1

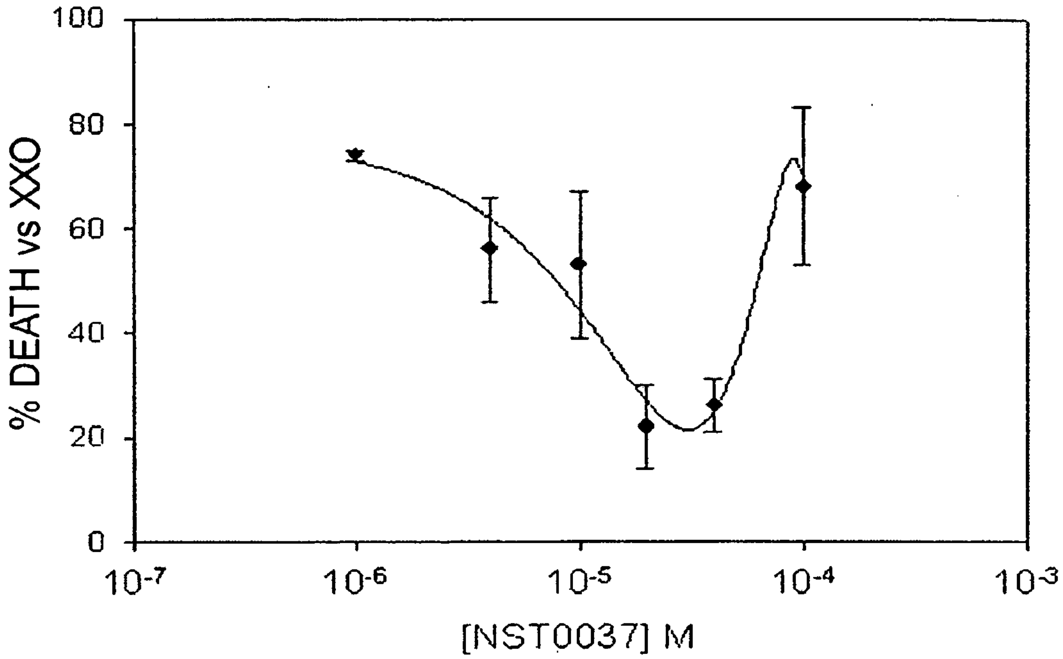


Figure 2

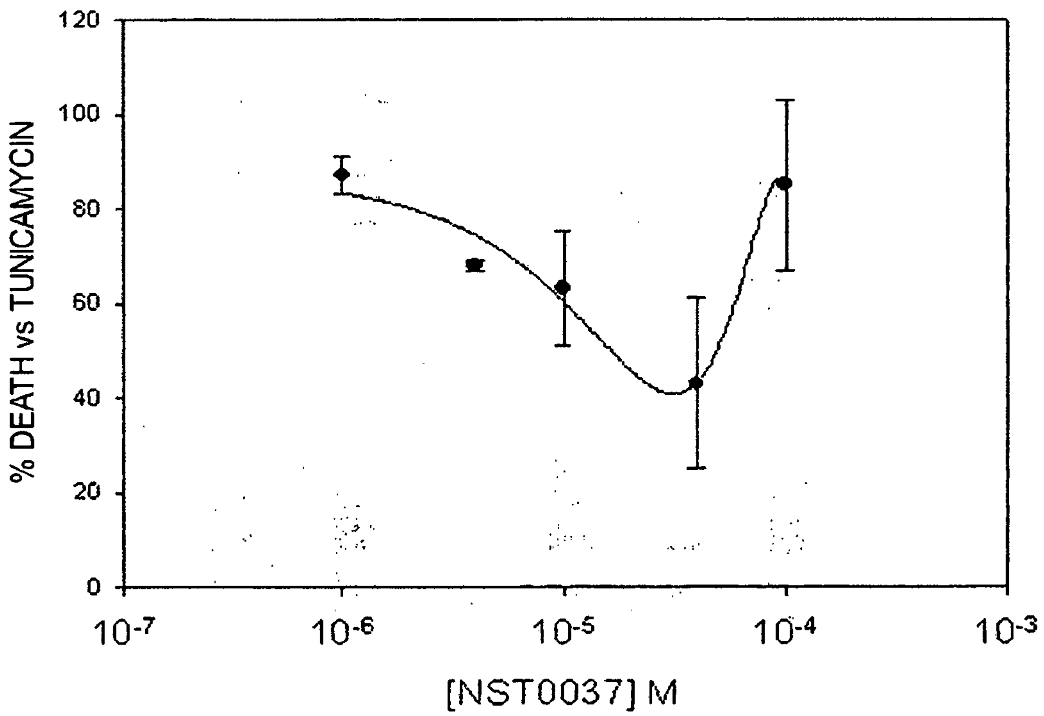

Figure 3

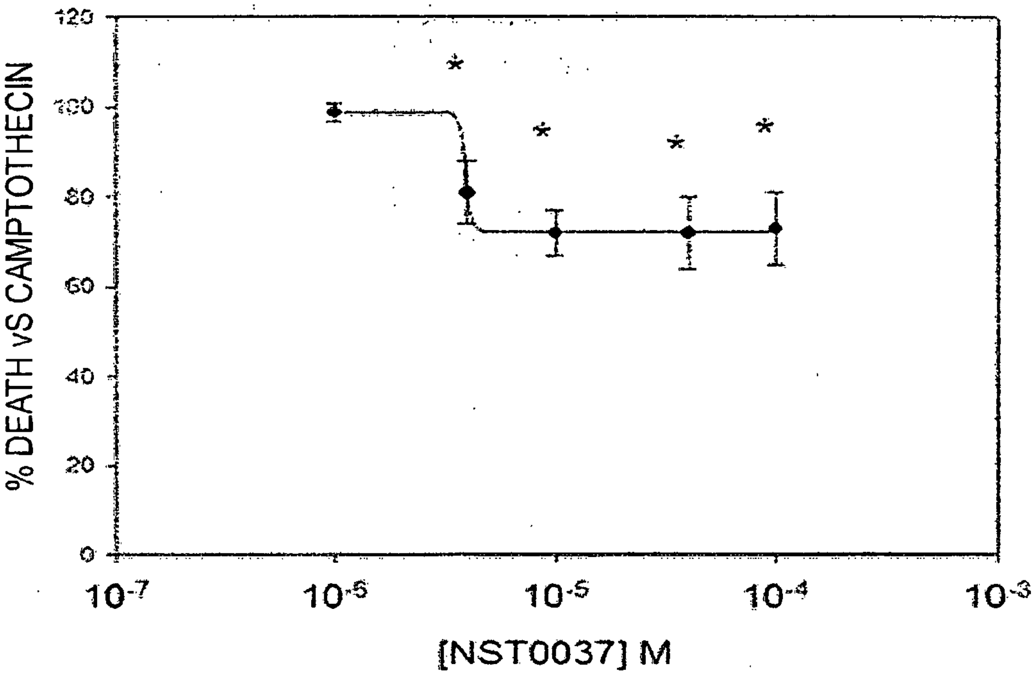


Figure 4

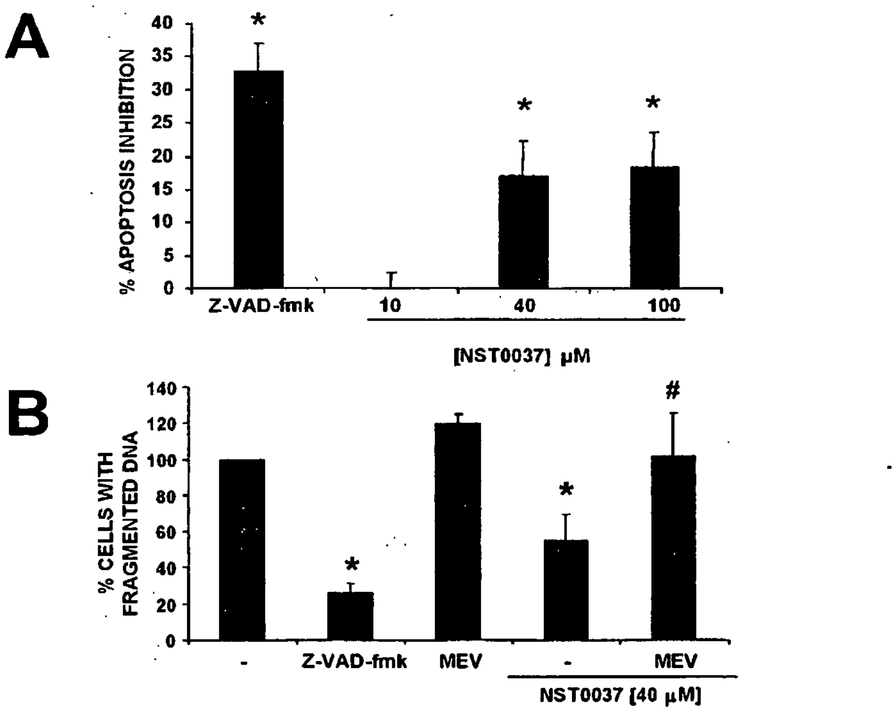

Figure 5

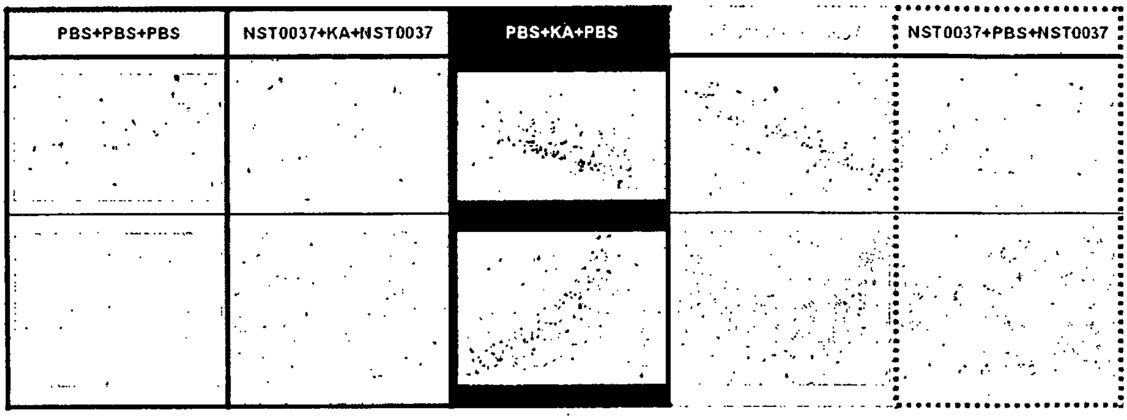


Figure 6

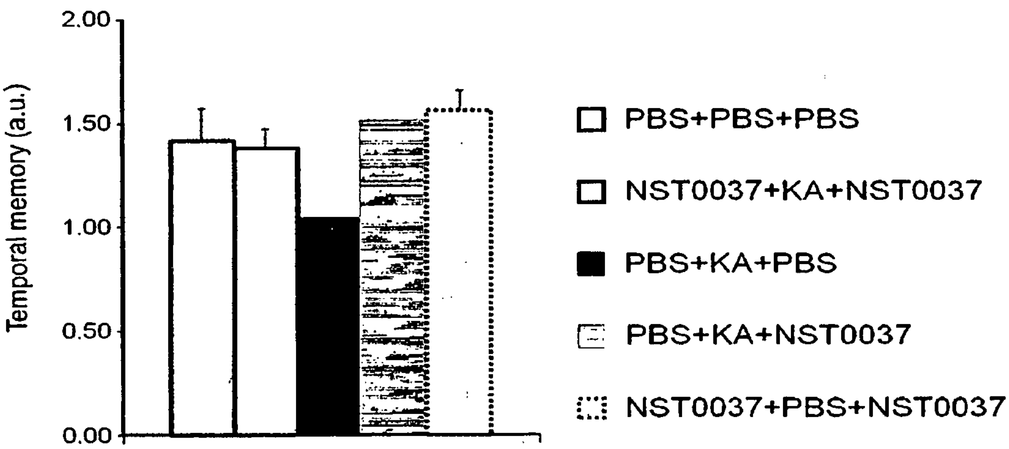


Figure 7

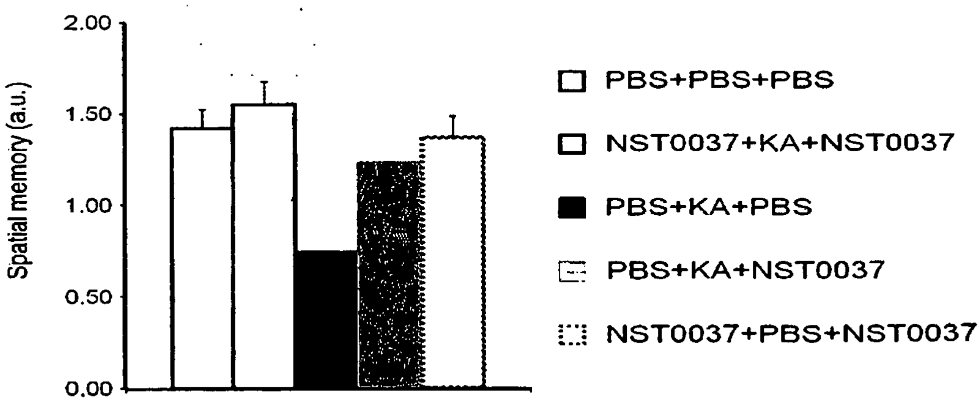

Figure 8

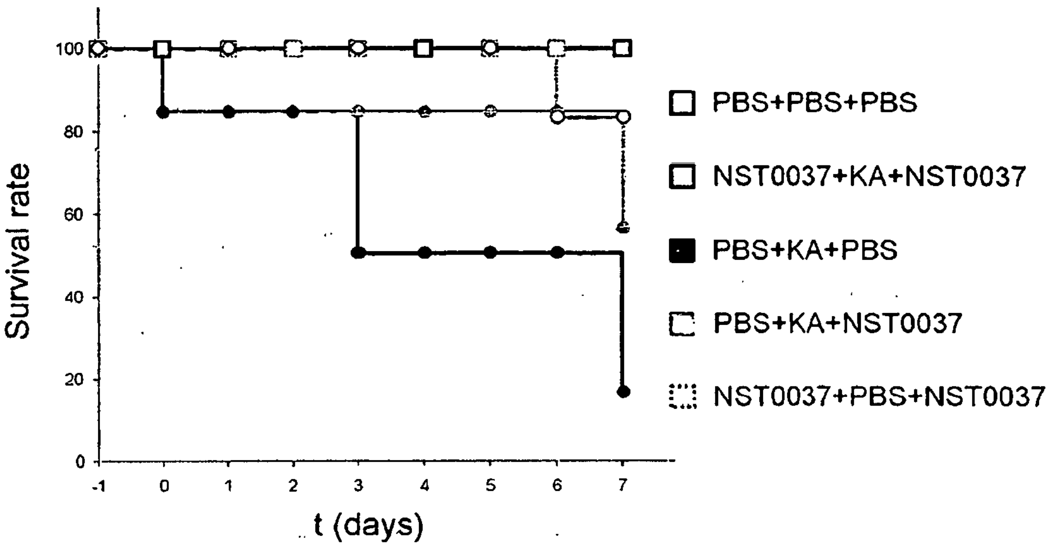


Figure 9

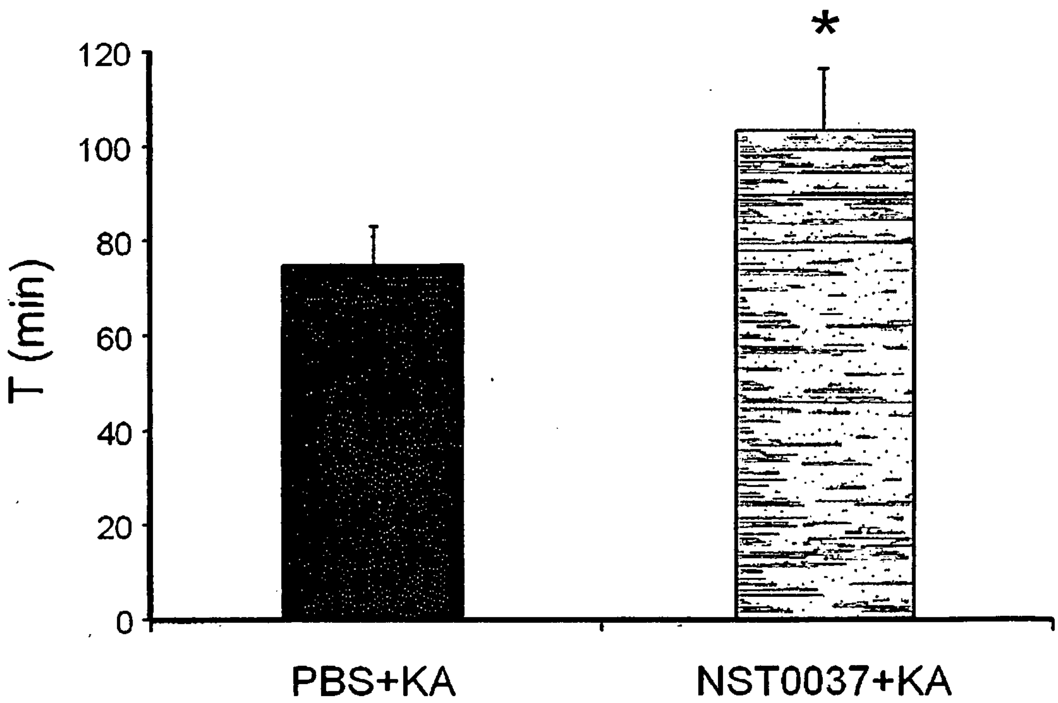

Figure 10

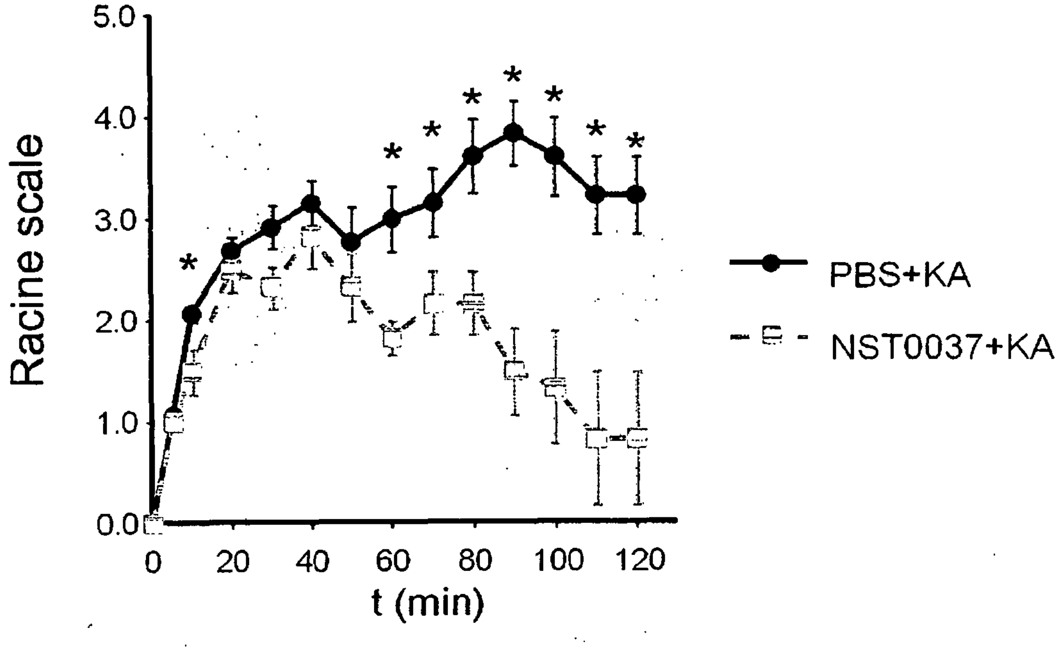


Figure 11

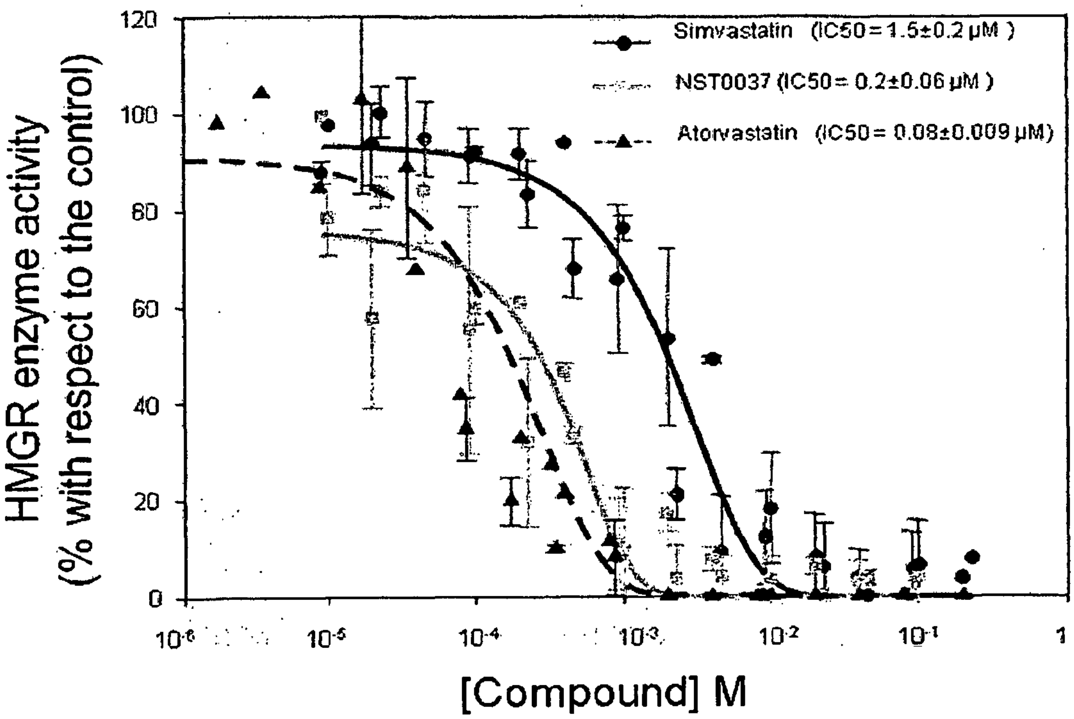

Figure 12

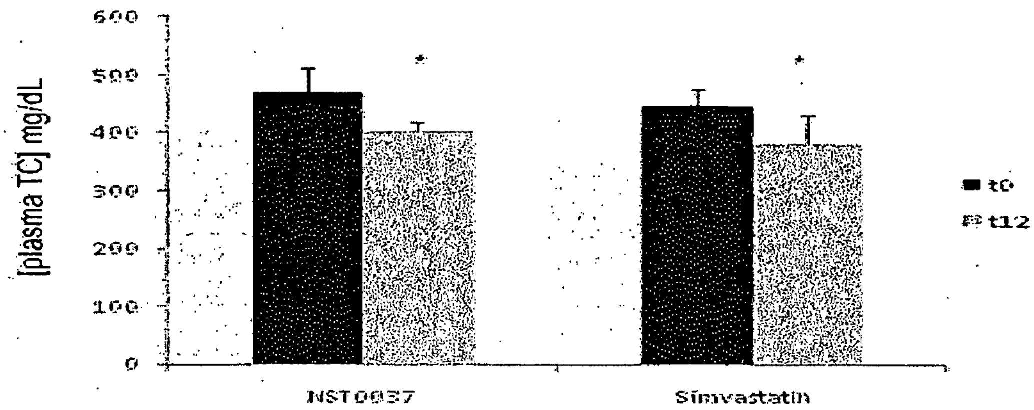


Figure 13

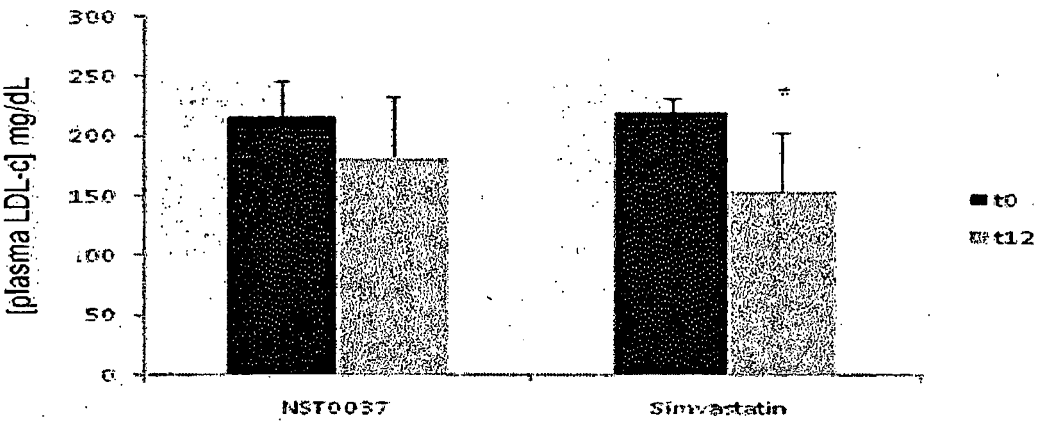


Figure 14

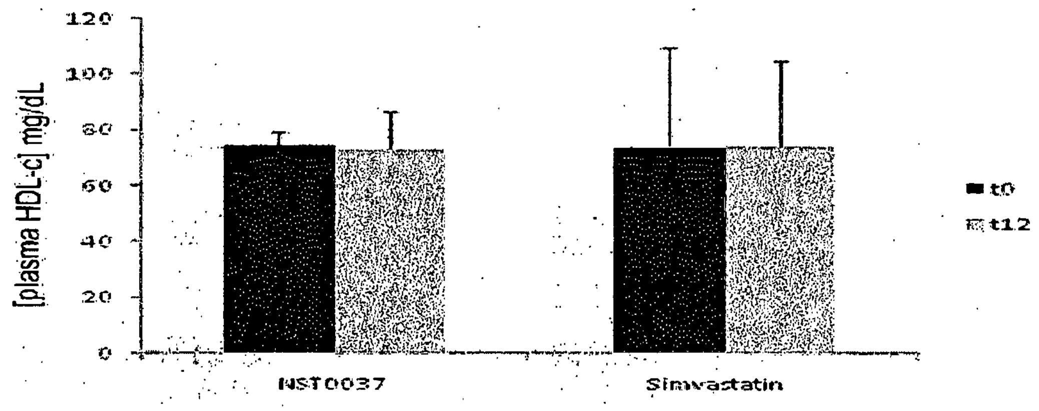

Figure 15

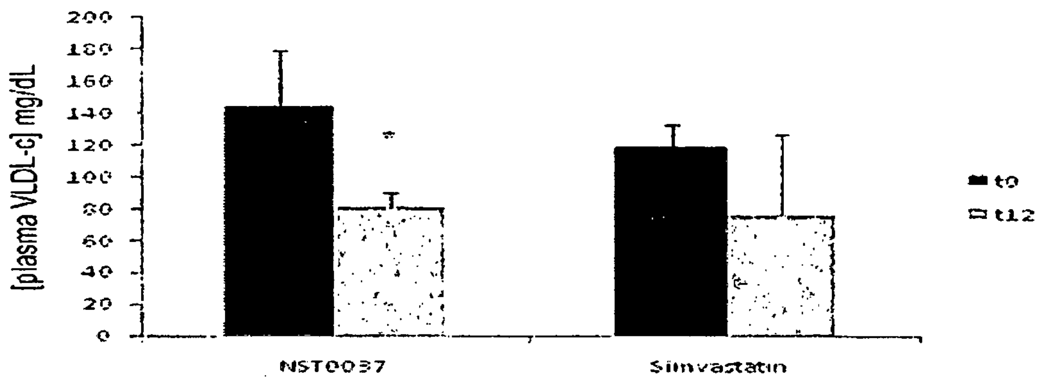


Figure 16

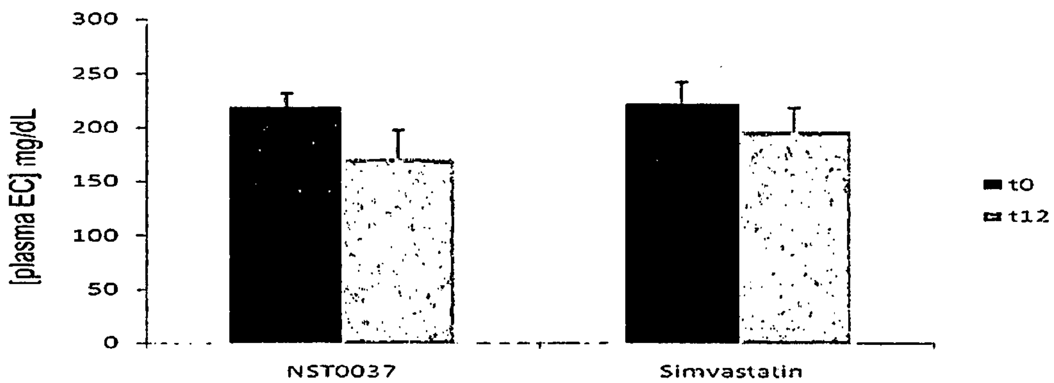


Figure 17

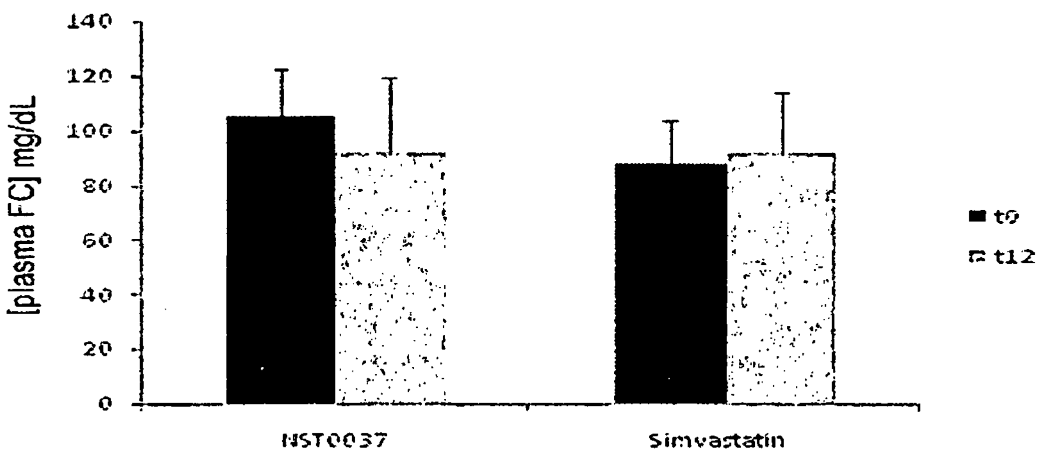

Figure 18

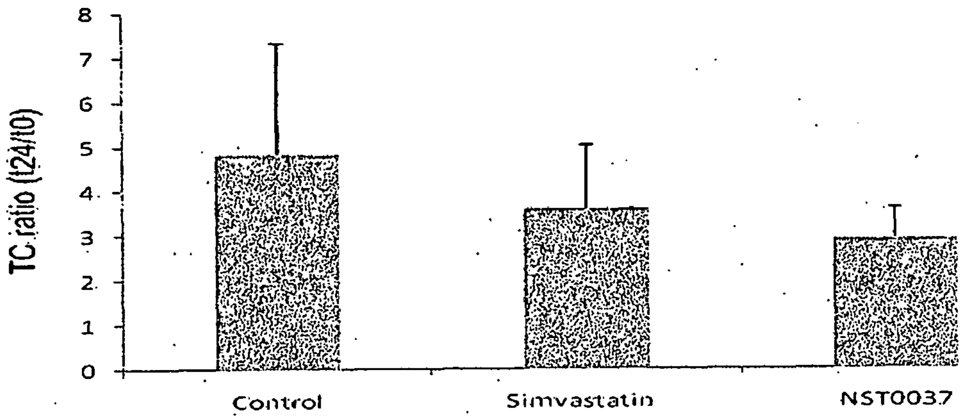


Figure 19

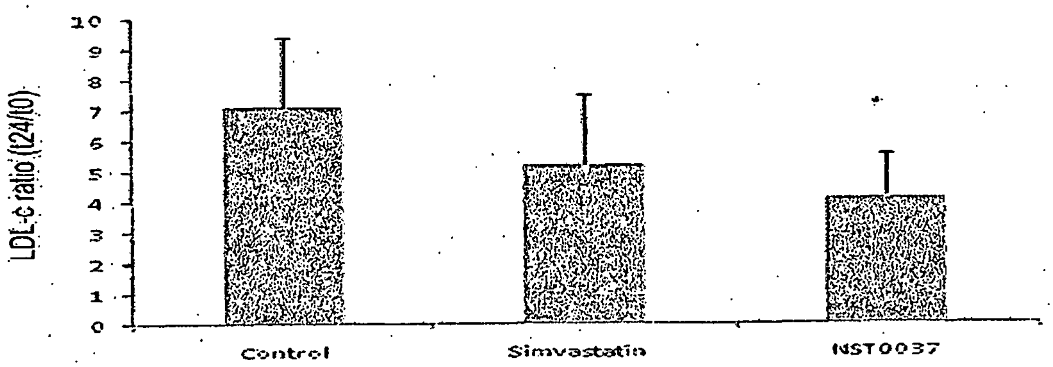


Figure 20

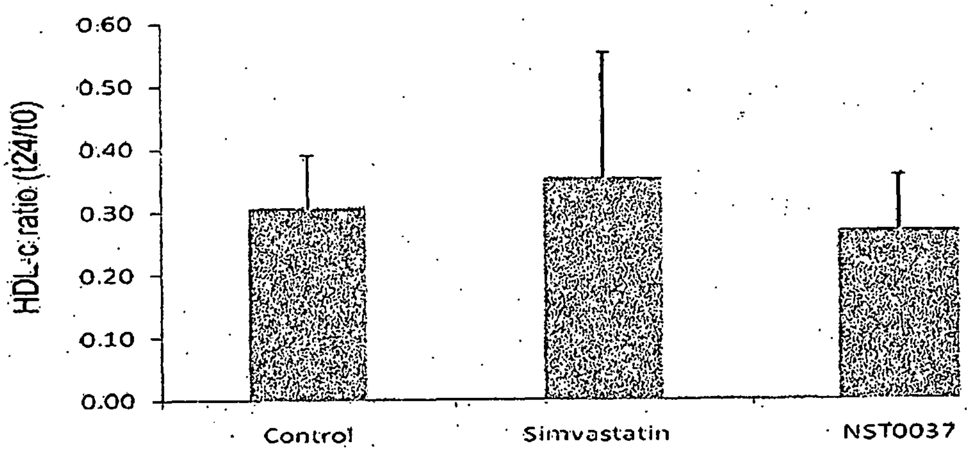

Figure 21

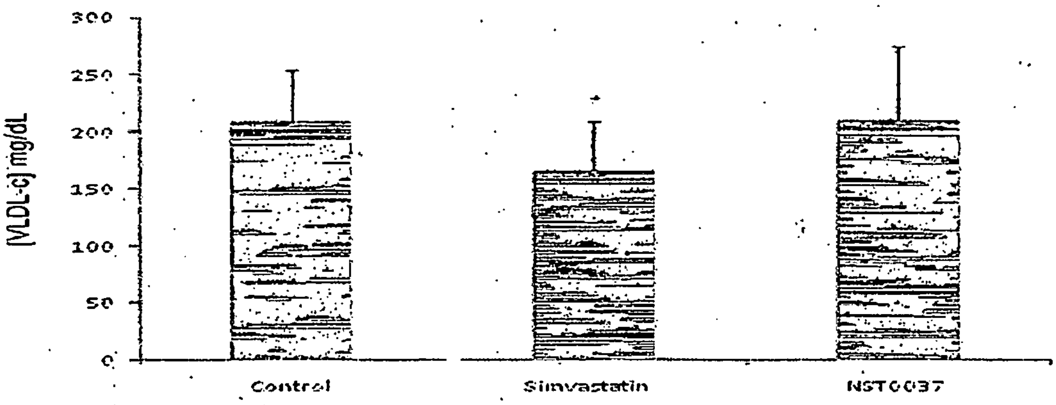


Figure 22

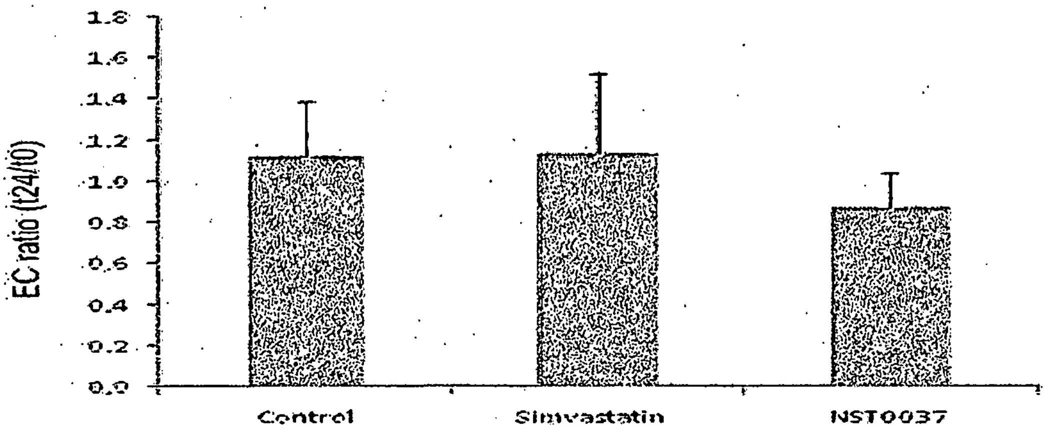


Figure 23

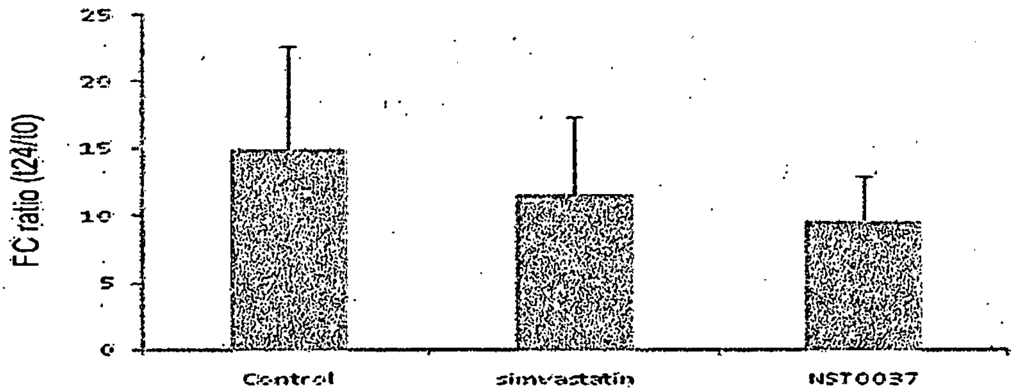

Figure 24

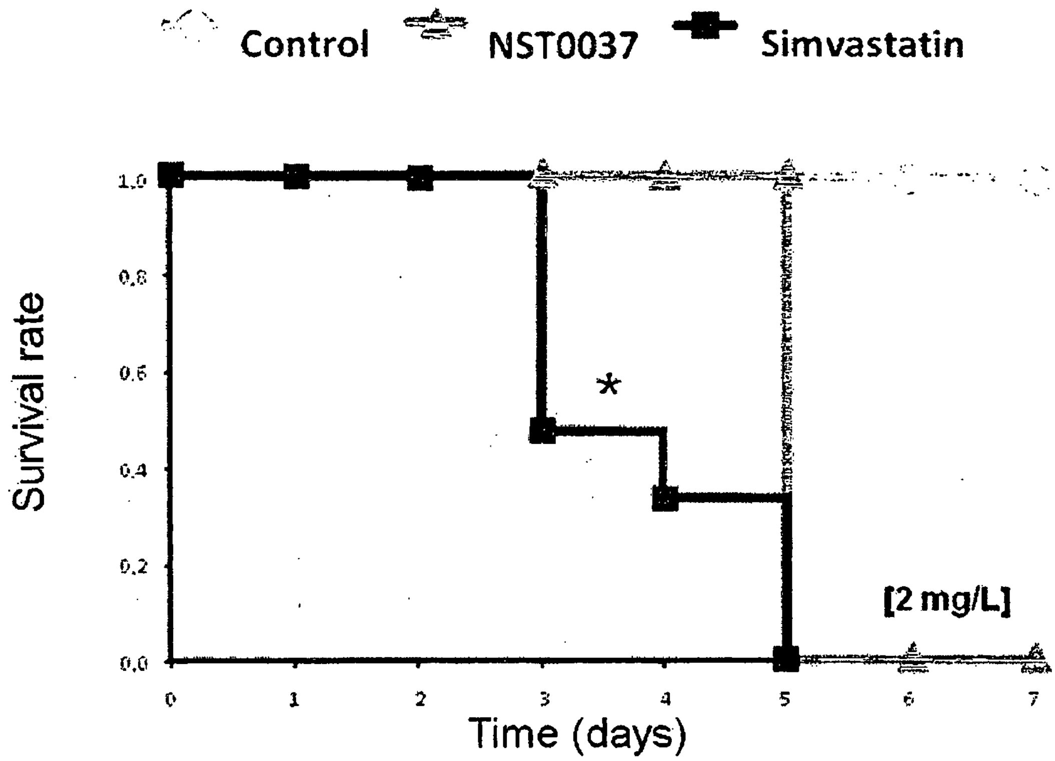


Figure 25

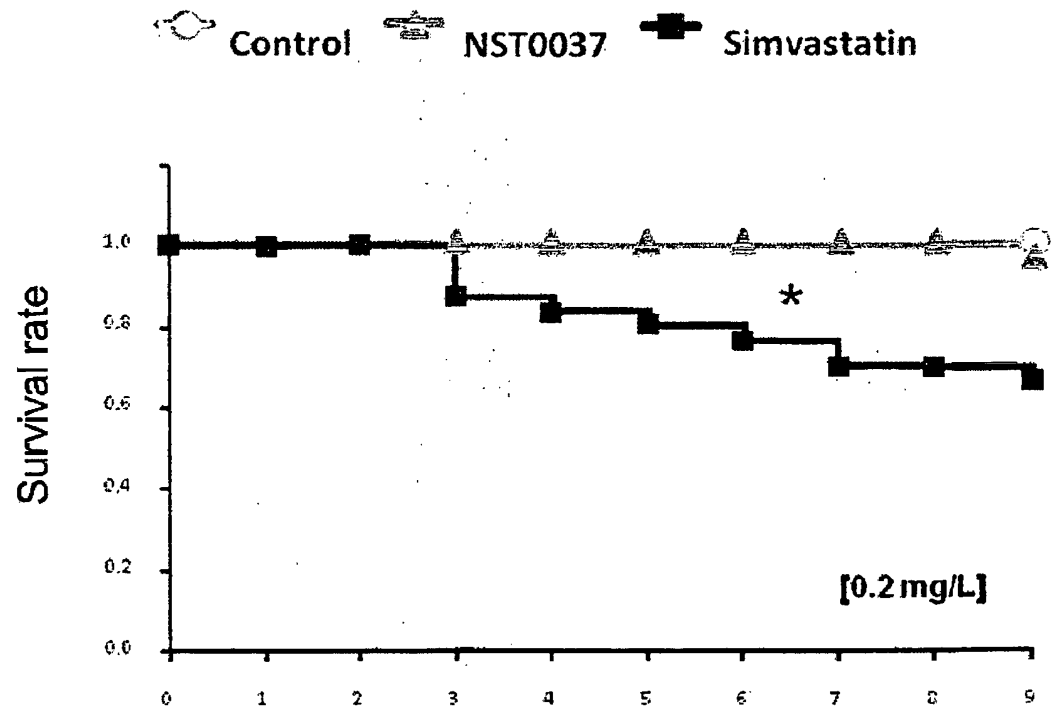

Figure 26

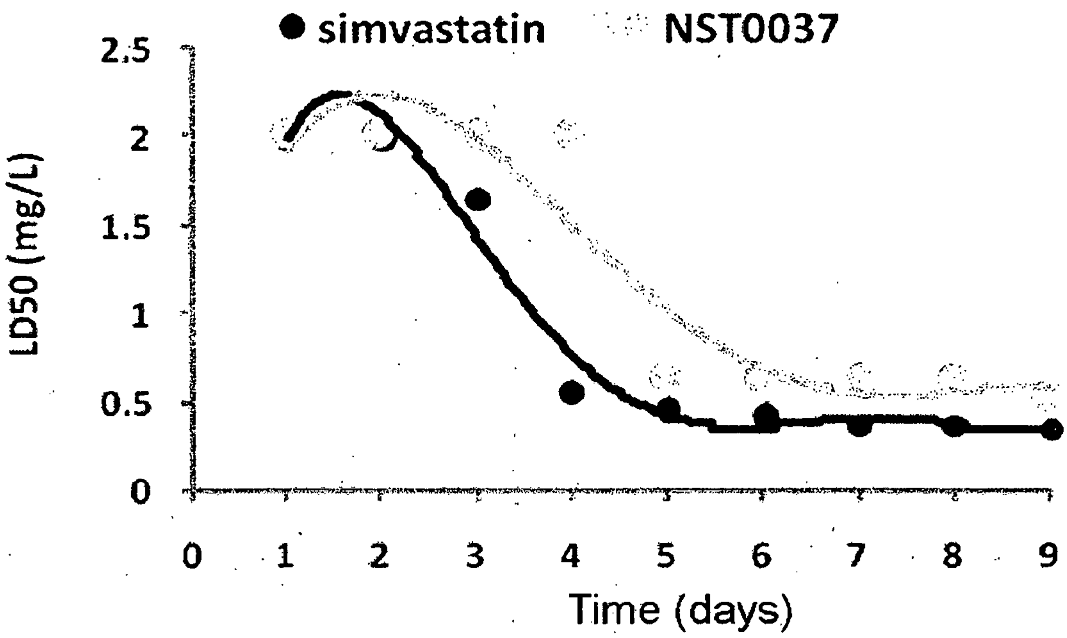


Figure 27

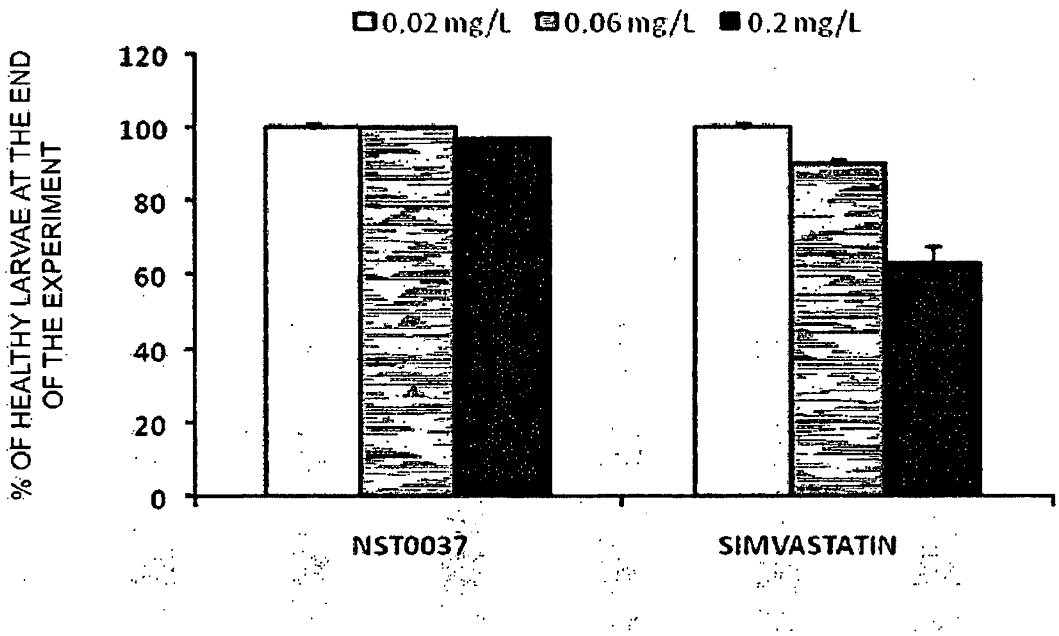

Figure 28

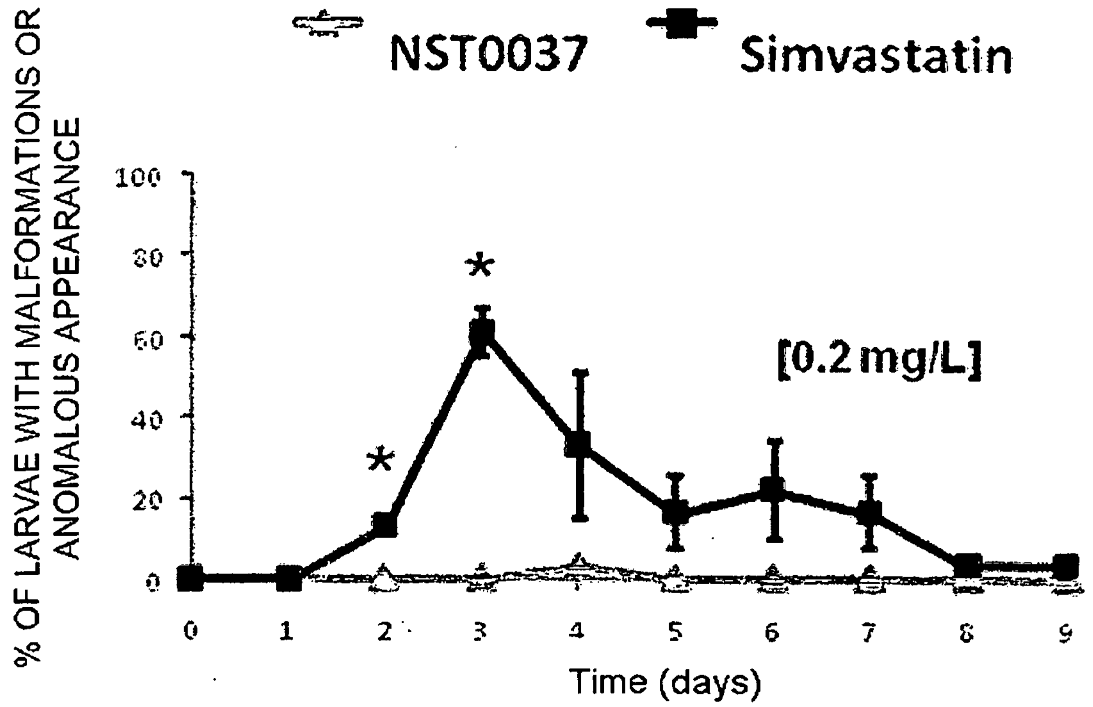


Figure 29

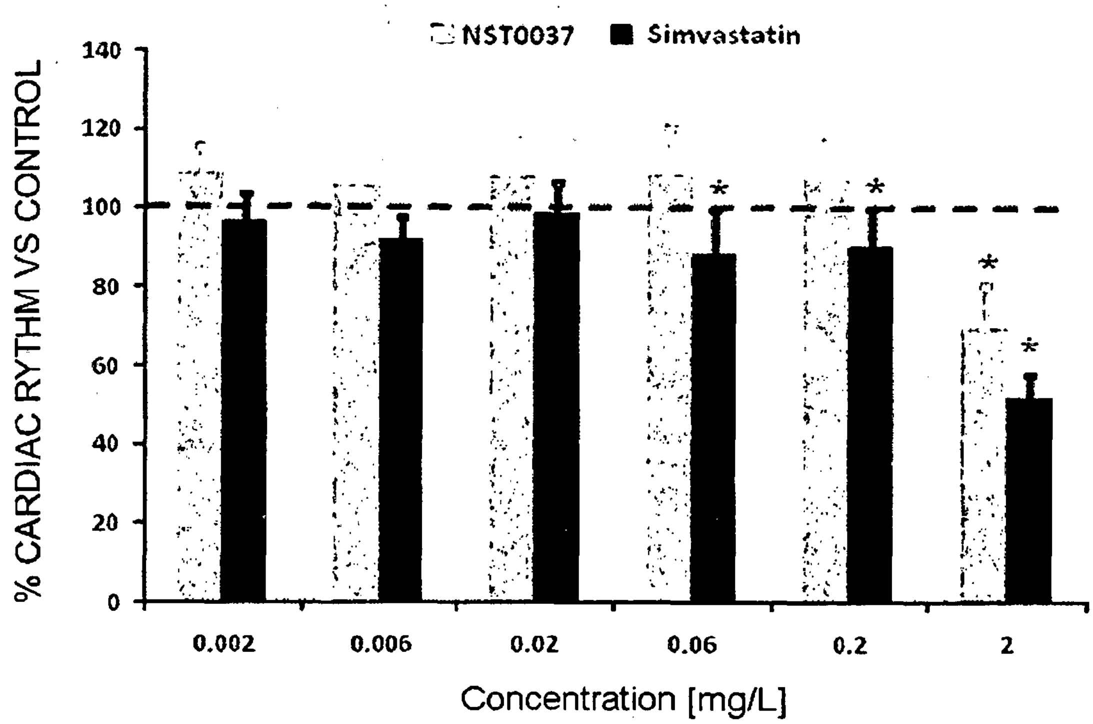

Figure 30

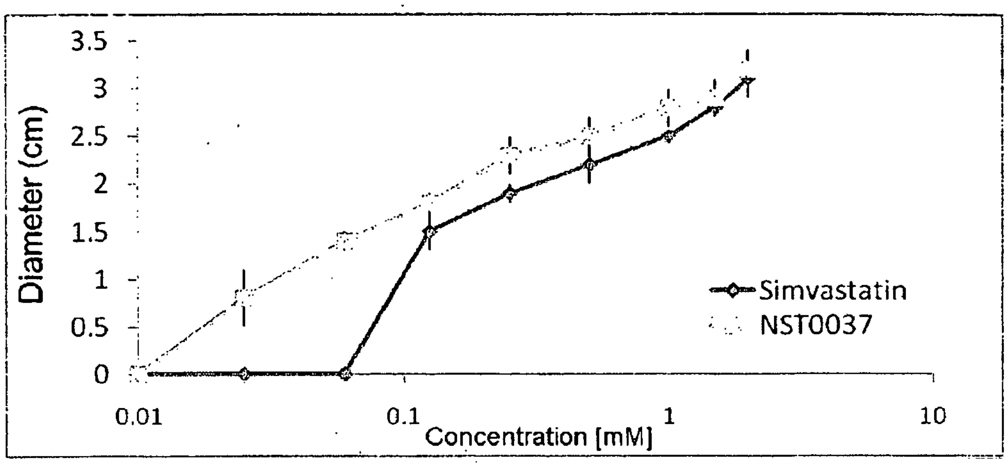


Figure 31

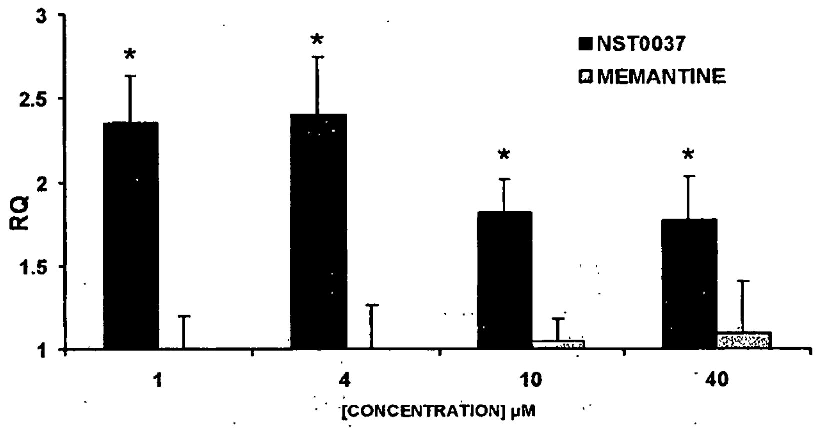


Figure 32

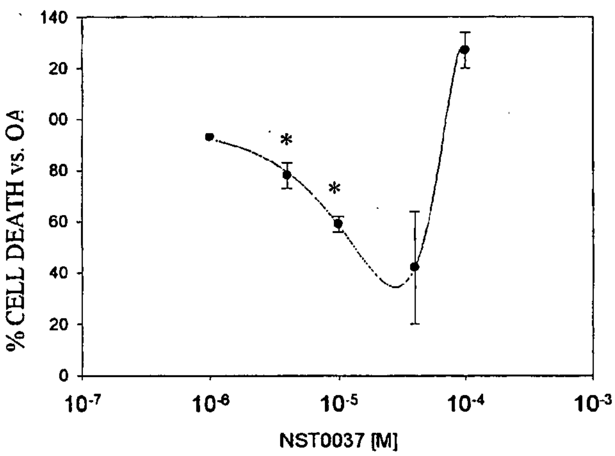

Figure 33

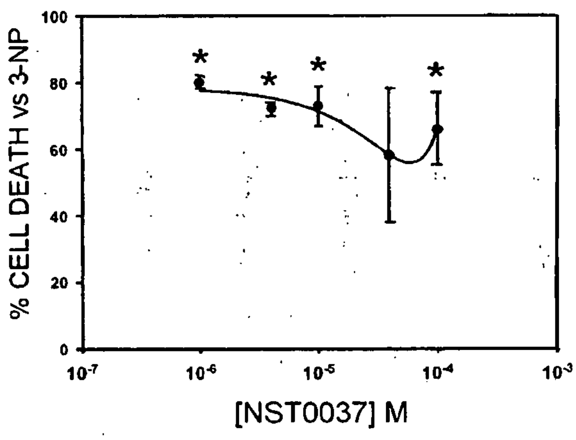


Figure 34

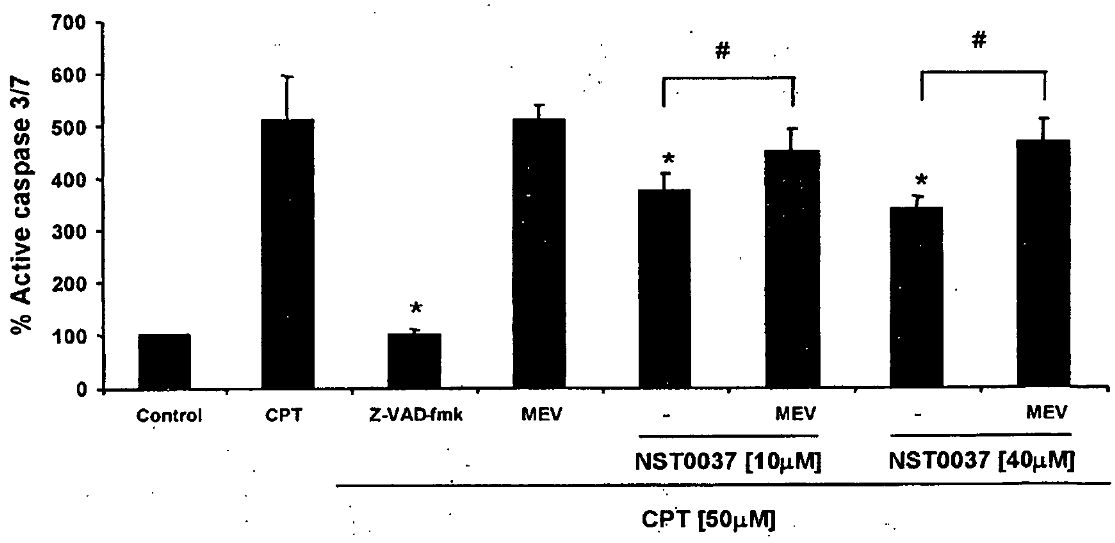

Figure 35

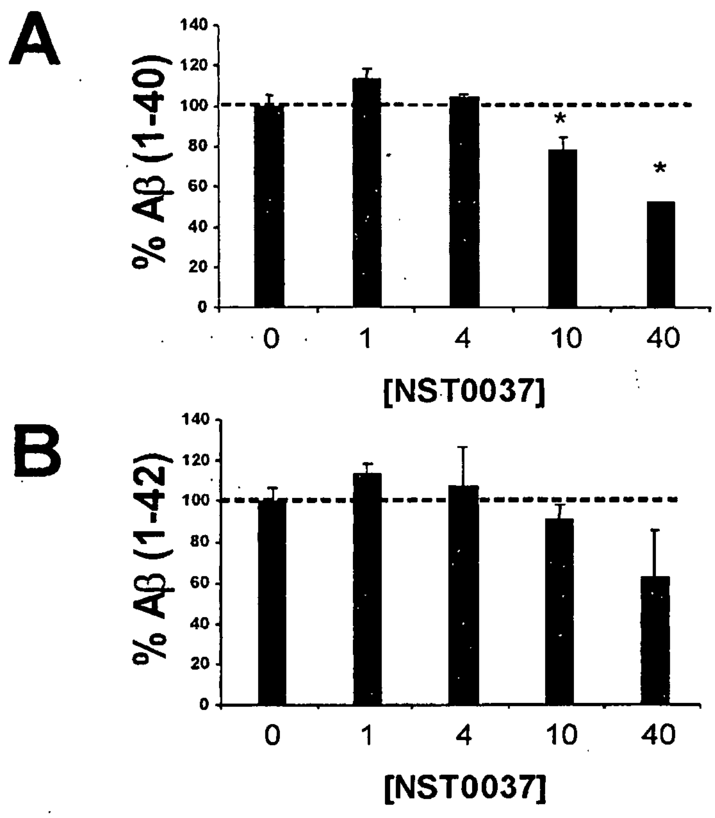


Figure 36

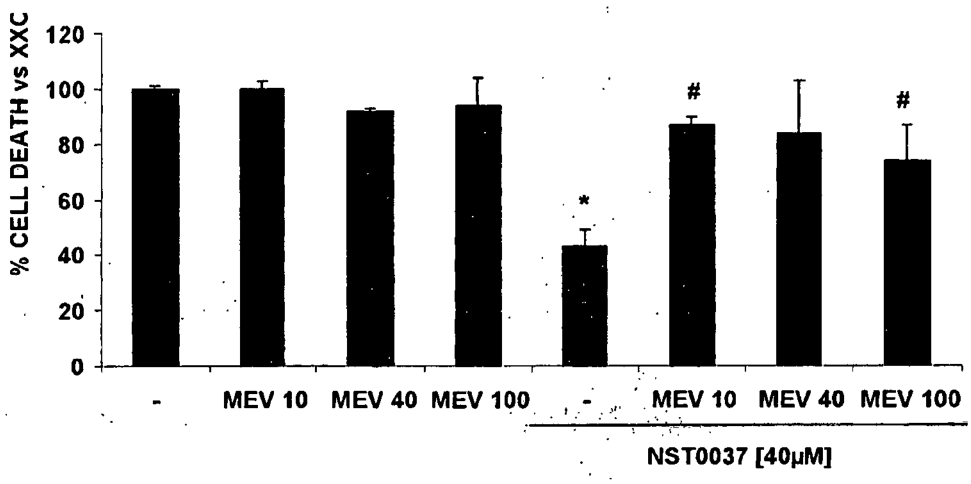

Figure 37

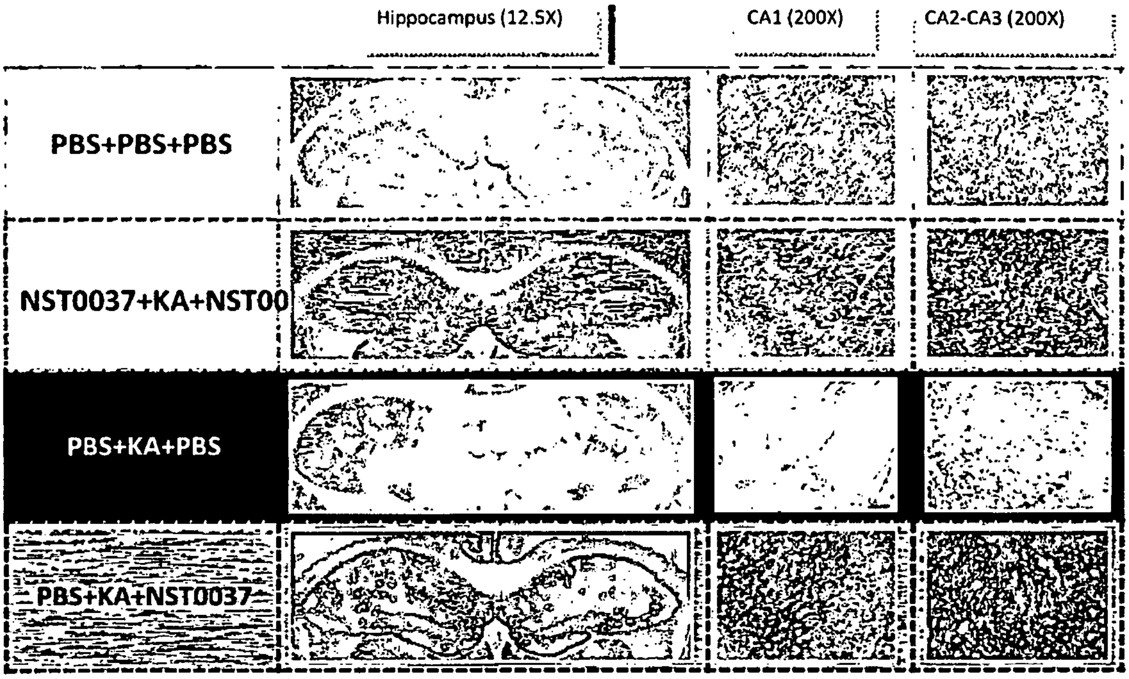


Figure 38

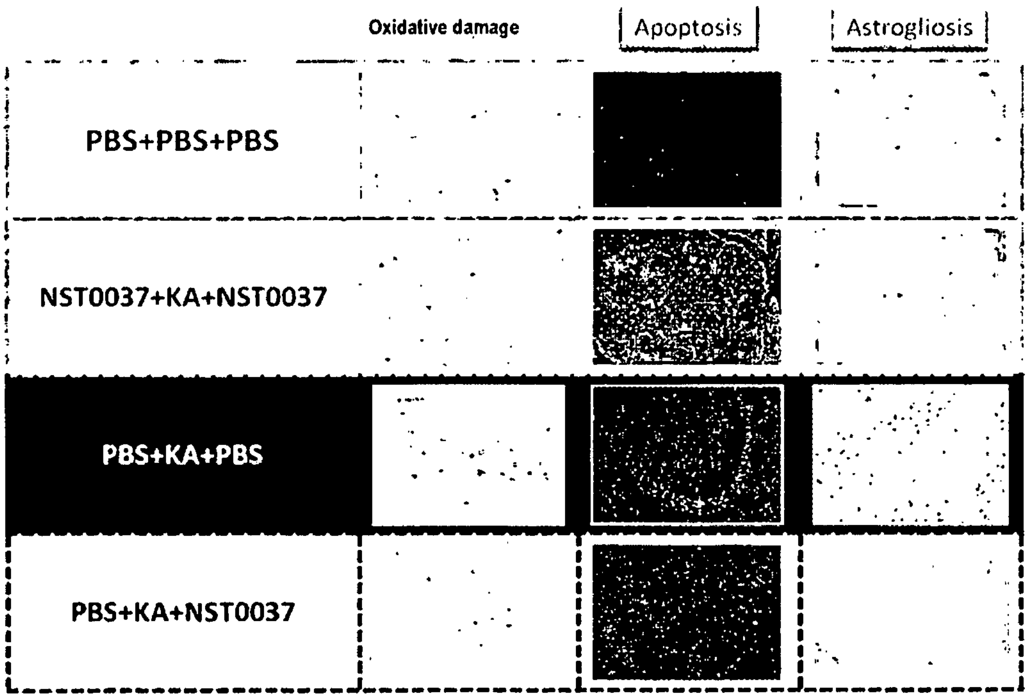

Figure 39

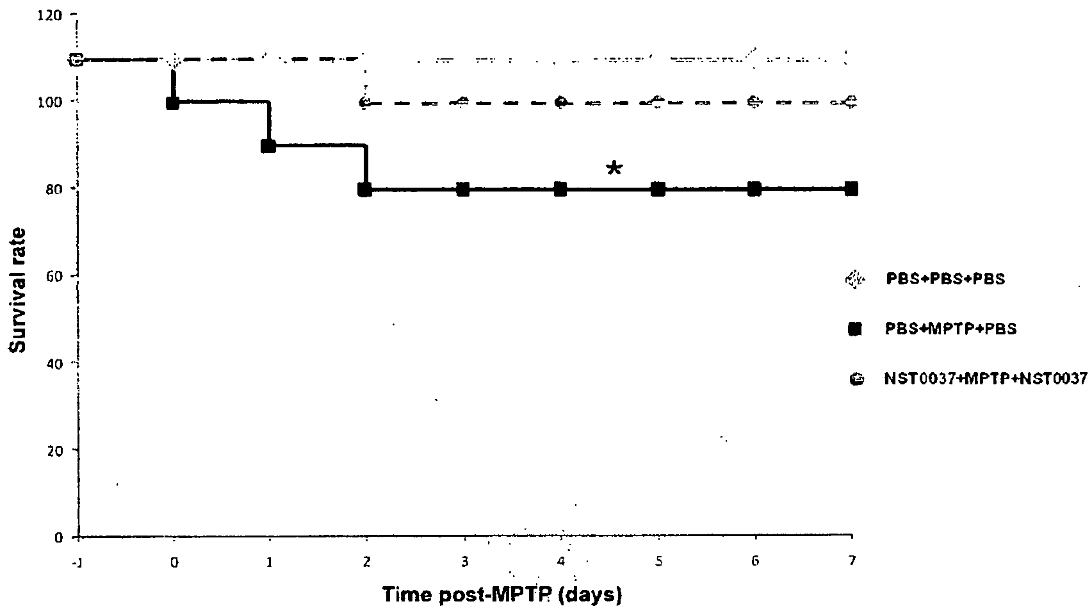


Figure 40

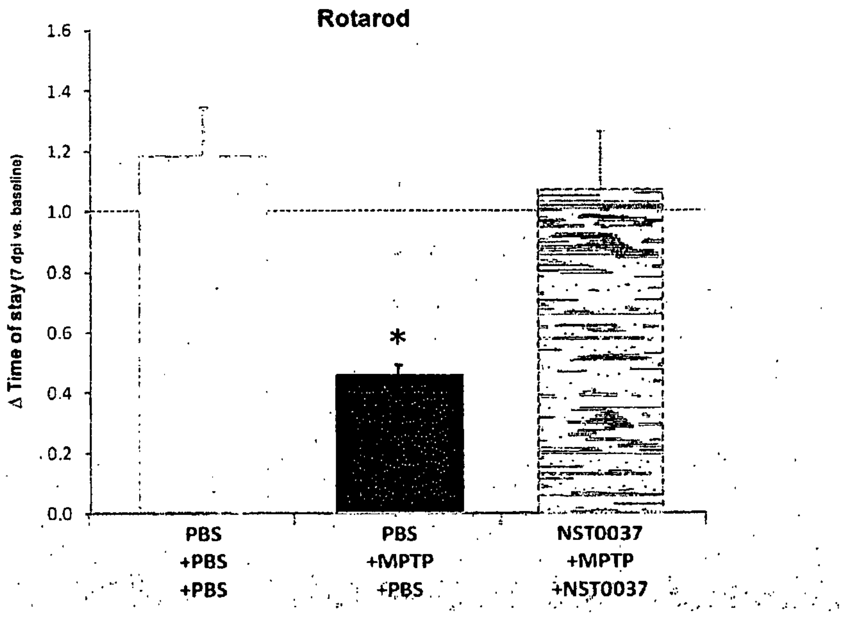

Figure 41

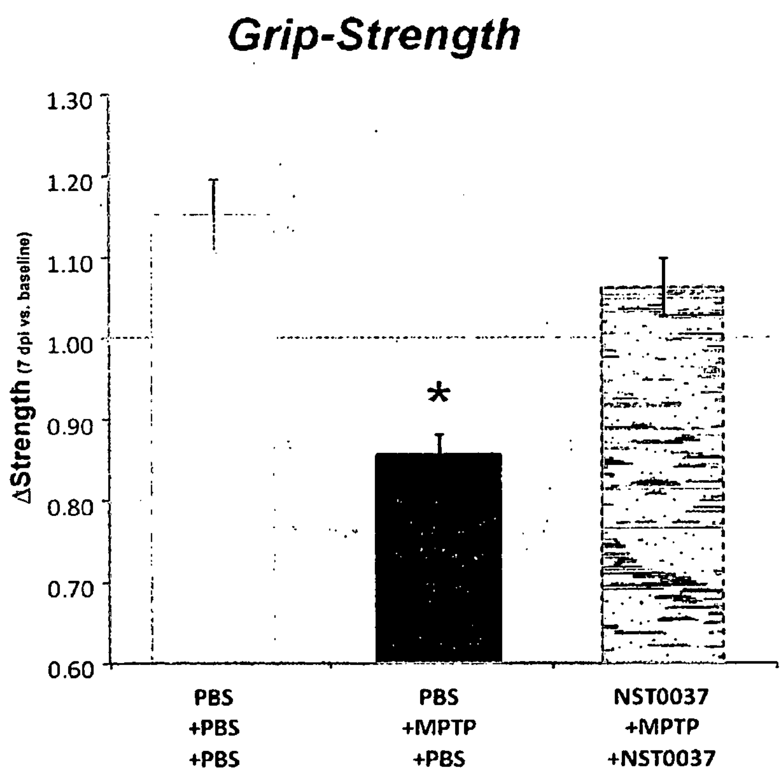


Figure 42

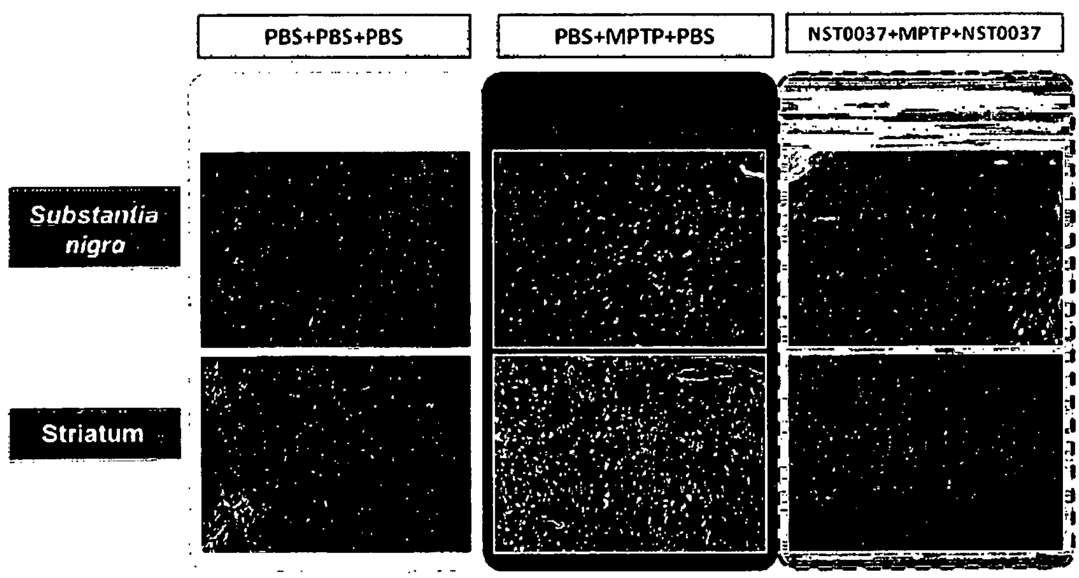

Figure 43

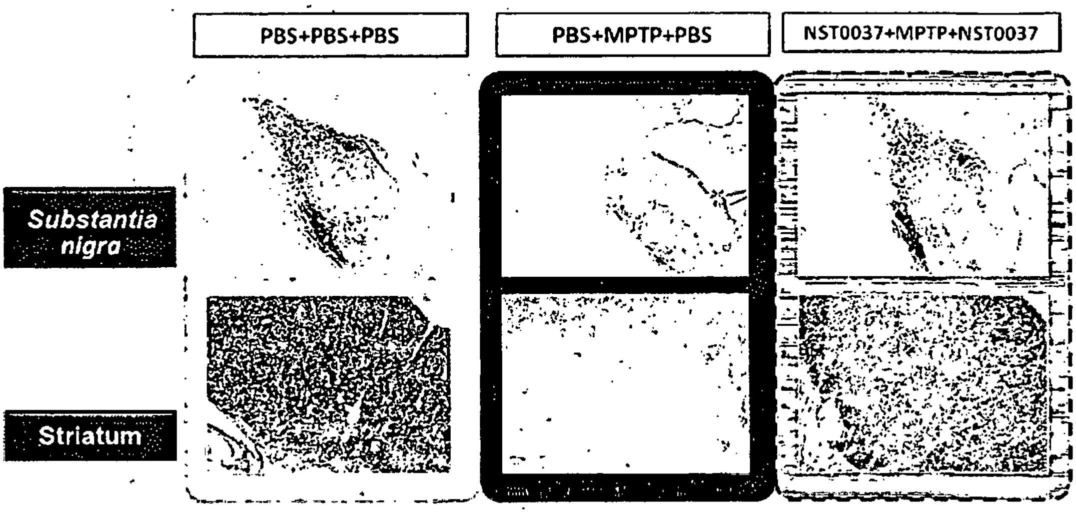


Figure 44

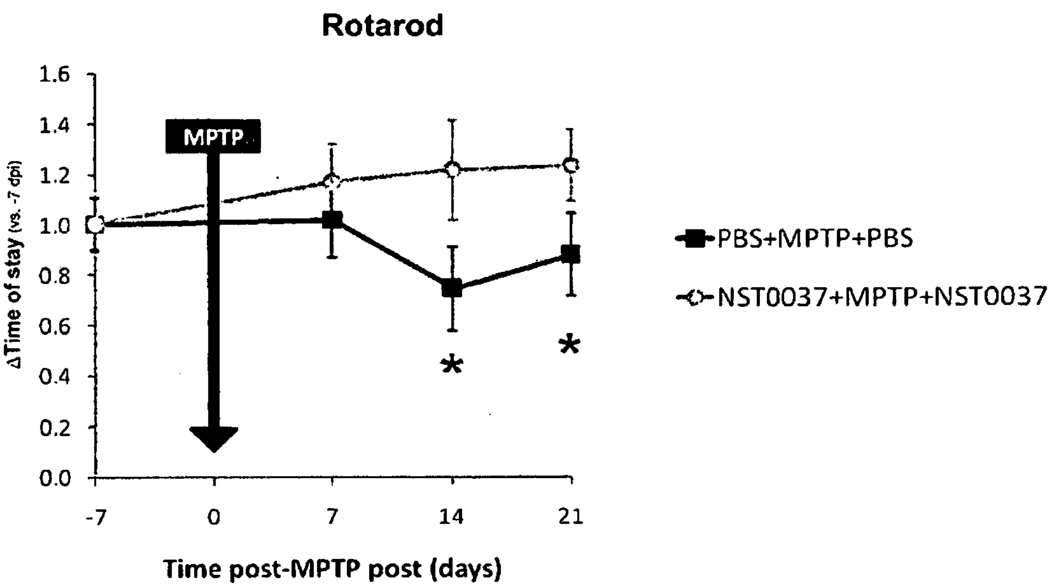

Figure 45

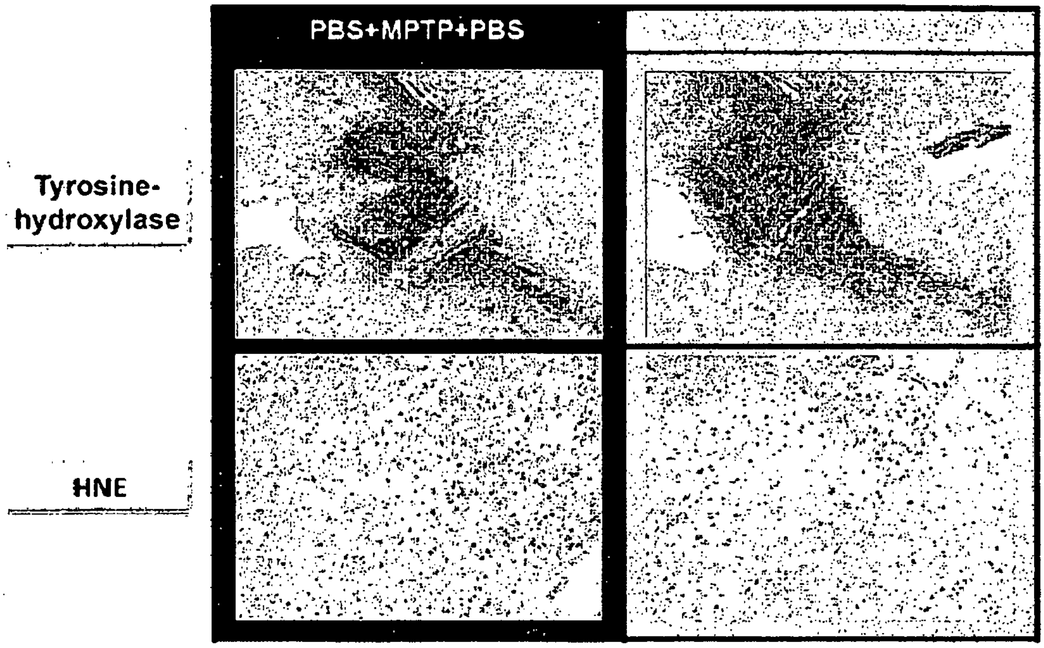


Figure 46

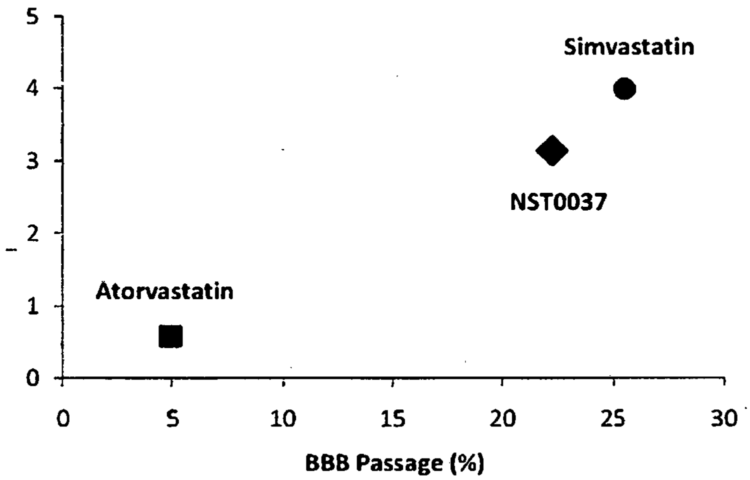

Figure 47

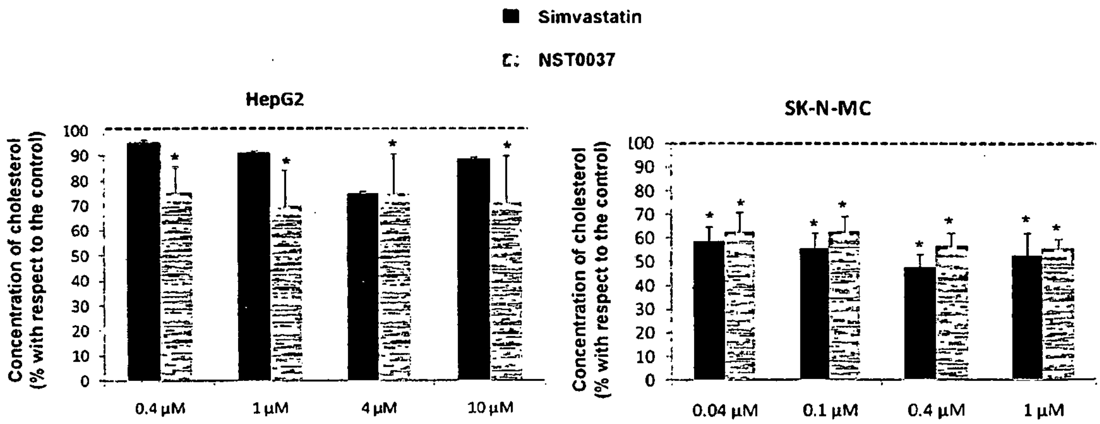


Figure 48

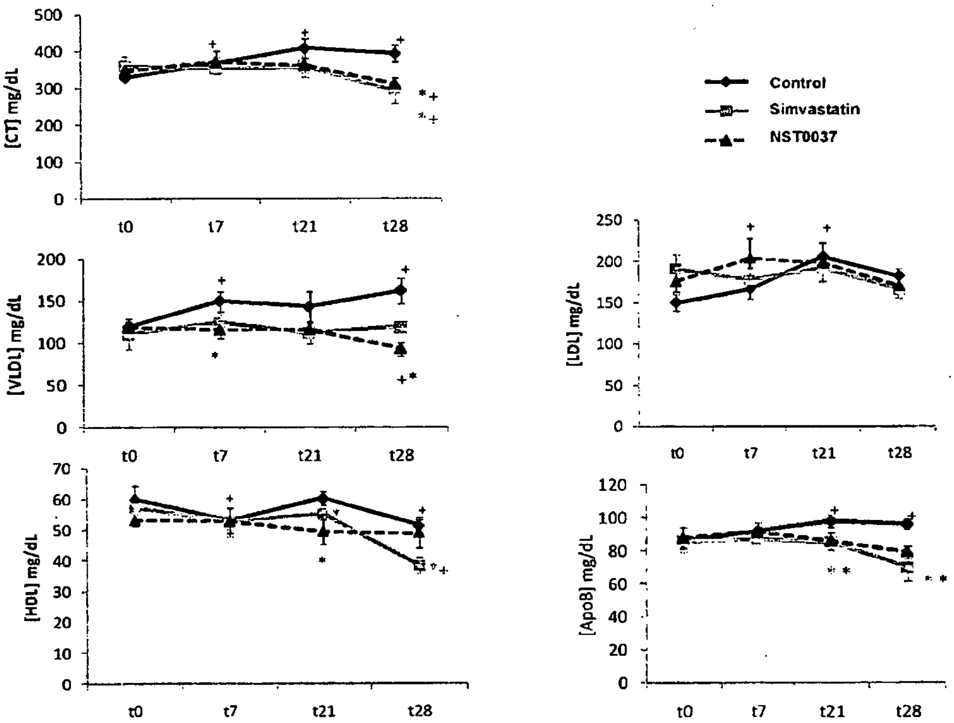


Figure 49

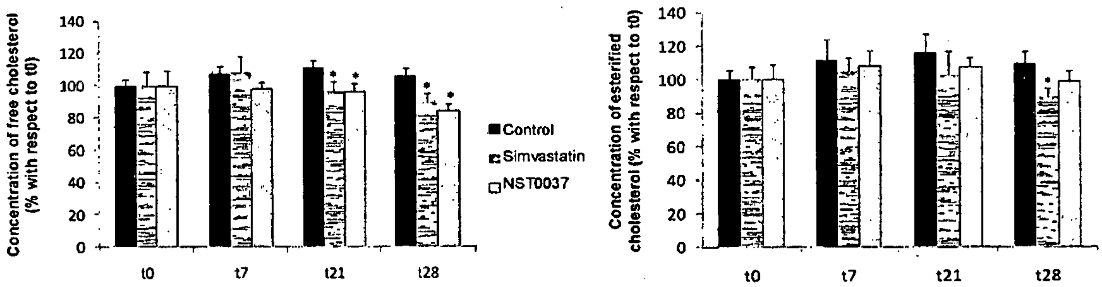

Figure 50

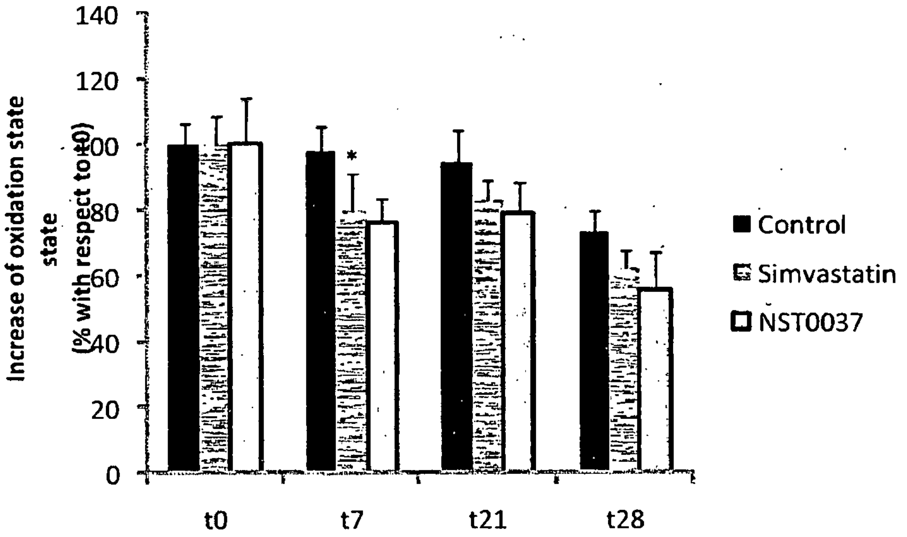


Figure 51

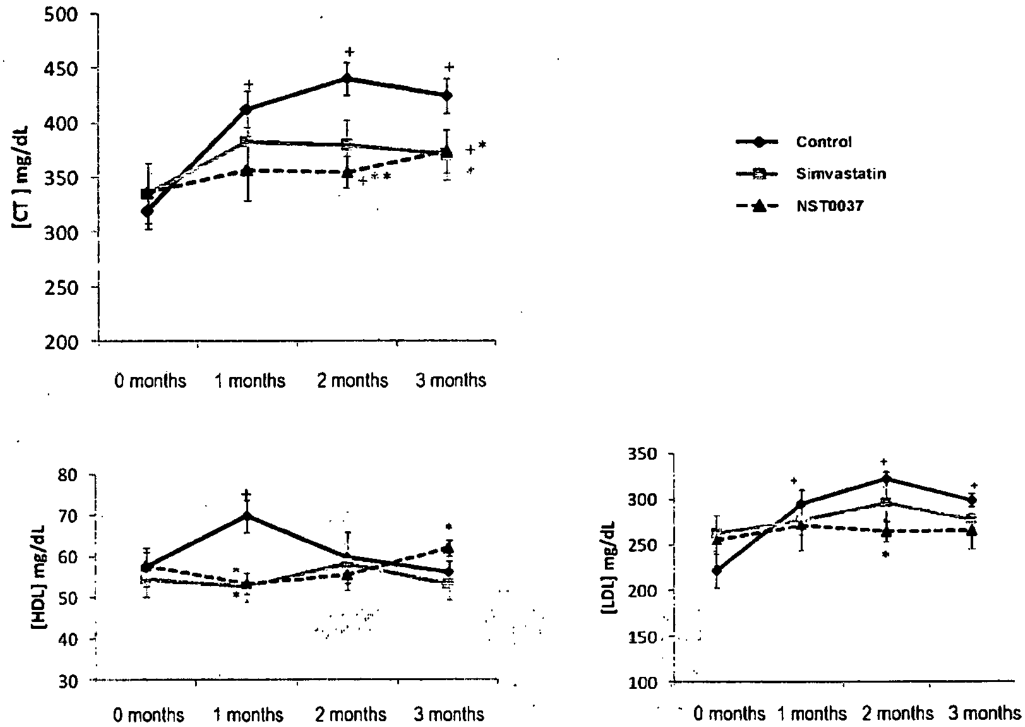

Figure 52

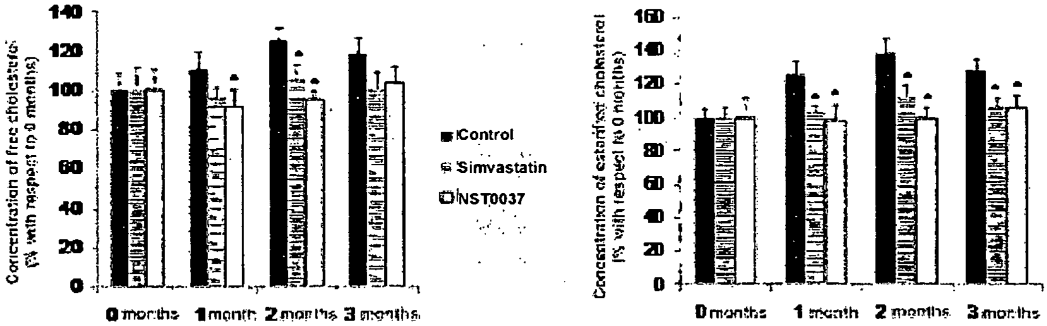


Figure 53

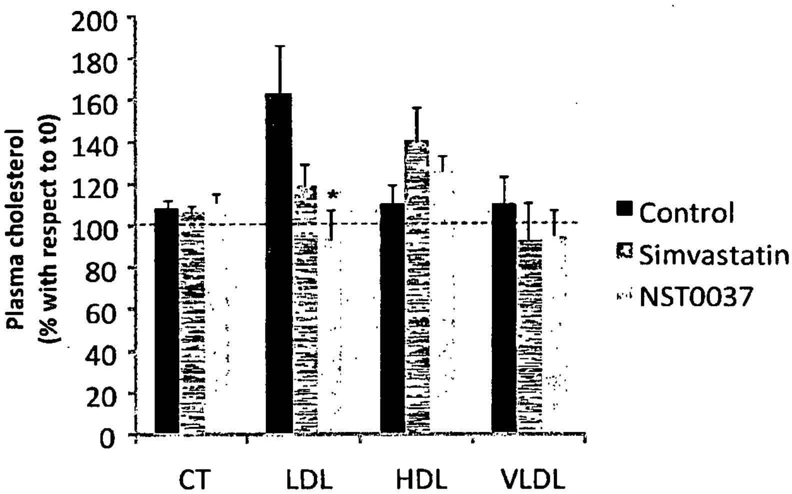


Figure 54

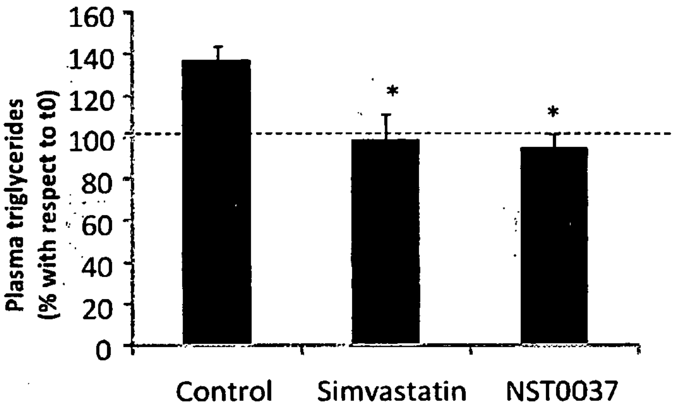

Figure 55

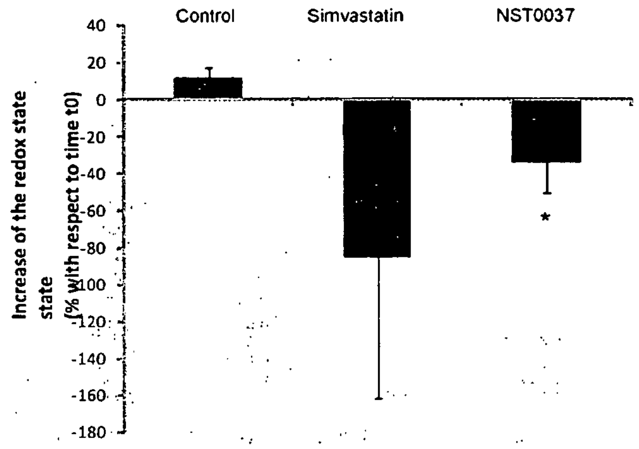


Figure 56

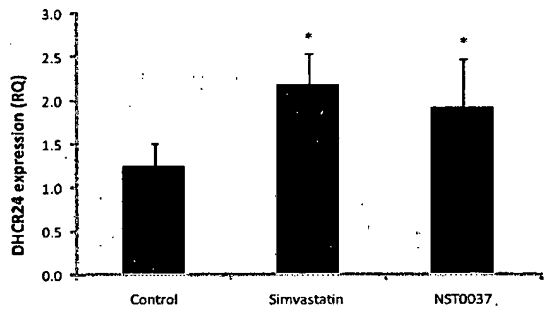


Figure 57

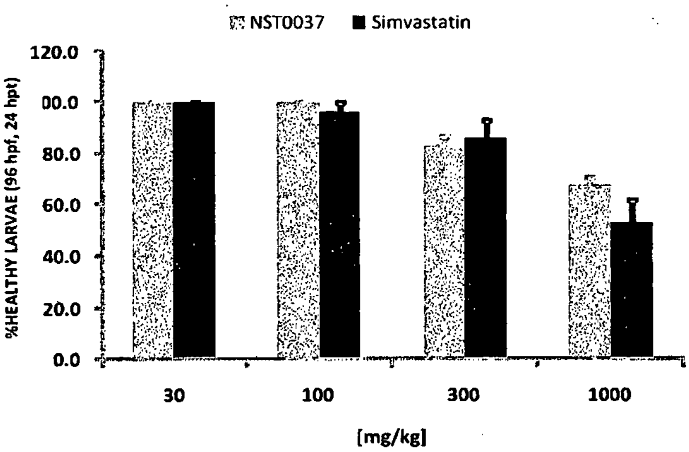

Figure 58

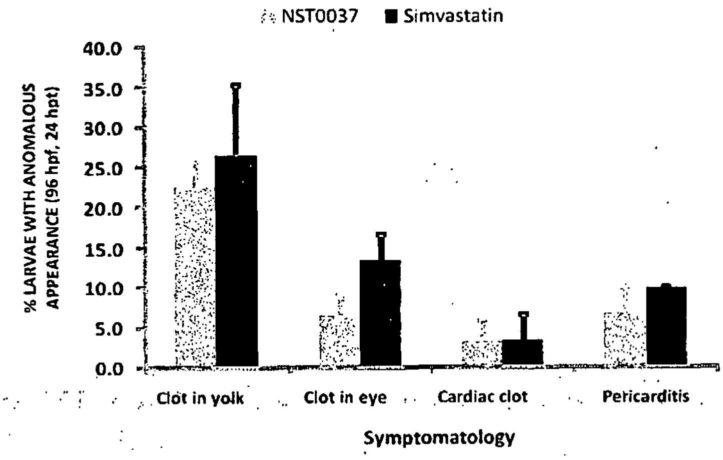


Figure 59

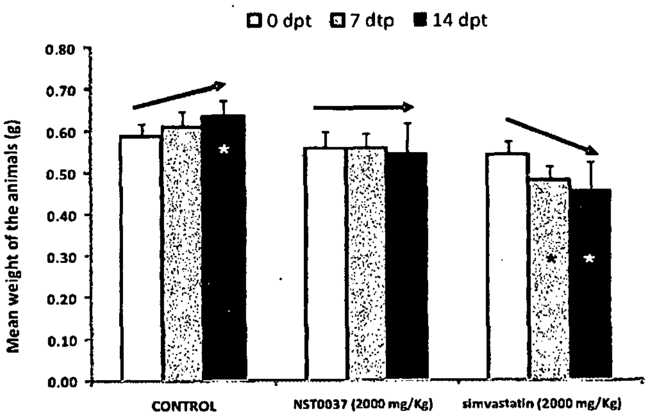

Figure 60

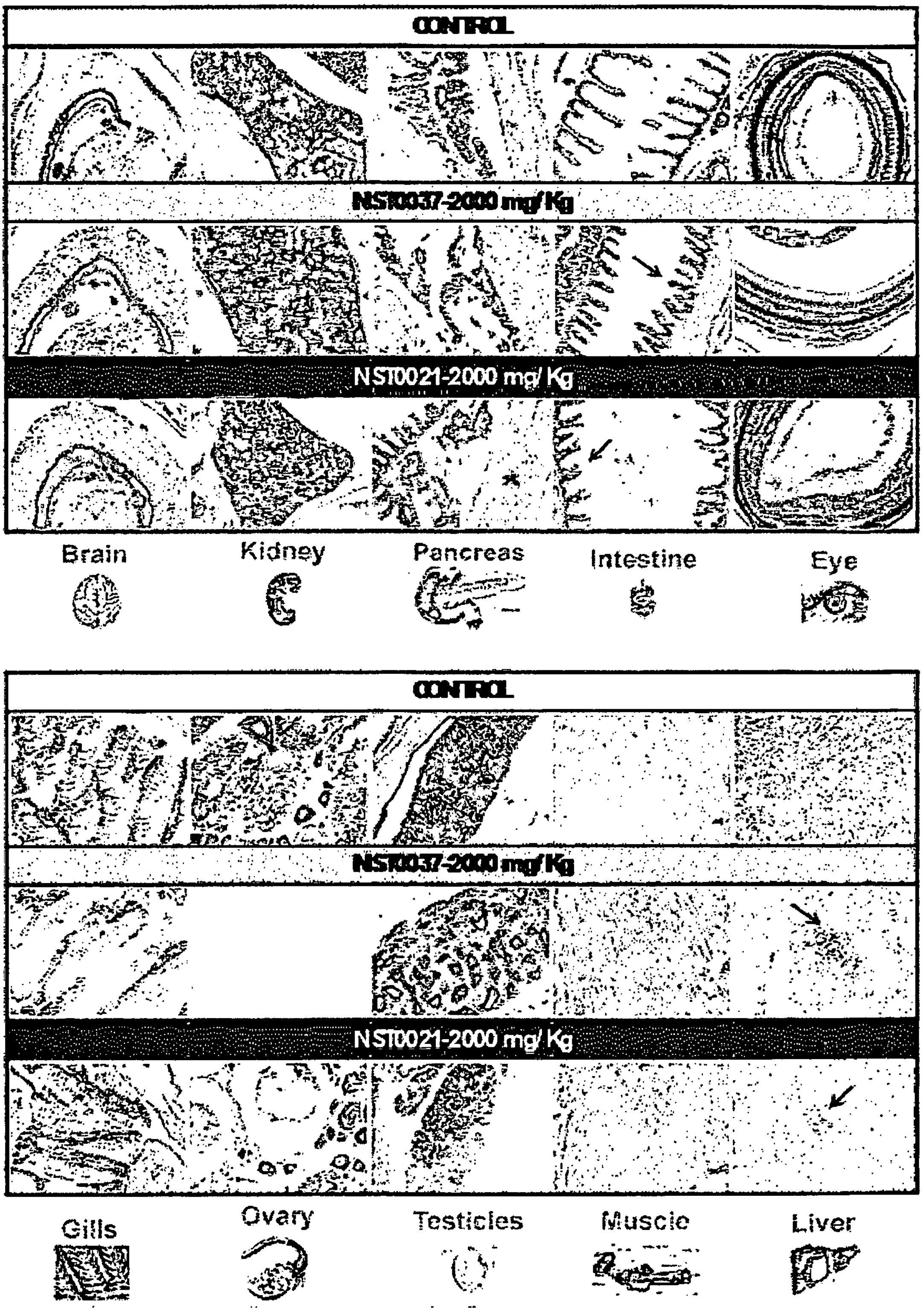
CONTROL
NST0037-2000 mg/Kg
NST0021-2000 mg/Kg
Brain
Kidney
Pancreas
Intestine
Eye
CONTROL
NST0037-2000 mg/Kg
NST0021-2000 mg/Kg
Gills
Ovary
Testicles
Muscle
Liver

Figure 61

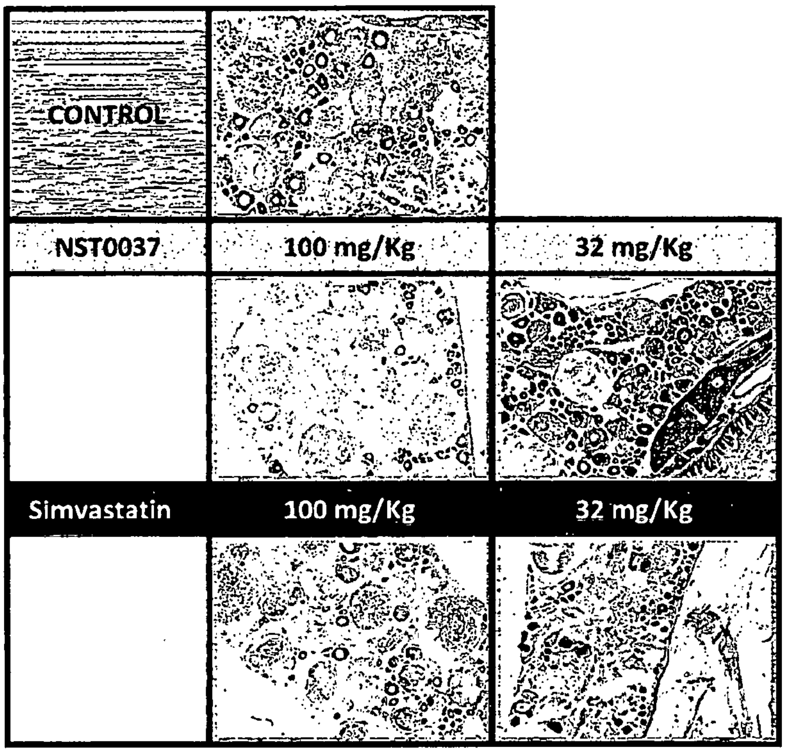
CONTROL
NST0037
100 mg/Kg
32 mg/Kg
Simvastatin
100 mg/Kg
32 mg/Kg

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9911258 A
- US 4866090 A


### Non-patent literature cited in the description


- **BLENNOW et al.** *Lancet,* 2006, vol. 368, 387-403
- **COLE ; VASSAR.** *Neurobiol Dis,* 2006, vol. 22 (2), 209-22
- **PAHAN.** *Cell Mol Life Sci.,* 2006, vol. 63 (10), 1165-78
- **LING et al.** *Ann Neurol.,* 2006, vol. 60 (6), 729-39
- **LEE et al.** *Neurosci Lett.,* 2008, vol. 440, 260-4
- **FANDINO et al.** *Neurocirugia,* 2007, vol. 18, 16-27
- **HONJO et al.** *Arch. Ophthalmol.,* 2002, vol. 120, 1707-13
- **DERE, E. ; HUSTON, J. P. ; DE SOUZA SILVA, M. A.** *Neurobiol Learn Mem,* 2005, vol. 84, 214-21
- **RACINE.** *Electroencephalogr Clin Neurophysiol,* 1972, vol. 32 (3), 281-94
- **CECHI et al.** *J. Cell. Mol. Med.,* 2008, vol. 12, 1990-2002
- **GREEVE et al.** *J. Neurosci.,* 2000, vol. 20, 7345-52
- Treated de Farmacia Galénica. 1993
- **HOFFMAN et al.** *J. Med. Chem.,* 1986, vol. 29, 849-852
- Guidance on the Operation of Animals. *Scientific Procedures, Act.,* 1986